# EUROPEAN PATENT SPECIFICATION

(11) **EP 2 147 103 B1**
(45) Date of publication and mention of the grant of the patent: **12.12.2018**
(21) Application number: 07821575.3
(22) Date of filing: 19.10.2007
(51) Int. Cl.: C12N 15/113, A61K 31/712, A61K 31/7125, C07K 14/715, C07H 21/04, A61K 48/00

(54) **SPLICE SWITCHING OLIGOMERS FOR TNF SUPERFAMILY RECEPTORS AND THEIR USE IN TREATMENT OF DISEASE**
SPLEISSWECHSEL-OLIGOMERE FÜR TNF-SUPERFAMILIE-REZEPTOREN UND DEREN VERWENDUNG BEI DER BEHANDLUNG VON KRANKHEITEN
OLIGOMÈRES PERMUTANT L'ÉPISSAGE POUR LA SUPERFAMILLE DES RÉCEPTEURS AU TNF ET LEUR UTILISATION DANS LE TRAITEMENT DE MALADIES

(30) Priority: 01.05.2007 WO PCT/US2007/010556; 01.05.2007 US 799117
(43) Date of publication of application: 27.01.2010
(73) Proprietor: Roche Innovation Center Copenhagen A/S, 2970 Hørsholm (DK); Ercole Biotech, INC., NC 27709 (US)
(72) Inventor: ØRUM, Henrik, DK-3500 Værløse (DK); SAZANI, Peter, L., Chapel Hill, NC 27514 (US)
(74) Representative: D Young & Co LLP
(86) International application number: PCT/EP2007/061211
(87) International publication number: WO 2008/131807

(56) References cited:
- WO-A-00/58512
- WO-A-02/088393
- WO-A-03/070897
- WO-A-2007/058894
- WO-A-2008/051306
- ROBERTS ET AL: "Efficient and Persistent Splice Switching by Systemically Delivered LNA Oligonucleotides in Mice" MOLECULAR THERAPY, vol. 14, no. 4, October 2006 (2006-10), pages 471-475, XP005644830 ISSN: 1525-0016
- LAINEZ, B. ET AL.: "Identification and characterization of a novel spliced variant that encodes human soluble tumor necrosis factor receptor 2." INTERNATIONAL IMMUNOLOGY, vol. 16, no. 1, January 2004 (2004-01), pages 169-177, XP002437188 ISSN: 0953-8178 cited in the application
- VAN GREEVENBROEK, M. ET AL.: "Soluble receptors for tumor necrosis factor-alpha (TNF-R p55 and TNF-R p75) in familial combined hyperlipidemia." ATHEROSCLEROSIS, vol. 153, no. 1, November 2000 (2000-11), pages 1-8, XP002428909 ISSN: 0021-9150 cited in the application
- SAZANI PETER ET AL: "Therapeutic potential of antisense oligonucleotides as modulators of alternative splicing." JOURNAL OF CLINICAL INVESTIGATION, vol. 112, no. 4, August 2003 (2003-08), pages 481-486, XP002506707 ISSN: 0021-9738
- WENGEL JESPER ET AL: "LNA (Locked Nucleic Acid)" NUCLEOSIDES & NUCLEOTIDES, vol. 18, no. 6-7, June 1999 (1999-06), pages 1365-1370, XP002180387 ISSN: 0732-8311

### SEQUENCE LISTING

<110> Henrik Ørum
<120> SPLICE SWITCHING OLIGOMERS FOR TNF SUPERFAMILY RECEPTORS AND THEIR USE IN TREATMENT OF DISEASE
<130> 17121PCT00
<150> PCT/US2006/043651
   <151> 2006-11-10
<150> US 11/595,485
   <151> 2006-11-10
<150> US 60/862,350
   <151> 2006-11-20
<160> 295
<170> PatentIn version 3.3
<210> 1
   <211> 214
   <212> DNA
   <213> homo sapiens
<400> 1
   tgcggccccc ctctgcccgc tcctctgacc aacacctgct ttgtctgcag gcaccacagt     60
   gctgttgccc ctggtcattt tctttggtct ttgcctttta tccctcctct tcattggttt    120
   aatgtatcgc taccaacggt ggaagtccaa gctctactcc attggtgagt gggggctttg    180
   ggagggagag ggagctggtg ggggtgaggg agga                                214
<210> 2
   <211> 129
   <212> DNA
   <213> homo sapiens
<400> 2
   gggctgagag aggaagtgaa atttatgarg ctttctttct ttttcctcag tttgtgggaa     60
   atcgacacct gaaaaagagg tgagatgaaa tgagagagtt actcccaaat gtccctgacc    120
   attccttat                                                            129
<210> 3
   <211> 178
   <212> DNA
   <213> homo sapiens
<400> 3
   acatttgagt ttgttttctg tagctgtctg agcttctctt ttctttctag gactgattgt     60
   gggtgtgaca gccttgggtc tactaataat aggagtggtg aactgtgtca tcatgaccca    120
   ggtgaaaagt aagagtccat ccttccttcc ttcatccact tgttcaggaa gcttttgt      178
<210> 4
   <211> 135
   <212> DNA
   <213> homo sapiens
<400> 4
   gatgtgcctg aggaagtcaa tctcttactt gtcccctctc ctctttatag agaagccctt     60
   gtgcctgcag agagaagcca aggtggtgag tgtctccact gccctctccc cctcttcccc    120
   tggtctcctt cccgg                                                     135
<210> 5
   <211> 20
   <212> RNA
   <213> artificial
<220>
   <223> 2'O-Me-ribonucleoside-phosphorothioate mouse targeted SSO
<400> 5
   ccgcaguacc ugcagaccag 20
<210> 6
   <211> 20
   <212> RNA
   <213> artificial
<220>
   <223> 2'O-Me-ribonucleoside-phosphorothioate mouse targeted SSO
<400> 6
   guaccugcag accagagagg 20
<210> 7
   <211> 20
   <212> RNA
   <213> artificial
<220>
   <223> 2'O-Me-ribonucleoside-phosphorothioate mouse targeted SSO
<400> 7
   cugcagacca gagagguugc 20
<210> 8
   <211> 20
   <212> RNA
   <213> artificial
<220>
   <223> 2'O-Me-ribonucleoside-phosphorothioate mouse targeted SSO
<400> 8
   acugauggag uagacuucgg 20
<210> 9
   <211> 20
   <212> RNA
   <213> artificial
<220>
   <223> 2'O-Me-ribonucleoside-phosphorothioate mouse targeted SSO
<400> 9
   aguccuacuu acugauggag 20
<210> 10
   <211> 20
   <212> RNA
   <213> artificial
<220>
   <223> 2'O-Me-ribonucleoside-phosphorothioate mouse targeted SSO
<400> 10
   ccaaaguccu acuuacugau 20
<210> 11
   <211> 20
   <212> RNA
   <213> artificial
<220>
   <223> 2'O-Me-ribonucleoside-phosphorothioate mouse targeted SSO
<400> 11
   agauaaccag gggcaacagc 20
<210> 12
   <211> 20
   <212> RNA
   <213> artificial
<220>
   <223> 2'O-Me-ribonucleoside-phosphorothioate mouse targeted SSO
<400> 12
   aggauagaag gcaaagaccu 20
<210> 13
   <211> 20
   <212> RNA
   <213> artificial
<220>
   <223> 2'O-Me-ribonucleoside-phosphorothioate mouse targeted SSO
<400> 13
   ggcacauuaa acugaugaag 20
<210> 14
   <211> 20
   <212> RNA
   <213> artificial
<220>
   <223> 2'O-Me-ribonucleoside-phosphorothioate mouse targeted SSO
<400> 14
   ggccuccacc ggggauaucg 20
<210> 15
   <211> 20
   <212> RNA
   <213> artificial
<220>
   <223> 2'O-Me-ribonucleoside-phosphorothioate mouse targeted SSO
<400> 15
   cuggagaaca aagaaacaag 20
<210> 16
   <211> 20
   <212> RNA
   <213> artificial
<220>
   <223> 2'O-Me-ribonucleoside-phosphorothioate mouse targeted SSO
<400> 16
   aucccuacaa acuggagaac 20
<210> 17
   <211> 20
   <212> RNA
   <213> artificial
<220>
   <223> 2'O-Me-ribonucleoside-phosphorothioate mouse targeted SSO
<400> 17
   ggcacgggau cccuacaaac 20
<210> 18
   <211> 20
   <212> RNA
   <213> artificial
<220>
   <223> 2'O-Me-ribonucleoside-phosphorothioate mouse targeted SSO
<400> 18
   cuucucaccu cuuugacagg 20
<210> 19
   <211> 20
   <212> RNA
   <213> artificial
<220>
   <223> 2'O-Me-ribonucleoside-phosphorothioate mouse targeted SSO
<400> 19
   uggagucguc ccuucucacc 20
<210> 20
   <211> 20
   <212> RNA
   <213> artificial
<220>
   <223> 2'O-Me-ribonucleoside-phosphorothioate mouse targeted SSO
<400> 20
   cuccaacaau cagaccuagg 20
<210> 21
   <211> 20
   <212> RNA
   <213> artificial
<220>
   <223> 2'O-Me-ribonucleoside-phosphorothioate mouse targeted SSO
<400> 21
   caaucagacc uaggaaaacg 20
<210> 22
   <211> 20
   <212> RNA
   <213> artificial
<220>
   <223> 2'O-Me-ribonucleoside-phosphorothioate mouse targeted SSO
<400> 22
   agaccuagga aaacggcagg 20
<210> 23
   <211> 20
   <212> RNA
   <213> artificial
<220>
   <223> 2'O-Me-ribonucleoside-phosphorothioate mouse targeted SSO
<400> 23
   ccuuacuuuu ccucugcacc 20
<210> 24
   <211> 20
   <212> RNA
   <213> artificial
<220>
   <223> 2'O-Me-ribonucleoside-phosphorothioate mouse targeted SSO
<400> 24
   gagcagaacc uuacuuuucc 20
<210> 25
   <211> 20
   <212> RNA
   <213> artificial
<220>
   <223> 2'O-Me-ribonucleoside-phosphorothioate mouse targeted SSO
<400> 25
   gacgagagca gaaccuuacu 20
<210> 26
   <211> 20
   <212> RNA
   <213> artificial
<220>
   <223> 2'O-Me-ribonucleoside-phosphorothioate mouse targeted SSO
<400> 26
   ucagcagacc cagugauguc 20
<210> 27
   <211> 20
   <212> RNA
   <213> artificial
<220>
   <223> 2'O-Me-ribonucleoside-phosphorothioate mouse targeted SSO
<400> 27
   augaugcagu ucaccagucc 20
<210> 28
   <211> 20
   <212> RNA
   <213> artificial
<220>
   <223> 2'O-Me-riboriucleoside-phosphorothioate mouse targeted SSO
<400> 28
   ucaccagucc uaacaucagc 20
<210> 29
   <211> 20
   <212> RNA
   <213> artificial
<220>
   <223> 2'O-Me-ribonucleoside-phosphorothioate mouse targeted SSO
<400> 29
   ccucugcacc aggaugaugc 20
<210> 30
   <211> 20
   <212> RNA
   <213> artificial
<220>
   <223> 2'O-Me-ribonucleoside-phosphorothioate mouse targeted SSO
<400> 30
   uucucuacaa ugaagagagg 20
<210> 31
   <211> 20
   <212> RNA
   <213> artificial
<220>
   <223> 2'O-Me-ribonucleoside-phosphorothioate mouse targeted SSO
<400> 31
   ggcuucucua caaugaagag 20
<210> 32
   <211> 20
   <212> RNA
   <213> artificial
<220>
   <223> 2'O-Me-ribonucleoside-phosphorothioate mouse targeted SSO
<400> 32
   uguaggcagg agggcuucuc 20
<210> 33
   <211> 20
   <212> RNA
   <213> artificial
<220>
   <223> 2'O-Me-ribonucleoside-phosphorothioate mouse targeted SSO
<400> 33
   acucaccacc uuggcaucuc 20
<210> 34
   <211> 20
   <212> RNA
   <213> artificial
<220>
   <223> 2'O-Me-ribonucleoside-phosphorothioate mouse targeted SSO
<400> 34
   gcagagggau acucaccacc 20
<210> 35
   <211> 16
   <212> DNA
   <213> artificial
<220>
   <223> LNA-2'deoxy-ribonucleosidephosphorothioate chimeric mouse targeted SSO
<400> 35
   caatcagacc taggaa 16
<210> 36
   <211> 16
   <212> DNA
   <213> artificial
<220>
   <223> 2'O-Me-ribonucleoside-phosphorothioate mouse targeted SSO
<400> 36
   caacaatcag acctag 16
<210> 37
   <211> 16
   <212> DNA
   <213> artificial
<220>
   <223> 2'O-Me-ribonucleoside-phosphorothioate mouse targeted SSO
<400> 37
   cagacctagg aaaacg 16
<210> 38
   <211> 16
   <212> DNA
   <213> artificial
<220>
   <223> 2'O-Me-ribonucleoside-phosphorothioate mouse targeted SSO
<400> 38
   agcagaccca gtgatg 16
<210> 39
   <211> 16
   <212> DNA
   <213> artificial
<220>
   <223> 2'O-Me-ribonucleoside-phosphorothioate mouse targeted SSO
<400> 39
   ccagtcctaa catcag 16
<210> 40
   <211> 16
   <212> DNA
   <213> artificial
<220>
   <223> 2'O-Me-ribonucleoside-phosphorothioate mouse targeted SSO
<400> 40
   caccagtcct aacatc 16
<210> 41
   <211> 16
   <212> DNA
   <213> artificial
<220>
   <223> 2'O-Me-ribonucleoside-phosphorothioate mouse targeted SSO
<400> 41
   ctgcaccagg atgatg 16
<210> 42
   <211> 16
   <212> DNA
   <213> artificial
<220>
   <223> 2'O-Me-ribonucleoside-phosphorothioate mouse targeted SSO
<400> 42
   acttttcctc tgcacc 16
<210> 43
   <211> 16
   <212> DNA
   <213> artificial
<220>
   <223> 2'O-Me-ribonucleoside-phosphorothioate mouse targeted SSO
<400> 43
   ccttactttt cctctg 16
<210> 44
   <211> 16
   <212> DNA
   <213> artificial
<220>
   <223> 2'O-Me-ribonucleoside-phosphorothioate mouse targeted SSO
<400> 44
   cagaacctta cttttc 16
<210> 45
   <211> 16
   <212> DNA
   <213> artificial
<220>
   <223> 2'O-Me-ribonucleoside-phosphorothioate mouse targeted SSO
<400> 45
   agagcagaac cttact 16
<210> 46
   <211> 16
   <212> DNA
   <213> artificial
<220>
   <223> 2'O-Me-ribonucleoside-phosphorothioate mouse targeted SSO
<400> 46
   gagagcagaa ccttac 16
<210> 47
   <211> 16
   <212> DNA
   <213> artificial
<220>
   <223> 2'O-Me-ribonucleoside-phosphorothioate mouse targeted SSO
<400> 47
   accttacttt tcctct 16
<210> 48
   <211> 16
   <212> DNA
   <213> artificial
<220>
   <223> 2'O-Me-ribonucleoside-phosphorothioate mouse targeted SSO
<400> 48
   cttctctaca atgaag 16
<210> 49
   <211> 16
   <212> DNA
   <213> artificial
<220>
   <223> 2'O-Me-ribonucleoside-phosphorothioate mouse targeted SSO
<400> 49
   ccttggcatc tctttg 16
<210> 50
   <211> 16
   <212> DNA
   <213> artificial
<220>
   <223> 2'O-Me-ribonucleoside-phosphorothioate mouse targeted SSO
<400> 50
   tcaccacctt ggcatc 16
<210> 51
   <211> 16
   <212> DNA
   <213> artificial
<220>
   <223> 2'O-Me-ribonucleoside-phosphorothioate mouse targeted SSO
<400> 51
   actcaccacc ttggca 16
<210> 52
   <211> 16
   <212> DNA
   <213> artificial
<220>
   <223> 2'O-Me-ribonucleoside-phosphorothioate mouse targeted SSO
<400> 52
   gatactcacc accttg 16
<210> 53
   <211> 16
   <212> DNA
   <213> artificial
<220>
   <223> 2'O-Me-ribonucleoside-phosphorothioate mouse targeted SSO
<400> 53
   ctacaatgaa gagagg 16
<210> 54
   <211> 16
   <212> DNA
   <213> artificial
<220>
   <223> 2'O-Me-ribonucleoside-phosphorothioate mouse targeted SSO
<400> 54
   ctctacaatg aagaga 16
<210> 55
   <211> 16
   <212> DNA
   <213> artificial
<220>
   <223> 2'O-Me-ribonucleoside-phosphorothioate mouse targeted SSO
<400> 55
   agggatactc accacc 16
<210> 56
   <211> 16
   <212> DNA
   <213> artificial
<220>
   <223> 2'O-Me-ribonucleoside-phosphorothioate mouse targeted SSO
<400> 56
   cagagggata ctcacc 16
<210> 57
   <211> 16
   <212> DNA
   <213> artificial
<220>
   <223> 2'O-Me-ribonucleoside-phosphorothioate mouse targeted SSO
<400> 57
   cgcagaggga tactca 16
<210> 58
   <211> 16
   <212> DNA
   <213> artificial
<220>
   <223> 2'O-Me-ribonucleoside-phosphorothioate mouse targeted SSO
<400> 58
   gaacaagtca gaggca 16
<210> 59
   <211> 16
   <212> DNA
   <213> artificial
<220>
   <223> 2'O-Me-ribonucleoside-phosphorothioate mouse targeted SSO
<400> 59
   gaggcaggac ttcttc 16
<210> 60
   <211> 16
   <212> DNA
   <213> artificial
<220>
   <223> 2'O-Me-ribonucleoside-phosphorothioate mouse targeted SSO
<400> 60
   cgcagtacct gcagac 16
<210> 61
   <211> 16
   <212> DNA
   <213> artificial
<220>
   <223> 2'O-Me-ribonucleoside-phosphorothioate mouse targeted SSO
<400> 61
   agtacctgca gaccag 16
<210> 62
   <211> 16
   <212> DNA
   <213> artificial
<220>
   <223> 2'O-Me-ribonucleoside-phosphorothioate mouse targeted SSO
<400> 62
   ggcaacagca ccgcag 16
<210> 63
   <211> 16
   <212> DNA
   <213> artificial
<220>
   <223> 2'O-Me-ribonucleoside-phosphorothioate mouse targeted SSO
<400> 63
   ctagcaagat aaccag 16
<210> 64
   <211> 16
   <212> DNA
   <213> artificial
<220>
   <223> 2'O-Me-ribonucleoside-phosphorothioate mouse targeted SSO
<400> 64
   gcacattaaa ctgatg 16
<210> 65
   <211> 16
   <212> DNA
   <213> artificial
<220>
   <223> 2'O-Me-riboriucleoside-phosphorothioate mouse targeted SSO
<400> 65
   cttcgggcct ccaccg 16
<210> 66
   <211> 16
   <212> DNA
   <213> artificial
<220>
   <223> 2'O-Me-ribonucleoside-phosphorothioate mouse targeted SSO
<400> 66
   cttactgatg gagtag 16
<210> 67
   <211> 16
   <212> DNA
   <213> artificial
<220>
   <223> 2'O-Me-ribonucleoside-phosphorothioate mouse targeted SSO
<400> 67
   cctacttact gatgga 16
<210> 68
   <211> 16
   <212> DNA
   <213> artificial
<220>
   <223> 2'O-Me-ribonucleoside-phosphorothioate mouse targeted SSO
<400> 68
   gtcctactta ctgatg 16
<210> 69
   <211> 16
   <212> DNA
   <213> artificial
<220>
   <223> 2'O-Me-ribonucleoside-phosphorothioate mouse targeted SSO
<400> 69
   tccctacaaa ctggag 16
<210> 70
   <211> 16
   <212> DNA
   <213> artificial
<220>
   <223> 2'O-Me-ribonucleoside-phosphorothioate mouse targeted SSO
<400> 70
   ggcacgggat ccctac 16
<210> 71
   <211> 16
   <212> DNA
   <213> artificial
<220>
   <223> 2'O-Me-ribonucleoside-phosphorothioate mouse targeted SSO
<400> 71
   ctctttgaca ggcacg 16
<210> 72
   <211> 16
   <212> DNA
   <213> artificial
<220>
   <223> 2'O-Me-ribonucleoside-phosphorothioate mouse targeted SSO
<400> 72
   ctcacctctt tgacag 16
<210> 73
   <211> 16
   <212> DNA
   <213> artificial
<220>
   <223> 2'O-Me-ribonucleoside-phosphorothioate mouse targeted SSO
<400> 73
   ccttctcacc tctttg 16
<210> 74
   <211> 16
   <212> DNA
   <213> artificial
<220>
   <223> LNA-2'deoxy-ribonucleosidephosphorothioate chimeric human targeted SSO
<400> 74
   ccacaatcag tcctag 16
<210> 75
   <211> 16
   <212> DNA
   <213> artificial
<220>
   <223> 2'O-Me-ribonucleoside-phosphorothioate mouse targeted SSO
<400> 75
   cagtcctaga aagaaa 16
<210> 76
   <211> 16
   <212> DNA
   <213> artificial
<220>
   <223> 2'O-Me-ribonucleoside-phosphorothioate mouse targeted SSO
<400> 76
   agtagaccca aggctg 16
<210> 77
   <211> 16
   <212> DNA
   <213> artificial
<220>
   <223> 2'O-Me-ribonucleoside-phosphorothioate mouse targeted SSO
<400> 77
   ccactcctat tattag 16
<210> 78
   <211> 16
   <212> DNA
   <213> artificial
<220>
   <223> 2'O-Me-ribonucleoside-phosphorothioate mouse targeted SSO
<400> 78
   caccactcct attatt 16
<210> 79
   <211> 16
   <212> DNA
   <213> artificial
<220>
   <223> 2'O-Me-ribonucleoside-phosphorothioate mouse targeted SSO
<400> 79
   ctgggtcatg atgaca 16
<210> 80
   <211> 16
   <212> DNA
   <213> artificial
<220>
   <223> 2'O-Me-ribonucleoside-phosphorothioate mouse targeted SSO
<400> 80
   acttttcacc tgggtc 16
<210> 81
   <211> 16
   <212> DNA
   <213> artificial
<220>
   <223> 2'O-Me-ribonucleoside-phosphorothioate mouse targeted SSO
<400> 81
   tcttactttt cacctg 16
<210> 82
   <211> 16
   <212> DNA
   <213> artificial
<220>
   <223> 2'O-Me-ribonucleoside-phosphorothioate mouse targeted SSO
<400> 82
   tggactctta cttttc 16
<210> 83
   <211> 16
   <212> DNA
   <213> artificial
<220>
   <223> 2'O-Me-ribonucleoside-phosphorothioate mouse targeted SSO
<400> 83
   aggatggact cttact 16
<210> 84
   <211> 16
   <212> DNA
   <213> artificial
<220>
   <223> 2'O-Me-ribonucleoside-phosphorothioate mouse targeted SSO
<400> 84
   aaggatggac tcttac 16
<210> 85
   <211> 16
   <212> DNA
   <213> artificial
<220>
   <223> 2'O-Me-ribonucleoside-phosphorothioate mouse targeted SSO
<400> 85
   cttctctata aagagg 16
<210> 86
   <211> 16
   <212> DNA
   <213> artificial
<220>
   <223> 2'O-Me-ribonucleoside-phosphorothioate mouse targeted SSO
<400> 86
   ccttggcttc tctctg 16
<210> 87
   <211> 16
   <212> DNA
   <213> artificial
<220>
   <223> 2'O-Me-ribonucleoside-phosphorothioate mouse targeted SSO
<400> 87
   tcaccacctt ggcttc 16
<210> 88
   <211> 16
   <212> DNA
   <213> artificial
<220>
   <223> 2'O-Me-ribonucleoside-phosphorothioate mouse targeted SSO
<400> 88
   actcaccacc ttggct 16
<210> 89
   <211> 16
   <212> DNA
   <213> artificial
<220>
   <223> 2'O-Me-ribonucleoside-phosphorothioate mouse targeted SSO
<400> 89
   gacactcacc accttg 16
<210> 90
   <211> 16
   <212> DNA
   <213> artificial
<220>
   <223> 2'O-Me-ribonucleoside-phosphorothioate mouse targeted SSO
<400> 90
   tgtggtgcct gcagac 16
<210> 91
   <211> 16
   <212> DNA
   <213> artificial
<220>
   <223> 2'O-Me-ribonucleoside-phosphorothioate mouse targeted SSO
<400> 91
   ggtgcctgca gacaaa 16
<210> 92
   <211> 16
   <212> DNA
   <213> artificial
<220>
   <223> 2'O-Me-ribonucleoside-phosphorothioate mouse targeted SSO
<400> 92
   ggcaacagca ctgtgg 16
<210> 93
   <211> 16
   <212> DNA
   <213> artificial
<220>
   <223> 2'O-Me-ribonucleoside-phosphorothioate mouse targeted SSO
<400> 93
   caaagaaaat gaccag 16
<210> 94
   <211> 16
   <212> DNA
   <213> artificial
<220>
   <223> 2'O-Me-ribonucleoside-phosphorothioate mouse targeted SSO
<400> 94
   atacattaaa ccaatg 16
<210> 95
   <211> 16
   <212> DNA
   <213> artificial
<220>
   <223> 2'O-Me-ribonucleoside-phosphorothioate mouse targeted SSO
<400> 95
   gcttggactt ccaccg 16
<210> 96
   <211> 16
   <212> DNA
   <213> artificial
<220>
   <223> 2'O-Me-ribonucleoside-phosphorothioate mouse targeted SSO
<400> 96
   ctcaccaatg gagtag 16
<210> 97
   <211> 16
   <212> DNA
   <213> artificial
<220>
   <223> 2'O-Me-ribonucleoside-phosphorothioate mouse targeted SSO
<400> 97
   cactcaccaa tggagt 16
<210> 98
   <211> 16
   <212> DNA
   <213> artificial
<220>
   <223> 2'O-Me-ribonucleoside-phosphorothioate mouse targeted SSO
<400> 98
   cccactcacc aatgga 16
<210> 99
   <211> 16
   <212> DNA
   <213> artificial
<220>
   <223> 2'O-Me-riboriucleoside-phosphorothioate mouse targeted SSO
<400> 99
   cccccactca ccaatg 16
<210> 100
   <211> 16
   <212> DNA
   <213> artificial
<220>
   <223> 2'O-Me-ribonucleoside-phosphorothioate mouse targeted SSO
<400> 100
   aaagccccca ctcacc 16
<210> 101
   <211> 16
   <212> DNA
   <213> artificial
<220>
   <223> 2'O-Me-ribonucleoside-phosphorothioate mouse targeted SSO
<400> 101
   tttcccacaa actgag 16
<210> 102
   <211> 16
   <212> DNA
   <213> artificial
<220>
   <223> 2'O-Me-ribonucleoside-phosphorothioate mouse targeted SSO
<400> 102
   ggtgtcgatt tcccac 16
<210> 103
   <211> 16
   <212> DNA
   <213> artificial
<220>
   <223> 2'O-Me-ribonucleoside-phosphorothioate mouse targeted SSO
<400> 103
   ctctttttca ggtgtc 16
<210> 104
   <211> 16
   <212> DNA
   <213> artificial
<220>
   <223> 2'O-Me-ribonucleoside-phosphorothioate mouse targeted SSO
<400> 104
   ctcacctctt tttcag 16
<210> 105
   <211> 16
   <212> DNA
   <213> artificial
<220>
   <223> 2'O-Me-ribonucleoside-phosphorothioate mouse targeted SSO
<400> 105
   tcatctcacc tctttt 16
<210> 106
   <211> 16
   <212> DNA
   <213> artificial
<220>
   <223> 2'O-Me-ribonucleoside-phosphorothioate mouse targeted SSO
<400> 106
   gctattacct taaccc 16
<210> 107
   <211> 214
   <212> DNA
   <213> Mus musculus
<400> 107
   cccctagtct ctgctgtggc ctcacactga gcaacctctc tggtctgcag gtactgcggt     60
   gctgttgccc ctggttatct tgctaggtct ttgccttcta tcctttatct tcatcagttt    120
   aatgtgccga tatccccggt ggaggcccga agtctactcc atcagtaagt aggactttgg    180
   ggatataggg tgttggtgga gatacgggag gggt                                214
<210> 108
   <211> 129
   <212> DNA
   <213> Mus musculus
<400> 108
   gcgttgaaag ggaagtgaaa ttcatgacac cttgtttctt tgttctccag tttgtaggga      60
   tcccgtgcct gtcaaagagg tgagaaggga cgactccagc ttccctgact actccttcca     120
   acgcctgat                                                             129
<210> 109
   <211> 178
   <212> DNA
   <213> Mus musculus
<400> 109
   caccagccac cctggaacct ttgtttctga gtaccctgcc gttttcctag gtctgattgt      60
   tggagtgaca tcactgggtc tgctgatgtt aggactggtg aactgcatca tcctggtgca     120
   gaggaaaagt aaggttctgc tctcgtcctg tttcccgccc cacgtcccta ccctaaca       178
<210> 110
   <211> 135
   <212> DNA
   <213> Mus musculus
<400> 110
   ctgttctgaa gaagtcctgc ctctgacttg ttcccctctc ttcattgtag agaagccctc      60
   ctgcctacaa agagatgcca aggtggtgag tatccctctg cggtcctcct cccccttctc     120
   tcctccagct ctccc                                                      135
<210> 111
   <211> 21
   <212> DNA
   <213> artificial
<220>
   <223> forward primer PS009
<400> 111
   gaaagtgagt gcgtcccttg c 21
<210> 112
   <211> 20
   <212> DNA
   <213> artificial
<220>
   <223> reverse primer PS010
<400> 112
   gcacggagca gagtgattcg 20
<210> 113
   <211> 21
   <212> DNA
   <213> artificial
<220>
   <223> forward primer PS003
<400> 113
   gagccccaaa tggaaatgtg c 21
<210> 114
   <211> 17
   <212> DNA
   <213> artificial
<220>
   <223> reverse primer PS004
<400> 114
   gctcaaggcc tactgcc 17
<210> 115
   <211> 24
   <212> DNA
   <213> artificial
<220>
   <223> Human TNFR2 mRNA forward primer
<400> 115
   actgaaacat cagacgtggt gtgc 24
<210> 116
   <211> 21
   <212> DNA
   <213> artificial
<220>
   <223> Human TNFR2 mRNA reverse primer
<400> 116
   ccttatcggc aggcaagtga g 21
<210> 117
   <211> 1368
   <212> DNA
   <213> Homo sapiens
<400> 117
   atgggcctct ccaccgtgcc tgacctgctg ctgccactgg tgctcctgga gctgttggtg      60
   ggaatatacc cctcaggggt tattggactg gtccctcacc taggggacag ggagaagaga     120
   gatagtgtgt gtccccaagg aaaatatatc caccctcaaa ataattcgat ttgctgtacc     180
   aagtgccaca aaggaaccta cttgtacaat gactgtccag gcccggggca ggatacggac     240
   tgcagggagt gtgagagcgg ctccttcacc gcttcagaaa accacctcag acactgcctc     300
   agctgctcca aatgccgaaa ggaaatgggt caggtggaga tctcttcttg cacagtggac     360
   cgggacaccg tgtgtggctg caggaagaac cagtaccggc attattggag tgaaaacctt     420
   ttccagtgct tcaattgcag cctctgcctc aatgggaccg tgcacctctc ctgccaggag     480
   aaacagaaca ccgtgtgcac ctgccatgca ggtttctttc taagagaaaa cgagtgtgtc     540
   tcctgtagta actgtaagaa aagcctggag tgcacgaagt tgtgcctacc ccagattgag     600
   aatgttaagg gcactgagga ctcaggcacc acagtgctgt tgcccctggt cattttcttt     660
   ggtctttgcc ttttatccct cctcttcatt ggtttaatgt atcgctacca acggtggaag     720
   tccaagctct actccattgt ttgtgggaaa tcgacacctg aaaaagaggg ggagcttgaa     780
   ggaactacta ctaagcccct ggccccaaac ccaagcttca gtcccactcc aggcttcacc     840
   cccaccctgg gcttcagtcc cgtgcccagt tccaccttca cctccagctc cacctatacc     900
   cccggtgact gtcccaactt tgcggctccc cgcagagagg tggcaccacc ctatcagggg     960
   gctgacccca tccttgcgac agccctcgcc tccgacccca tccccaaccc ccttcagaag    1020
   tgggaggaca gcgcccacaa gccacagagc ctagacactg atgaccccgc gacgctgtac    1080
   gccgtggtgg agaacgtgcc cccgttgcgc tggaaggaat tcgtgcggcg cctagggctg    1140
   agcgaccacg agatcgatcg gctggagctg cagaacgggc gctgcctgcg cgaggcgcaa    1200
   tacagcatgc tggcgacctg gaggcggcgc acgccgcggc gcgaggccac gctggagctg    1260
   ctgggacgcg tgctccgcga catggacctg ctgggctgcc tggaggacat cgaggaggcg    1320
   ctttgcggcc ccgccgccct cccgcccgcg cccagtcttc tcagatga                  1368
<210> 118
   <211> 455
   <212> PRT
   <213> Homo sapiens
<400> 118
<210> 119
   <211> 1386
   <212> DNA
   <213> Homo sapiens
<400> 119
   atggcgcccg tcgccgtctg ggccgcgctg gccgtcggac tggagctctg ggctgcggcg      60
   cacgccttgc ccgcccaggt ggcatttaca ccctacgccc cggagcccgg gagcacatgc     120
   cggctcagag aatactatga ccagacagct cagatgtgct gcagcaaatg ctcgccgggc     180
   caacatgcaa aagtcttctg taccaagacc tcggacaccg tgtgtgactc ctgtgaggac     240
   agcacataca cccagctctg gaactgggtt cccgagtgct tgagctgtgg ctcccgctgt     300
   agctctgacc aggtggaaac tcaagcctgc actcgggaac agaaccgcat ctgcacctgc     360
   aggcccggct ggtactgcgc gctgagcaag caggaggggt gccggctgtg cgcgccgctg     420
   cgcaagtgcc gcccgggctt cggcgtggcc agaccaggaa ctgaaacatc agacgtggtg     480
   tgcaagccct gtgccccggg gacgttctcc aacacgactt catccacgga tatttgcagg     540
   ccccaccaga tctgtaacgt ggtggccatc cctgggaatg caagcatgga tgcagtctgc     600
   acgtccacgt cccccacccg gagtatggcc ccaggggcag tacacttacc ccagccagtg     660
   tccacacgat cccaacacac gcagccaact ccagaaccca gcactgctcc aagcacctcc     720
   ttcctgctcc caatgggccc cagcccccca gctgaaggga gcactggcga cttcgctctt     780
   ccagttggac tgattgtggg tgtgacagcc ttgggtctac taataatagg agtggtgaac     840
   tgtgtcatca tgacccaggt gaaaaagaag cccttgtgcc tgcagagaga agccaaggtg     900
   cctcacttgc ctgccgataa ggcccggggt acacagggcc ccgagcagca gcacctgctg     960
   atcacagcgc cgagctccag cagcagctcc ctggagagct cggccagtgc gttggacaga    1020
   agggcgccca ctcggaacca gccacaggca ccaggcgtgg aggccagtgg ggccggggag    1080
   gcccgggcca gcaccgggag ctcagattct tcccctggtg gccatgggac ccaggtcaat    1140
   gtcacctgca tcgtgaacgt ctgtagcagc tctgaccaca gctcacagtg ctcctcccaa 1200
   gccagctcca caatgggaga cacagattcc agcccctcgg agtccccgaa ggacgagcag 1260
   gtccccttct ccaaggagga atgtgccttt cggtcacagc tggagacgcc agagaccctg 1320
   ctggggagca ccgaagagaa gcccctgccc cttggagtgc ctgatgctgg gatgaagccc 1380
   agttaa 1386
<210> 120
   <211> 461
   <212> PRT
   <213> Homo sapiens
<400> 120
   Met Ala Pro Val Ala Val Trp Ala Ala Leu Ala Val Gly Leu Glu Leu
   1               5                   10                  15
   Trp Ala Ala Ala His Ala Leu Pro Ala Gln Val Ala Phe Thr Pro Tyr
               20                  25                  30
   Ala Pro Glu Pro Gly Ser Thr Cys Arg Leu Arg Glu Tyr Tyr Asp Gln
           35                  40                  45
   Thr Ala Gln Met Cys Cys Ser Lys Cys Ser Pro Gly Gln His Ala Lys
       50                  55                  60
   Val Phe Cys Thr Lys Thr Ser Asp Thr Val Cys Asp Ser Cys Glu Asp
   65                  70                  75                  80
   Ser Thr Tyr Thr Gln Leu Trp Asn Trp Val Pro Glu Cys Leu Ser Cys
                   85                  90                  95
   Gly Ser Arg Cys Ser Ser Asp Gln Val Glu Thr Gln Ala Cys Thr Arg
               100                 105                 110
   Glu Gln Asn Arg Ile Cys Thr Cys Arg Pro Gly Trp Tyr Cys Ala Leu
           115                 120                 125
   Ser Lys Gln Glu Gly Cys Arg Leu Cys Ala Pro Leu Arg Lys Cys Arg
       130                 135                 140
   Pro Gly Phe Gly Val Ala Arg Pro Gly Thr Glu Thr Ser Asp Val Val
   145                 150                 155                 160
   Cys Lys Pro Cys Ala Pro Gly Thr Phe Ser Asn Thr Thr Ser Ser Thr
                   165                 170                 175
   Asp Ile Cys Arg Pro His Gln Ile Cys Asn Val Val Ala Ile Pro Gly
               180                 185                 190
   Asn Ala Ser Met Asp Ala Val Cys Thr Ser Thr Ser Pro Thr Arg Ser
           195                 200                 205
   Met Ala Pro Gly Ala Val His Leu Pro Gln Pro Val Ser Thr Arg Ser
       210                 215                 220
   Gln His Thr Gln Pro Thr Pro Glu Pro Ser Thr Ala Pro Ser Thr Ser
   225                 230                 235                 240
   Phe Leu Leu Pro Met Gly Pro Ser Pro Pro Ala Glu Gly Ser Thr Gly
                   245                 250                 255
   Asp Phe Ala Leu Pro Val Gly Leu Ile Val Gly Val Thr Ala Leu Gly
               260                 265                 270
   Leu Leu Ile Ile Gly Val Val Asn Cys Val Ile Met Thr Gln Val Lys
           275                 280                 285
   Lys Lys Pro Leu Cys Leu Gln Arg Glu Ala Lys Val Pro His Leu Pro
       290                 295                 300
   Ala Asp Lys Ala Arg Gly Thr Gln Gly Pro Glu Gln Gln His Leu Leu
   305                 310                 315                 320
   Ile Thr Ala Pro Ser Ser Ser Ser Ser Ser Leu Glu Ser Ser Ala Ser
                   325                 330                 335
   Ala Leu Asp Arg Arg Ala Pro Thr Arg Asn Gln Pro Gln Ala Pro Gly
               340                 345                 350
   Val Glu Ala Ser Gly Ala Gly Glu Ala Arg Ala Ser Thr Gly Ser Ser
           355                 360                 365
   Asp Ser Ser Pro Gly Gly His Gly Thr Gln Val Asn Val Thr Cys Ile
       370                 375                 380
   Val Asn Val Cys Ser Ser Ser Asp His Ser Ser Gln Cys Ser Ser Gln
   385                 390                 395                 400
   Ala Ser Ser Thr Met Gly Asp Thr Asp Ser Ser Pro Ser Glu Ser Pro
                   405                 410                 415
   Lys Asp Glu Gln Val Pro Phe Ser Lys Glu Glu Cys Ala Phe Arg Ser
                   420                 425                 430
   Gln Leu Glu Thr Pro Glu Thr Leu Leu Gly Ser Thr Glu Glu Lys Pro
           435                 440                 445
   Leu Pro Leu Gly Val Pro Asp Ala Gly Met Lys Pro Ser
       450                 455                 460
<210> 121
   <211> 1254
   <212> DNA
   <213> Homo sapiens
<400> 121
   atgggcctct ccaccgtgcc tgacctgctg ctgccactgg tgctcctgga gctgttggtg     60
   ggaatatacc cctcaggggt tattggactg gtccctcacc taggggacag ggagaagaga    120
   gatagtgtgt gtccccaagg aaaatatatc caccctcaaa ataattcgat ttgctgtacc    180
   aagtgccaca aaggaaccta cttgtacaat gactgtccag gcccggggca ggatacggac    240
   tgcagggagt gtgagagcgg ctccttcacc gcttcagaaa accacctcag acactgcctc    300
   agctgctcca aatgccgaaa ggaaatgggt caggtggaga tctcttcttg cacagtggac    360
   cgggacaccg tgtgtggctg caggaagaac cagtaccggc attattggag tgaaaacctt    420
   ttccagtgct tcaattgcag cctctgcctc aatgggaccg tgcacctctc ctgccaggag    480
   aaacagaaca ccgtgtgcac ctgccatgca ggtttctttc taagagaaaa cgagtgtgtc    540
   tcctgtagta actgtaagaa aagcctggag tgcacgaagt tgtgcctacc ccagattgag    600
   aatgttaagg gcactgagga ctcagtttgt gggaaatcga cacctgaaaa agagggggag   660
   cttgaaggaa ctactactaa gcccctggcc ccaaacccaa gcttcagtcc cactccaggc    720
   ttcaccccca ccctgggctt cagtcccgtg cccagttcca ccttcacctc cagctccacc    780
   tatacccccg gtgactgtcc caactttgcg gctccccgca gagaggtggc accaccctat    840
   cagggggctg accccatcct tgcgacagcc ctcgcctccg accccatccc caaccccctt   900
   cagaagtggg aggacagcgc ccacaagcca cagagcctag acactgatga ccccgcgacg    960
   ctgtacgccg tggtggagaa cgtgcccccg ttgcgctgga aggaattcgt gcggcgccta   1020
   gggctgagcg accacgagat cgatcggctg gagctgcaga acgggcgctg cctgcgcgag   1080
   gcgcaataca gcatgctggc gacctggagg cggcgcacgc cgcggcgcga ggccacgctg   1140
   gagctgctgg gacgcgtgct ccgcgacatg gacctgctgg gctgcctgga ggacatcgag   1200
   gaggcgcttt gcggccccgc cgccctcccg cccgcgccca gtcttctcag atga         1254
<210> 122
   <211> 417
   <212> PRT
   <213> Homo sapiens
<400> 122
   Met Gly Leu Ser Thr Val Pro Asp Leu Leu Leu Pro Leu Val Leu Leu
   1               5                   10                  15
   Glu Leu Leu Val Gly Ile Tyr Pro Ser Gly Val Ile Gly Leu Val Pro
               20                  25                  30
   His Leu Gly Asp Arg Glu Lys Arg Asp Ser Val Cys Pro Gln Gly Lys
           35                  40                  45
   Tyr Ile His Pro Gln Asn Asn Ser Ile Cys Cys Thr Lys Cys His Lys
       50                  55                  60
   Gly Thr Tyr Leu Tyr Asn Asp Cys Pro Gly Pro Gly Gln Asp Thr Asp
   65                  70                  75                  80
   Cys Arg Glu Cys Glu Ser Gly Ser Phe Thr Ala Ser Glu Asn His Leu
                   85                  90                  95
   Arg His Cys Leu Ser Cys Ser Lys Cys Arg Lys Glu Met Gly Gln Val
               100                 105                 110
   Glu Ile Ser Ser Cys Thr Val Asp Arg Asp Thr Val Cys Gly Cys Arg
           115                 120                 125
   Lys Asn Gln Tyr Arg His Tyr Trp Ser Glu Asn Leu Phe Gln Cys Phe
       130                 135                 140
   Asn Cys Ser Leu Cys Leu Asn Gly Thr Val His Leu Ser Cys Gln Glu
   145                 150                 155                 160
   Lys Gln Asn Thr Val Cys Thr Cys His Ala Gly Phe Phe Leu Arg Glu
                   165                 170                 175
   Asn Glu Cys Val Ser Cys Ser Asn Cys Lys Lys Ser Leu Glu Cys Thr
               180                 185                 190
   Lys Leu Cys Leu Pro Gln Ile Glu Asn Val Lys Gly Thr Glu Asp Ser
           195                 200                 205
   Val Cys Gly Lys Ser Thr Pro Glu Lys Glu Gly Glu Leu Glu Gly Thr
       210                 215                 220
   Thr Thr Lys Pro Leu Ala Pro Asn Pro Ser Phe Ser Pro Thr Pro Gly
   225                 230                 235                 240
   Phe Thr Pro Thr Leu Gly Phe Ser Pro Val Pro Ser Ser Thr Phe Thr
                   245                 250                 255
   Ser Ser Ser Thr Tyr Thr Pro Gly Asp Cys Pro Asn Phe Ala Ala Pro
               260                 265                 270
   Arg Arg Glu Val Ala Pro Pro Tyr Gln Gly Ala Asp Pro Ile Leu Ala
           275                 280                 285
   Thr Ala Leu Ala Ser Asp Pro Ile Pro Asn Pro Leu Gln Lys Trp Glu
       290                 295                 300
   Asp Ser Ala His Lys Pro Gln Ser Leu Asp Thr Asp Asp Pro Ala Thr
   305                 310                 315                 320
   Leu Tyr Ala Val Val Glu Asn Val Pro Pro Leu Arg Trp Lys Glu Phe
                   325                 330                 335
   Val Arg Arg Leu Gly Leu Ser Asp His Glu Ile Asp Arg Leu Glu Leu
               340                 345                 350
   Gln Asn Gly Arg Cys Leu Arg Glu Ala Gln Tyr Ser Met Leu Ala Thr
           355                 360                 365
   Trp Arg Arg Arg Thr Pro Arg Arg Glu Ala Thr Leu Glu Leu Leu Gly
       370                 375                 380
   Arg Val Leu Arg Asp Met Asp Leu Leu Gly Cys Leu Glu Asp Ile Glu
   385                 390                 395                 400
   Glu Ala Leu Cys Gly Pro Ala Ala Leu Pro Pro Ala Pro Ser Leu Leu
                   405                 410                 415
   Arg
<210> 123
   <211> 1251
   <212> DNA
   <213> Mus sp.
<400> 123
   atgggtctcc ccaccgtgcc tggcctgctg ctgtcactgg tgctcctggc tctgctgatg      60
   gggatacatc catcaggggt cactggacta gtcccttctc ttggtgaccg ggagaagagg     120
   gatagcttgt gtccccaagg aaagtatgtc cattctaaga acaattccat ctgctgcacc    180
   aagtgccaca aaggaaccta cttggtgagt gactgtccga gcccagggcg ggatacagtc    240
   tgcagggagt gtgaaaaggg cacctttacg gcttcccaga attacctcag gcagtgtctc    300
   agttgcaaga catgtcggaa agaaatgtcc caggtggaga tctctccttg ccaagctgac    360
   aaggacacgg tgtgtggctg taaggagaac cagttccaac gctacctgag tgagacacac    420
   ttccagtgcg tggactgcag cccctgcttc aacggcaccg tgacaatccc ctgtaaggag    480
   actcagaaca ccgtgtgtaa ctgccatgca gggttctttc tgagagaaag tgagtgcgtc    540
   ccttgcagcc actgcaagaa aaatgaggag tgtatgaagt tgtgcctacc tcctccgctt    600
   gcaaatgtca caaaccccca ggactcagtt tgtagggatc ccgtgcctgt caaagaggag    660
   aaggctggaa agcccctaac tccagccccc tccccagcct tcagccccac ctccggcttc    720
   aaccccactc tgggcttcag caccccaggc tttagttctc ctgtctccag tacccccatc    780
   agccccatct tcggtcctag taactggcac ttcatgccac ctgtcagtga ggtagtccca    840
   acccagggag ctgaccctct gctctacgaa tcactctgct ccgtgccagc ccccacctct    900
   gttcagaaat gggaagactc cgcccacccg caacgtcctg acaatgcaga ccttgcgatt    960
   ctgtatgctg tggtggatgg cgtgcctcca gcgcgctgga aggagttcat gcgtttcatg   1020
   gggctgagcg agcacgagat cgagaggctg gagatgcaga acgggcgctg cctgcgcgag   1080
   gctcagtaca gcatgctgga agcctggcgg cgccgcacgc cgcgccacga ggacacgctg   1140
   gaagtagtgg gcctcgtgct ttccaagatg aacctggctg ggtgcctgga gaatatcctc   1200
   gaggctctga gaaatcccgc cccctcgtcc acgacccgcc tcccgcgata a            1251
<210> 124
   <211> 416
   <212> PRT
   <213> Mus sp.
<400> 124
   Met Gly Leu Pro Thr Val Pro Gly Leu Leu Leu Ser Leu Val Leu Leu
   1               5                   10                  15
   Ala Leu Leu Met Gly Ile His Pro Ser Gly Val Thr Gly Leu Val Pro
               20                  25                  30
   Ser Leu Gly Asp Arg Glu Lys Arg Asp Ser Leu Cys Pro Gln Gly Lys
           35                  40                  45
   Tyr Val His Ser Lys Asn Asn Ser Ile Cys Cys Thr Lys Cys His Lys
       50                  55                  60
   Gly Thr Tyr Leu Val Ser Asp Cys Pro Ser Pro Gly Arg Asp Thr Val
   65                  70                  75                  80
   Cys Arg Glu Cys Glu Lys Gly Thr Phe Thr Ala Ser Gln Asn Tyr Leu
                   85                  90                  95
   Arg Gln Cys Leu Ser Cys Lys Thr Cys Arg Lys Glu Met Ser Gln Val
               100                 105                 110
   Glu Ile Ser Pro Cys Gln Ala Asp Lys Asp Thr Val Cys Gly Cys Lys
           115                 120                 125
   Glu Asn Gln Phe Gln Arg Tyr Leu Ser Glu Thr His Phe Gln Cys Val
       130                 135                 140
   Asp Cys Ser Pro Cys Phe Asn Gly Thr Val Thr Ile Pro Cys Lys Glu
   145                 150                 155                 160
   Thr Gln Asn Thr Val Cys Asn Cys His Ala Gly Phe Phe Leu Arg Glu
                   165                 170                 175
   Ser Glu Cys Val Pro Cys Ser His Cys Lys Lys Asn Glu Glu Cys Met
               180                 185                 190
   Lys Leu Cys Leu Pro Pro Pro Leu Ala Asn Val Thr Asn Pro Gln Asp
           195                 200                 205
   Ser Val Cys Arg Asp Pro Val Pro Val Lys Glu Glu Lys Ala Gly Lys
       210                 215                 220
   Pro Leu Thr Pro Ala Pro Ser Pro Ala Phe Ser Pro Thr Ser Gly Phe
   225                 230                 235                 240
   Asn Pro Thr Leu Gly Phe Ser Thr Pro Gly Phe Ser Ser Pro Val Ser
                   245                 250                 255
   Ser Thr Pro Ile Ser Pro Ile Phe Gly Pro Ser Asn Trp His Phe Met
               260                 265                 270
   Pro Pro Val Ser Glu Val Val Pro Thr Gln Gly Ala Asp Pro Leu Leu
           275                 280                 285
   Tyr Glu Ser Leu Cys Ser Val Pro Ala Pro Thr Ser Val Gln Lys Trp
       290                 295                 300
   Glu Asp Ser Ala His Pro Gln Arg Pro Asp Asn Ala Asp Leu Ala Ile
   305                 310                 315                 320
   Leu Tyr Ala Val Val Asp Gly Val Pro Pro Ala Arg Trp Lys Glu Phe
                   325                 330                 335
   Met Arg Phe Met Gly Leu Ser Glu His Glu Ile Glu Arg Leu Glu Met
               340                 345                 350
   Gln Asn Gly Arg Cys Leu Arg Glu Ala Gln Tyr Ser Met Leu Glu Ala
           355                 360                 365
   Trp Arg Arg Arg Thr Pro Arg His Glu Asp Thr Leu Glu Val Val Gly
       370                 375                 380
   Leu Val Leu Ser Lys Met Asn Leu Ala Gly Cys Leu Glu Asn Ile Leu
   385                 390                 395                 400
   Glu Ala Leu Arg Asn Pro Ala Pro Ser Ser Thr Thr Arg Leu Pro Arg
                   405                 410                 415
<210> 125
   <211> 1308
   <212> DNA
   <213> Homo sapiens
<400> 125
   atggcgcccg tcgccgtctg ggccgcgctg gccgtcggac tggagctctg ggctgcggcg      60
   cacgccttgc ccgcccaggt ggcatttaca ccctacgccc cggagcccgg gagcacatgc     120
   cggctcagag aatactatga ccagacagct cagatgtgct gcagcaaatg ctcgccgggc     180
   caacatgcaa aagtcttctg taccaagacc tcggacaccg tgtgtgactc ctgtgaggac     240
   agcacataca cccagctctg gaactgggtt cccgagtgct tgagctgtgg ctcccgctgt     300
   agctctgacc aggtggaaac tcaagcctgc actcgggaac agaaccgcat ctgcacctgc     360
   aggcccggct ggtactgcgc gctgagcaag caggaggggt gccggctgtg cgcgccgctg     420
   cgcaagtgcc gcccgggctt cggcgtggcc agaccaggaa ctgaaacatc agacgtggtg     480
   tgcaagccct gtgccccggg gacgttctcc aacacgactt catccacgga tatttgcagg     540
   ccccaccaga tctgtaacgt ggtggccatc cctgggaatg caagcatgga tgcagtctgc     600
   acgtccacgt cccccacccg gagtatggcc ccaggggcag tacacttacc ccagccagtg     660
   tccacacgat cccaacacac gcagccaact ccagaaccca gcactgctcc aagcacctcc     720
   ttcctgctcc caatgggccc cagcccccca gctgaaggga gcactggcga cttcgctctt     780
   ccagttgaga agcccttgtg cctgcagaga gaagccaagg tgcctcactt gcctgccgat     840
   aaggcccggg gtacacaggg ccccgagcag cagcacctgc tgatcacagc gccgagctcc     900
   agcagcagct ccctggagag ctcggccagt gcgttggaca gaagggcgcc cactcggaac     960
   cagccacagg caccaggcgt ggaggccagt ggggccgggg aggcccgggc cagcaccggg    1020
   agctcagatt cttcccctgg tggccatggg acccaggtca atgtcacctg catcgtgaac    1080
   gtctgtagca gctctgacca cagctcacag tgctcctccc aagccagctc cacaatggga    1140
   gacacagatt ccagcccctc ggagtccccg aaggacgagc aggtcccctt ctccaaggag    1200
   gaatgtgcct ttcggtcaca gctggagacg ccagagaccc tgctggggag caccgaagag    1260
   aagcccctgc cccttggagt gcctgatgct gggatgaagc ccagttaa                 1308
<210> 126
   <211> 435
   <212> PRT
   <213> Homo sapiens
<400> 126
   Met Ala Pro Val Ala Val Trp Ala Ala Leu Ala Val Gly Leu Glu Leu
   1               5                   10                  15
   Trp Ala Ala Ala His Ala Leu Pro Ala Gln Val Ala Phe Thr Pro Tyr
               20                  25                  30
   Ala Pro Glu Pro Gly Ser Thr Cys Arg Leu Arg Glu Tyr Tyr Asp Gln
           35                  40                  45
   Thr Ala Gln Met Cys Cys Ser Lys Cys Ser Pro Gly Gln His Ala Lys
       50                  55                  60
   Val Phe Cys Thr Lys Thr Ser Asp Thr Val Cys Asp Ser Cys Glu Asp
   65                  70                  75                  80
   Ser Thr Tyr Thr Gln Leu Trp Asn Trp Val Pro Glu Cys Leu Ser Cys
                   85                  90                  95
   Gly Ser Arg Cys Ser Ser Asp Gln Val Glu Thr Gln Ala Cys Thr Arg
               100                 105                 110
   Glu Gln Asn Arg Ile Cys Thr Cys Arg Pro Gly Trp Tyr Cys Ala Leu
           115                 120                 125
   Ser Lys Gln Glu Gly Cys Arg Leu Cys Ala Pro Leu Arg Lys Cys Arg
       130                 135                 140
   Pro Gly Phe Gly Val Ala Arg Pro Gly Thr Glu Thr Ser Asp Val Val
   145                 150                 155                 160
   Cys Lys Pro Cys Ala Pro Gly Thr Phe Ser Asn Thr Thr Ser Ser Thr
                   165                 170                 175
   Asp Ile Cys Arg Pro His Gln Ile Cys Asn Val Val Ala Ile Pro Gly
               180                 185                 190
   Asn Ala Ser Met Asp Ala Val Cys Thr Ser Thr Ser Pro Thr Arg Ser
           195                 200                 205
   Met Ala Pro Gly Ala Val His Leu Pro Gln Pro Val Ser Thr Arg Ser
       210                 215                 220
   Gln His Thr Gln Pro Thr Pro Glu Pro Ser Thr Ala Pro Ser Thr Ser
   225                 230                 235                 240
   Phe Leu Leu Pro Met Gly Pro Ser Pro Pro Ala Glu Gly Ser Thr Gly
                   245                 250                 255
   Asp Phe Ala Leu Pro Val Glu Lys Pro Leu Cys Leu Gln Arg Glu Ala
               260                 265                 270
   Lys Val Pro His Leu Pro Ala Asp Lys Ala Arg Gly Thr Gln Gly Pro
           275                 280                 285
   Glu Gln Gln His Leu Leu Ile Thr Ala Pro Ser Ser Ser Ser Ser Ser
       290                 295                 300
   Leu Glu Ser Ser Ala Ser Ala Leu Asp Arg Arg Ala Pro Thr Arg Asn
   305                 310                 315                 320
   Gln Pro Gln Ala Pro Gly Val Glu Ala Ser Gly Ala Gly Glu Ala Arg
                   325                 330                 335
   Ala Ser Thr Gly Ser Ser Asp Ser Ser Pro Gly Gly His Gly Thr Gln
               340                 345                 350
   Val Asn Val Thr Cys Ile Val Asn Val Cys Ser Ser Ser Asp His Ser
           355                 360                 365
   Ser Gln Cys Ser Ser Gln Ala Ser Ser Thr Met Gly Asp Thr Asp Ser
       370                 375                 380
   Ser Pro Ser Glu Ser Pro Lys Asp Glu Gln Val Pro Phe Ser Lys Glu
   385                 390                 395                 400
   Glu Cys Ala Phe Arg Ser Gln Leu Glu Thr Pro Glu Thr Leu Leu Gly
                   405                 410                 415
   Ser Thr Glu Glu Lys Pro Leu Pro Leu Gly Val Pro Asp Ala Gly Met
               420                 425                 430
   Lys Pro Ser
           435
<210> 127
   <211> 1347
   <212> DNA
   <213> Mus sp.
<400> 127
   atggcgcccg ccgccctctg ggtcgcgctg gtcttcgaac tgcagctgtg ggccaccggg     60
   cacacagtgc ccgcccaggt tgtcttgaca ccctacaaac cggaacctgg gtacgagtgc    120
   cagatctcac aggaatacta tgacaggaag gctcagatgt gctgtgctaa gtgtcctcct    180
   ggccaatatg tgaaacattt ctgcaacaag acctcggaca ccgtgtgtgc ggactgtgag    240
   gcaagcatgt atacccaggt ctggaaccag tttcgtacat gtttgagctg cagttcttcc    300
   tgtaccactg accaggtgga gatccgcgcc tgcactaaac agcagaaccg agtgtgtgct    360
   tgcgaagctg gcaggtactg cgccttgaaa acccattctg gcagctgtcg acagtgcatg    420
   aggctgagca agtgcggccc tggcttcgga gtggccagtt caagagcccc aaatggaaat    480
   gtgctatgca aggcctgtgc cccagggacg ttctctgaca ccacatcatc cactgatgtg    540
   tgcaggcccc accgcatctg tagcatcctg gctattcccg gaaatgcaag cacagatgca    600
   gtctgtgcgc ccgagtcccc aactctaagt gccatcccaa ggacactcta cgtatctcag    660
   ccagagccca caagatccca acccctggat caagagccag ggcccagcca aactccaagc    720
   atccttacat cgttgggttc aacccccatt attgaacaaa gtaccaaggg tggcatctct    780
   cttccaattg agaagccctc ctgcctacaa agagatgcca aggtgcctca tgtgcctgat    840
   gagaaatccc aggatgcagt aggccttgag cagcagcacc tgttgaccac agcacccagt    900
   tccagcagca gctccctaga gagctcagcc agcgctgggg accgaagggc gccccctggg    960
   ggccatcccc aagcaagagt catggcggag gcccaagggt ttcaggaggc ccgtgccagc   1020
   tccaggattt cagattcttc ccacggaagc cacgggaccc acgtcaacgt cacctgcatc   1080
   gtgaacgtct gtagcagctc tgaccacagt tctcagtgct cttcccaagc cagcgccaca   1140
   gtgggagacc cagatgccaa gccctcagcg tccccaaagg atgagcaggt ccccttctct   1200
   caggaggagt gtccgtctca gtccccgtgt gagactacag agacactgca gagccatgag   1260
   aagcccttgc cccttggtgt gccggatatg ggcatgaagc ccagccaagc tggctggttt   1320
   gatcagattg cagtcaaagt ggcctga                                       1347
<210> 128
   <211> 448
   <212> PRT
   <213> Mus sp.
<400> 128
   Met Ala Pro Ala Ala Leu Trp Val Ala Leu Val Phe Glu Leu Gln Leu
   1               5                   10                  15
   Trp Ala Thr Gly His Thr Val Pro Ala Gln Val Val Leu Thr Pro Tyr
               20                  25                  30
   Lys Pro Glu Pro Gly Tyr Glu Cys Gln Ile Ser Gln Glu Tyr Tyr Asp
           35                  40                  45
   Arg Lys Ala Gln Met Cys Cys Ala Lys Cys Pro Pro Gly Gln Tyr Val
       50                  55                  60
   Lys His Phe Cys Asn Lys Thr Ser Asp Thr Val Cys Ala Asp Cys Glu
   65                  70                  75                  80
   Ala Ser Met Tyr Thr Gln Val Trp Asn Gln Phe Arg Thr Cys Leu Ser
                   85                  90                  95
   Cys Ser Ser Ser Cys Thr Thr Asp Gln Val Glu Ile Arg Ala Cys Thr
               100                 105                 110
   Lys Gln Gln Asn Arg Val Cys Ala Cys Glu Ala Gly Arg Tyr Cys Ala
           115                 120                 125
   Leu Lys Thr His Ser Gly Ser Cys Arg Gln Cys Met Arg Leu Ser Lys
       130                 135                 140
   Cys Gly Pro Gly Phe Gly Val Ala Ser Ser Arg Ala Pro Asn Gly Asn
   145                 150                 155                 160
   Val Leu Cys Lys Ala Cys Ala Pro Gly Thr Phe Ser Asp Thr Thr Ser
                   165                 170                 175
   Ser Thr Asp Val Cys Arg Pro His Arg Ile Cys Ser Ile Leu Ala Ile
               180                 185                 190
   Pro Gly Asn Ala Ser Thr Asp Ala Val Cys Ala Pro Glu Ser Pro Thr
               195                 200                 205
   Leu Ser Ala Ile Pro Arg Thr Leu Tyr Val Ser Gln Pro Glu Pro Thr
       210                 215                 220
   Arg Ser Gln Pro Leu Asp Gln Glu Pro Gly Pro Ser Gln Thr Pro Ser
   225                 230                 235                 240
   Ile Leu Thr Ser Leu Gly Ser Thr Pro Ile Ile Glu Gln Ser Thr Lys
                   245                 250                 255
   Gly Gly Ile Ser Leu Pro Ile Glu Lys Pro Ser Cys Leu Gln Arg Asp
               260                 265                 270
   Ala Lys Val Pro His Val Pro Asp Glu Lys Ser Gln Asp Ala Val Gly
           275                 280                 285
   Leu Glu Gln Gln His Leu Leu Thr Thr Ala Pro Ser Ser Ser Ser Ser
       290                 295                 300
   Ser Leu Glu Ser Ser Ala Ser Ala Gly Asp Arg Arg Ala Pro Pro Gly
   305                 310                 315                 320
   Gly His Pro Gln Ala Arg Val Met Ala Glu Ala Gln Gly Phe Gln Glu
                   325                 330                 335
   Ala Arg Ala Ser Ser Arg Ile Ser Asp Ser Ser His Gly Ser His Gly
               340                 345                 350
   Thr His Val Asn Val Thr Cys Ile Val Asn Val Cys Ser Ser Ser Asp
           355                 360                 365
   His Ser Ser Gln Cys Ser Ser Gln Ala Ser Ala Thr Val Gly Asp Pro
       370                 375                 380
   Asp Ala Lys Pro Ser Ala Ser Pro Lys Asp Glu Gln Val Pro Phe Ser
   385                 390                 395                 400
   Gln Glu Glu Cys Pro Ser Gln Ser Pro Cys Glu Thr Thr Glu Thr Leu
                   405                 410                 415
   Gln Ser His Glu Lys Pro Leu Pro Leu Gly Val Pro Asp Met Gly Met
               420                 425                 430
   Lys Pro Ser Gln Ala Gly Trp Phe Asp Gln Ile Ala Val Lys Val Ala
           435                 440                 445
<210> 129
   <211> 178
   <212> DNA
   <213> Homo sapiens
<400> 129
   acatttgagt ttgttttctg tagctgtctg agcttctctt ttctttctag gactgattgt     60
   gggtgtgaca gccttgggtc tactaataat aggagtggtg aactgtgtca tcatgaccca    120
   ggtgaaaagt aagagtccat ccttccttcc ttcatccact tgttcaggaa gcttttgt      178
<210> 130
   <211> 16
   <212> DNA
   <213> Artificial Sequence
<220>
   <223> Description of Artificial Sequence: Synthetic
   oligonucleotide
<400> 130
   ccacaatcag tcctag 16
<210> 131
   <211> 14
   <212> DNA
   <213> Artificial Sequence
<220>
   <223> Description of Artificial Sequence: Synthetic
   oligonucleotide
<400> 131
   acaatcagtc ctag 14
<210> 132
   <211> 12
   <212> DNA
   <213> Artificial Sequence
<220>
   <223> Description of Artificial Sequence: Synthetic
   oligonucleotide
<400> 132
   aatcagtcct ag 12
<210> 133
   <211> 10
   <212> DNA
   <213> Artificial Sequence
<220>
   <223> Description of Artificial Sequence: Synthetic oligonucleotide
<400> 133
   tcagtcctag 10
<210> 134
   <211> 14
   <212> DNA
   <213> Artificial Sequence
<220>
   <223> Description of Artificial Sequence: Synthetic
   oligonucleotide
<400> 134
   ccacaatcag tcct 14
<210> 135
   <211> 12
   <212> DNA
   <213> Artificial Sequence
<220>
   <223> Description of Artificial Sequence: Synthetic
   oligonucleotide
<400> 135
   ccacaatcag tc 12
<210> 136
   <211> 10
   <212> DNA
   <213> Artificial Sequence
<220>
   <223> Description of Artificial Sequence: Synthetic
   oligonucleotide
<400> 136
   ccacaatcag 10
<210> 137
   <211> 14
   <212> DNA
   <213> Artificial Sequence
<220>
   <223> Description of Artificial Sequence: Synthetic
   oligonucleotide
<400> 137
   cacaatcagt ccta 14
<210> 138
   <211> 12
   <212> DNA
   <213> Artificial Sequence
<220>
   <223> Description of Artificial Sequence: Synthetic
   oligonucleotide
<400> 138
   cacaatcagt cc 12
<210> 139
   <211> 12
   <212> DNA
   <213> Artificial Sequence
<220>
   <223> Description of Artificial Sequence: Synthetic
   oligonucleotide
<400> 139
   acaatcagtc ct 12
<210> 140
   <211> 12
   <212> DNA
   <213> Artificial Sequence
<220>
   <223> Description of Artificial Sequence: Synthetic
   oligonucleotide
<400> 140
   caatcagtcc ta 12
<210> 141
   <211> 10
   <212> DNA
   <213> Artificial Sequence
<220>
   <223> Description of Artificial Sequence: Synthetic
   oligonucleotide
<400> 141
   cacaatcagt 10
<210> 142
   <211> 10
   <212> DNA
   <213> Artificial Sequence
<220>
   <223> Description of Artificial Sequence: Synthetic
   oligonucleotide
<400> 142
   acaatcagtc 10
<210> 143
   <211> 10
   <212> DNA
   <213> Artificial Sequence
<220>
   <223> Description of Artificial Sequence: Synthetic
   oligonucleotide
<400> 143
   caatcagtcc 10
<210> 144
   <211> 10
   <212> DNA
   <213> Artificial Sequence
<220>
   <223> Description of Artificial Sequence: Synthetic
   oligonucleotide
<400> 144
   aatcagtcct 10
<210> 145
   <211> 10
   <212> DNA
   <213> Artificial Sequence
<220>
   <223> Description of Artificial Sequence: Synthetic
   oligonucleotide
<400> 145
   atcagtccta 10
<210> 146
   <211> 16
   <212> DNA
   <213> Artificial Sequence
<220>
   <223> Description of Artificial Sequence: Synthetic
   oligonucleotide
<400> 146
   cagtcctaga aagaaa 16
<210> 147
   <211> 14
   <212> DNA
   <213> Artificial Sequence
<220>
   <223> Description of Artificial Sequence: Synthetic
   oligonucleotide
<400> 147
   gtcctagaaa gaaa 14
<210> 148
   <211> 12
   <212> DNA
   <213> Artificial Sequence
<220>
   <223> Description of Artificial Sequence: Synthetic
   oligonucleotide
<400> 148
   cctagaaaga aa 12
<210> 149
   <211> 10
   <212> DNA
   <213> Artificial Sequence
<220>
   <223> Description of Artificial Sequence: Synthetic
   oligonucleotide
<400> 149
   tagaaagaaa 10
<210> 150
   <211> 14
   <212> DNA
   <213> Artificial Sequence
<220>
   <223> Description of Artificial Sequence: Synthetic
   oligonucleotide
<400> 150
   cagtcctaga aaga 14
<210> 151
   <211> 12
   <212> DNA
   <213> Artificial Sequence
<220>
   <223> Description of Artificial Sequence: Synthetic
   oligonucleotide
<400> 151
   cagtcctaga aa 12
<210> 152
   <211> 10
   <212> DNA
   <213> Artificial Sequence
<220>
   <223> Description of Artificial Sequence: Synthetic
   oligonucleotide
<400> 152
   cagtcctaga 10
<210> 153
   <211> 14
   <212> DNA
   <213> Artificial Sequence
<220>
   <223> Description of Artificial Sequence: Synthetic
   oligonucleotide
<400> 153
   agtcctagaa agaa 14
<210> 154
   <211> 12
   <212> DNA
   <213> Artificial Sequence
<220>
   <223> Description of Artificial Sequence: Synthetic
   oligonucleotide
<400> 154
   agtcctagaa ag 12
<210> 155
   <211> 12
   <212> DNA
   <213> Artificial Sequence
<220>
   <223> Description of Artificial Sequence: Synthetic
   oligonucleotide
<400> 155
   gtcctagaaa ga 12
<210> 156
   <211> 12
   <212> DNA
   <213> Artificial Sequence
<220>
   <223> Description of Artificial Sequence: Synthetic
   oligonucleotide
<400> 156
   tcctagaaag aa 12
<210> 157
   <211> 10
   <212> DNA
   <213> Artificial Sequence
<220>
   <223> Description of Artificial Sequence: Synthetic
   oligonucleotide
<400> 157
   agtcctagaa 10
<210> 158
   <211> 10
   <212> DNA
   <213> Artificial Sequence
<220>
   <223> Description of Artificial Sequence: Synthetic
   oligonucleotide
<400> 158
   gtcctagaaa 10
<210> 159
   <211> 10
   <212> DNA
   <213> Artificial Sequence
<220>
   <223> Description of Artificial Sequence: Synthetic
   oligonucleotide
<400> 159
   tcctagaaag 10
<210> 160
   <211> 10
   <212> DNA
   <213> Artificial Sequence
<220>
   <223> Description of Artificial Sequence: Synthetic
   oligonucleotide
<400> 160
   cctagaaaga 10
<210> 161
   <211> 10
   <212> DNA
   <213> Artificial Sequence
<220>
   <223> Description of Artificial Sequence: Synthetic
   oligonucleotide
<400> 161
   ctagaaagaa 10
<210> 162
   <211> 16
   <212> DNA
   <213> Artificial Sequence
<220>
   <223> Description of Artificial Sequence: Synthetic
   oligonucleotide
<400> 162
   acttttcacc tgggtc 16
<210> 163
   <211> 14
   <212> DNA
   <213> Artificial Sequence
<220>
   <223> Description of Artificial Sequence: Synthetic
   oligonucleotide
<400> 163
   ttttcacctg ggtc 14
<210> 164
   <211> 12
   <212> DNA
   <213> Artificial Sequence
<220>
   <223> Description of Artificial Sequence: Synthetic
   oligonucleotide
<400> 164
   ttcacctggg tc 12
<210> 165
   <211> 10
   <212> DNA
   <213> Artificial Sequence
<220>
   <223> Description of Artificial Sequence: Synthetic
   oligonucleotide
<400> 165
   cacctgggtc 10
<210> 166
   <211> 14
   <212> DNA
   <213> Artificial Sequence
<220>
   <223> Description of Artificial Sequence: Synthetic
   oligonucleotide
<400> 166
   acttttcacc tggg 14
<210> 167
   <211> 12
   <212> DNA
   <213> Artificial Sequence
<220>
   <223> Description of Artificial Sequence: Synthetic
   oligonucleotide
<400> 167
   acttttcacc tg 12
<210> 168
   <211> 10
   <212> DNA
   <213> Artificial Sequence
<220>
   <223> Description of Artificial Sequence: Synthetic
   oligonucleotide
<400> 168
   acttttcacc 10
<210> 169
   <211> 14
   <212> DNA
   <213> Artificial Sequence
<220>
   <223> Description of Artificial Sequence: Synthetic
   oligonucleotide
<400> 169
   cttttcacct gggt 14
<210> 170
   <211> 12
   <212> DNA
   <213> Artificial Sequence
<220>
   <223> Description of Artificial Sequence: Synthetic
   oligonucleotide
<400> 170
   cttttcacct gg 12
<210> 171
   <211> 12
   <212> DNA
   <213> Artificial Sequence
<220>
   <223> Description of Artificial Sequence: Synthetic
   oligonucleotide
<400> 171
   ttttcacctg gg 12
<210> 172
   <211> 12
   <212> DNA
   <213> Artificial Sequence
<220>
   <223> Description of Artificial Sequence: Synthetic
   oligonucleotide
<400> 172
   tttcacctgg gt 12
<210> 173
   <211> 10
   <212> DNA
   <213> Artificial Sequence
<220>
   <223> Description of Artificial Sequence: Synthetic
   oligonucleotide
<400> 173
   cttttcacct 10
<210> 174
   <211> 10
   <212> DNA
   <213> Artificial Sequence
<220>
   <223> Description of Artificial Sequence: Synthetic
   oligonucleotide
<400> 174
   ttttcacctg 10
<210> 175
   <211> 10
   <212> DNA
   <213> Artificial Sequence
<220>
   <223> Description of Artificial Sequence: Synthetic
   oligonucleotide
<400> 175
   tttcacctgg 10
<210> 176
   <211> 10
   <212> DNA
   <213> Artificial Sequence
<220>
   <223> Description of Artificial Sequence: Synthetic
   oligonucleotide
<400> 176
   ttcacctggg 10
<210> 177
   <211> 10
   <212> DNA
   <213> Artificial Sequence
<220>
   <223> Description of Artificial Sequence: Synthetic
   oligonucleotide
<400> 177
   tcacctgggt 10
<210> 178
   <211> 16
   <212> DNA
   <213> Artificial Sequence
<220>
   <223> Description of Artificial Sequence: Synthetic
   oligonucleotide
<400> 178
   agagcagaac cttact 16
<210> 179
   <211> 10
   <212> DNA
   <213> Artificial Sequence
<220>
   <223> Description of DNA/RNA hybrid: Synthetic oligonculeotide
<220>
   <223> Description of Artificial Sequence: Synthetic
   oligonucleotide
<400> 179
   gaacctuact 10
<210> 180
   <211> 10
   <212> DNA
   <213> Artificial Sequence
<220>
   <223> Description of Artificial Sequence: Synthetic
   oligonucleotide
<400> 180
   agagcagaac 10
<210> 181
   <211> 10
   <212> DNA
   <213> Artificial Sequence
<220>
   <223> Description of Artificial Sequence: Synthetic
   oligonucleotide
<400> 181
   gagcagaacc 10
<210> 182
   <211> 10
   <212> DNA
   <213> Artificial Sequence
<220>
   <223> Description of Artificial Sequence: Synthetic
   oligonucleotide
<400> 182
   agcagaacct 10
<210> 183
   <211> 10
   <212> DNA
   <213> Artificial Sequence
<220>
   <223> Description of DNA/RNA hybrid: Synthetic oligonculeotide
<220>
   <223> Description of Artificial Sequence: Synthetic
   oligonucleotide
<400> 183
   gcagaaccut 10
<210> 184
   <211> 10
   <212> DNA
   <213> Artificial Sequence
<220>
   <223> Description of DNA/RNA hybrid: Synthetic oligonculeotide
<220>
   <223> Description of Artificial Sequence: Synthetic
   oligonucleotide
<400> 184
   cagaacctua 10
<210> 185
   <211> 10
   <212> DNA
   <213> Artificial Sequence
<220>
   <223> Description of DNA/RNA hybrid: Synthetic oligonculeotide
<220>
   <223> Description of Artificial Sequence: Synthetic
   oligonucleotide
<400> 185
   agaaccutac 10
<211> 16
   <212> DNA
   <213> Artificial Sequence
<220>
   <223> Description of Artificial Sequence: Synthetic
   oligonucleotide
<400> 186
   ccactcctat tattag 16
<210> 187
   <211> 16
   <212> DNA
   <213> Artificial Sequence
<220>
   <223> Description of Artificial Sequence: Synthetic
   oligonucleotide
<400> 187
   caccactcct attatt 16
<210> 188
   <211> 16
   <212> DNA
   <213> Artificial Sequence
<220>
   <223> Description of Artificial Sequence: Synthetic
   oligonucleotide
<400> 188
   tggactctta cttttc 16
<210> 189
   <211> 16
   <212> DNA
   <213> Artificial Sequence
<220>
   <223> Description of Artificial Sequence: Synthetic
   oligonucleotide
<400> 189
   aaggatggac tcttac 16
<210> 190
   <211> 21
   <212> DNA
   <213> Artificial Sequence
<220>
   <223> Description of Artificial Sequence: Synthetic
   oligonucleotide
<400> 190
   actgggcttc atcccagcat c 21
<210> 191
   <211> 25
   <212> DNA
   <213> Artificial Sequence
<220>
   <223> Description of Artificial Sequence: Synthetic
   oligonucleotide
<400> 191
   caccatggcg cccgtcgccg tctgg 25
<210> 192
   <211> 40
   <212> DNA
   <213> Artificial Sequence
<220>
   <223> Description of Artificial Sequence: Synthetic
   oligonucleotide
<400> 192
   cgacttcgct cttccagttg agaagccctt gtgcctgcag 40
<210> 193
   <211> 24
   <212> DNA
   <213> Artificial Sequence
<220>
   <223> Description of Artificial Sequence: Synthetic
   oligonucleotide
<400> 193
   ttaactgggc ttcatcccag catc 24
<210> 194
   <211> 40
   <212> DNA
   <213> Artificial Sequence
<220>
   <223> Description of Artificial Sequence: Synthetic
   oligonucleotide
<400> 194
   ctgcaggcac aagggcttct caactggaag agcgaagtcg 40
<210> 195
   <211> 21
   <212> DNA
   <213> Artificial Sequence
<220>
   <223> Description of Artificial Sequence: Synthetic
   oligonucleotide
<400> 195
   ttaactgggc ttcatcccag c 21
<210> 196
   <211> 32
   <212> DNA
   <213> Artificial Sequence
<220>
   <223> Description of Artificial Sequence: Synthetic
   oligonucleotide
<400> 196
   cgatagaatt catggcgccc gtcgccgtct gg 32
<210> 197
   <211> 32
   <212> DNA
   <213> Artificial Sequence
<220>
   <223> Description of Artificial Sequence: Synthetic
   oligonucleotide
<400> 197
   cctaactcga gttaactggg cttcatccca gc 32
<210> 198
   <211> 39
   <212> DNA
   <213> Artificial Sequence
<220>
   <223> Description of Artificial Sequence: Synthetic
   oligonucleotide
<400> 198
   gactgagcgg ccgccaccat ggcgcccgtc gccgtctgg 39
<210> 199
   <211> 38
   <212> DNA
   <213> Artificial Sequence
<220>
   <223> Description of Artificial Sequence: Synthetic
   oligonucleotide
<400> 199
   ctaagcgcgg ccgcttaact gggcttcatc ccagcatc 38
<210> 200
   <211> 20
   <212> DNA
   <213> Artificial Sequence
<220>
   <223> Description of Artificial Sequence: Synthetic
   oligonucleotide
<400> 200
   cgttctccaa cacgacttca 20
<210> 201
   <211> 21
   <212> DNA
   <213> Artificial Sequence
<220>
   <223> Description of Artificial Sequence: Synthetic
   oligonucleotide
<400> 201
   cttatcggca ggcaagtgag g 21
<210> 202
   <211> 24
   <212> DNA
   <213> Artificial Sequence
<220>
   <223> Description of Artificial Sequence: Synthetic
   oligonucleotide
<400> 202
   actgaaacat cagacgtggt gtgc 24
<210> 203
   <211> 21
   <212> DNA
   <213> Artificial Sequence
<220>
   <223> Description of Artificial Sequence: Synthetic
   oligonucleotide
<400> 203
   ccttatcggc aggcaagtga g 21
<210> 204
   <211> 22
   <212> DNA
   <213> Artificial Sequence
<220>
   <223> Description of Artificial Sequence: Synthetic
   oligonucleotide
<400> 204
   cctcatctga gaagactggg cg 22
<210> 205
   <211> 25
   <212> DNA
   <213> Artificial Sequence
<220>
   <223> Description of Artificial Sequence: Synthetic
   oligonucleotide
<400> 205
   gccaccatgg gcctctccac cgtgc 25
<210> 206
   <211> 38
   <212> DNA
   <213> Artificial Sequence
<220>
   <223> Description of Artificial Sequence: Synthetic
   oligonucleotide
<400> 206
   gggcactgag gactcagttt gtgggaaatc gacacctg 38
<210> 207
   <211> 38
   <212> DNA
   <213> Artificial Sequence
<220>
   <223> Description of Artificial Sequence: Synthetic
   oligonucleotide
<400> 207
   caggtgtcga tttcccacaa actgagtcct cagtgccc 38
<210> 208
   <211> 23
   <212> DNA
   <213> Artificial Sequence
<220>
   <223> Description of Artificial Sequence: Synthetic
   oligonucleotide
<400> 208
   caccatgggc ctctccaccg tgc 23
<210> 209
   <211> 17
   <212> DNA
   <213> Artificial Sequence
<220>
   <223> Description of Artificial Sequence: Synthetic
   oligonucleotide
<400> 209
   tctgagaaga ctgggcg 17
<210> 210
   <211> 30
   <212> DNA
   <213> Artificial Sequence
<220>
   <223> Description of Artificial Sequence: Synthetic
   oligonucleotide
<400> 210
   cgataggatc catgggcctc tccaccgtgc 30
<210> 211
   <211> 31
   <212> DNA
   <213> Artificial Sequence
<220>
   <223> Description of Artificial Sequence: Synthetic
   oligonucleotide
<400> 211
   cctaactcga gtcatctgag aagactgggc g 31
<210> 212
   <211> 37
   <212> DNA
   <213> Artificial Sequence
<220>
   <223> Description of Artificial Sequence: Synthetic oligonucleotide
<400> 212
   gactgagcgg ccgccaccat gggcctctcc accgtgc 37
<210> 213
   <211> 34
   <212> DNA
   <213> Artificial Sequence
<220>
   <223> Description of Artificial Sequence: Synthetic oligonucleotide
<400> 213
   ctaagcgcgg ccgctcatct gagaagactg ggcg 34
<210> 214
   <211> 20
   <212> DNA
   <213> Artificial Sequence
<220>
   <223> Description of Artificial Sequence: Synthetic oligonucleotide
<400> 214
   ggtcaggcca ctttgactgc 20
<210> 215
   <211> 19
   <212> DNA
   <213> Artificial Sequence
<220>
   <223> Description of Artificial Sequence: Synthetic oligonucleotide
<400> 215
   caccgctgcc cctatggcg 19
<210> 216
   <211> 19
   <212> DNA
   <213> Artificial Sequence
<220>
   <223> Description of Artificial Sequence: Synthetic oligonucleotide
<400> 216
   caccgctgcc actatggcg 19
<210> 217
   <211> 24
   <212> DNA
   <213> Artificial Sequence
<220>
   <223> Description of Artificial Sequence: Synthetic
   oligonucleotide
<400> 217
   ggtcaggcca ctttgactgc aatc 24
<210> 218
   <211> 27
   <212> DNA
   <213> Artificial Sequence
<220>
   <223> Description of Artificial Sequence: Synthetic
   oligonucleotide
<400> 218
   gccaccatgg cgcccgccgc cctctgg 27
<210> 219
   <211> 41
   <212> DNA
   <213> Artificial Sequence
<220>
   <223> Description of Artificial Sequence: Synthetic
   oligonucleotide
<400> 219
   ggcatctctc ttccaattga gaagccctcc tgcctacaaa g 41
<210> 220
   <211> 41
   <212> DNA
   <213> Artificial Sequence
<220>
   <223> Description of Artificial Sequence: Synthetic
   oligonucleotide
<400> 220
   ctttgtaggc aggagggctt ctcaattgga agagagatgc c 41
<210> 221
   <211> 21
   <212> DNA
   <213> Artificial Sequence
<220>
   <223> Description of Artificial Sequence: Synthetic
   oligonucleotide
<400> 221
   ggccactttg actgcaatct g 21
<210> 222
   <211> 25
   <212> DNA
   <213> Artificial Sequence
<220>
   <223> Description of Artificial Sequence: Synthetic
   oligonucleotide
<400> 222
   caccatggcg cccgccgccc tctgg 25
<210> 223
   <211> 22
   <212> DNA
   <213> Artificial Sequence
<220>
   <223> Description of Artificial Sequence: Synthetic
   oligonucleotide
<400> 223
   tcaggccact ttgactgcaa tc 22
<210> 224
   <211> 32
   <212> DNA
   <213> Artificial Sequence
<220>
   <223> Description of Artificial Sequence: Synthetic
   oligonucleotide
<400> 224
   cgatagaatt catggcgccc gccgccctct gg 32
<210> 225
   <211> 33
   <212> DNA
   <213> Artificial Sequence
<220>
   <223> Description of Artificial Sequence: Synthetic
   oligonucleotide
<400> 225
   cctaactcga gtcaggccac tttgactgca atc 33
<210> 226
   <211> 39
   <212> DNA
   <213> Artificial Sequence
<220>
   <223> Description of Artificial Sequence: Synthetic
   oligonucleotide
<400> 226
   gactgagcgg ccgccaccat ggcgcccgcc gccctctgg 39
<210> 227
   <211> 36
   <212> DNA
   <213> Artificial Sequence
<220>
   <223> Description of Artificial Sequence: Synthetic
   oligonucleotide
<400> 227
   ctaagcgcgg ccgctcaggc cactttgact gcaatc 36
<210> 228
   <211> 21
   <212> DNA
   <213> Artificial Sequence
<220>
   <223> Description of Artificial Sequence: Synthetic
   oligonucleotide
<400> 228
   gagccccaaa tggaaatgtg c 21
<210> 229
   <211> 20
   <212> DNA
   <213> Artificial Sequence
<220>
   <223> Description of Artificial Sequence: Synthetic
   oligonucleotide
<400> 229
   gctcaaggcc tactgcatcc 20
<210> 230
   <211> 22
   <212> DNA
   <213> Artificial Sequence
<220>
   <223> Description of Artificial Sequence: Synthetic
   oligonucleotide
<400> 230
   ggttatcgcg ggaggcgggt cg 22
<210> 231
   <211> 26
   <212> DNA
   <213> Artificial Sequence
<220>
   <223> Description of Artificial Sequence: Synthetic
   oligonucleotide
<400> 231
   gccaccatgg gtctccccac cgtgcc 26
<210> 232
   <211> 40
   <212> DNA
   <213> Artificial Sequence
<220>
   <223> Description of Artificial Sequence: Synthetic
   oligonucleotide
<400> 232
   cacaaacccc caggactcag tttgtaggga tcccgtgcct 40
<210> 233
   <211> 40
   <212> DNA
   <213> Artificial Sequence
<220>
   <223> Description of Artificial Sequence: Synthetic
   oligonucleotide
<400> 233
   aggcacggga tccctacaaa ctgagtcctg ggggtttgtg 40
<210> 234
   <211> 24
   <212> DNA
   <213> Artificial Sequence
<220>
   <223> Description of Artificial Sequence: Synthetic
   oligonucleotide
<400> 234
   caccatgggt ctccccaccg tgcc 24
<210> 235
   <211> 20
   <212> DNA
   <213> Artificial Sequence
<220>
   <223> Description of Artificial Sequence: Synthetic
   oligonucleotide
<400> 235
   tcgcgggagg cgggtcgtgg 20
<210> 236
   <211> 31
   <212> DNA
   <213> Artificial Sequence
<220>
   <223> Description of Artificial Sequence: Synthetic
   oligonucleotide
<400> 236
   cgatagtcga catgggtctc cccaccgtgc c 31
<210> 237
   <211> 31
   <212> DNA
   <213> Artificial Sequence
<220>
   <223> Description of Artificial Sequence: Synthetic
   oligonucleotide
<400> 237
   cctaagaatt cttatcgcgg gaggcgggtc g 31
<210> 238
   <211> 38
   <212> DNA
   <213> Artificial Sequence
<220>
   <223> Description of Artificial Sequence: Synthetic
   oligonucleotide
<400> 238
   gactgagcgg ccgccaccat gggtctcccc accgtgcc 38
<210> 239
   <211> 34
   <212> DNA
   <213> Artificial Sequence
<220>
   <223> Description of Artificial Sequence: Synthetic
   oligonucleotide
<400> 239
   ctaagcgcgg ccgcttatcg cgggaggcgg gtcg 34
<210> 240
   <211> 1347
   <212> DNA
   <213> Mus sp.
<400> 240
   atggcgcccg ccgccctctg ggtcgcgctg gtcttcgaac tgcagctgtg ggccaccggg      60
   cacacagtgc ccgcccaggt tgtcttgaca ccctacaaac cggaacctgg gtacgagtgc     120
   cagatctcac aggaatacta tgacaggaag gctcagatgt gctgtgctaa gtgtcctcct     180
   ggccaatatg tgaaacattt ctgcaacaag acctcggaca ccgtgtgtgc ggactgtgag     240
   gcaagcatgt atacccaggt ctggaaccag tttcgtacat gtttgagctg cagttcttcc     300
   tgtagcactg accaggtgga gacccgcgcc tgcactaaac agcagaaccg agtgtgtgct     360
   tgcgaagctg gcaggtactg cgccttgaaa acccattctg gcagctgtcg acagtgcatg     420
   aggctgagca agtgcggccc tggcttcgga gtggccagtt caagagcccc aaatggaaat     480
   gtgctatgca aggcctgtgc cccagggacg ttctctgaca ccacatcatc cacagatgtg     540
   tgcaggcccc accgcatctg tagcatcctg gctattcccg gaaatgcaag cacagatgca     600
   gtctgtgcgc ccgagtcccc aactctaagt gccatcccaa ggacactcta cgtatctcag     660
   ccagagccca caagatccca acccctggat caagagccag ggcccagcca aactccaagc     720
   atccttacat cgttgggttc aacccccatt attgaacaaa gtaccaaggg tggcatctct     780
   cttccaattg agaagccctc ctgcctacaa agagatgcca aggtgcctca tgtgcctgat     840
   gagaaatccc aggatgcagt aggccttgag cagcagcacc tgttgactac agcacccagt     900
   tccagcagca gctccctaga gagctcagcc agcgctgggg atcgaagggc gccccctggg     960
   ggccatcccc aagcaagagt catggcggag gcccaagggt ctcaggaggc ccgcgccagc    1020
   tccaggattt cagattcttc ccacggaagc cacgggaccc acgtcaacgt cacctgcatc    1080
   gtgaacgtct gtagcagctc tgaccacagc tctcagtgct cttcccaagc cagcgccacg    1140
   gtgggagacc cagatgccaa gccctcagcg tccccaaagg atgagcaggt ccccttctct    1200
   caggaggagt gtccgtctca gtccccgtat gagactacag agacactgca gagccatgag    1260
   aagcccttgc cccttggtgt gccagatatg ggcatgaagc ccagccaagc tggctggttt    1320
   gatcagattg cagtcaaagt ggcctga                                        1347
<210> 241
   <211> 1308
   <212> DNA
   <213> Homo sapiens
<400> 241
   atggcgcccg tcgccgtctg ggccgcgctg gccgtcggac tggagctctg ggctgcggcg 60
   cacgccttgc ccgcccaggt ggcatttaca ccctacgccc cggagcccgg gagcacatgc 120
   cggctcagag aatactatga ccagacagct cagatgtgct gcagcaaatg ctcgccgggc 180
   caacatgcaa aagtcttctg taccaagacc tcggacaccg tgtgtgactc ctgtgaggac 240
   agcacataca cccagctctg gaactgggtt cccgagtgct tgagctgtgg ctcccgctgt 300
   agctctgacc aggtggaaac tcaagcctgc actcgggaac agaaccgcat ctgcacctgc 360
   aggcccggct ggtactgcgc gctgagcaag caggaggggt gccggctgtg cgcgccgctg 420
   cgcaagtgcc gcccgggctt cggcgtggcc agaccaggaa ctgaaacatc agacgtggtg 480
   tgcaagccct gtgccccggg gacgttctcc aacacgactt catccacgga tatttgcagg 540
   ccccaccaga tctgtaacgt ggtggccatc cctgggaatg caagcatgga tgcagtctgc 600
   acgtccacgt cccccacccg gagtatggcc ccaggggcag tacacttacc ccagccagtg 660
   tccacacgat cccaacacac gcagccaact ccagaaccca gcactgctcc aagcacctcc 720
   ttcctgctcc caatgggccc cagcccccca gctgaaggga gcactggcga cttcgctctt 780
   ccagttgaga agcccttgtg cctgcagaga gaagccaagg tgcctcactt gcctgccgat 840
   aaggcccggg gtacacaggg ccccgagcag cagcacctgc tgatcacagc gccgagctcc 900
   agcagcagct ccctggagag ctcggccagt gcgttggaca gaagggcgcc cactcggaac 960
   cagccacagg caccaggcgt ggaggccagt ggggccgggg aggcccgggc cagcaccggg 1020
   agctcagatt cttcccctgg tggccatggg acccgggtca atgtcacctg catcgtgaac 1080
   gtctgtagca gctctgacca cagctcacag tgctcctccc aagccagctc cacaatggga 1140
   gacacagatt ccagcccctc ggagtccccg aaggacgagc aggtcccctt ctccaaggag 1200
   gaatgtgcct ttcggtcaca gctggagacg ccagagaccc tgctggggag caccgaagag 1260
   aagcccctgc cccttggagt gcctgatgct gggatgaagc ccagttaa 1308
<210> 242
   <211> 144
   <212> DNA
   <213> Artificial Sequence
<220>
   <223> Description of Artificial Sequence: Synthetic nucleotide
<400> 242
   aagggtcaag acaattctgc agatatccag cacagtggcg gccgctcgag tctagagggc 60
   ccgcggttcg aaggtaagcc tatccctaac cctctcctcg gtctcgattc tacgcgtacc 120
   ggtcatcatc accatcacca ttga 144
<210> 243
   <211> 16
   <212> DNA
   <213> artificial
<220>
   <223> 16mer LNA Oligomer compound
<220>
   <221> Phosphorothioate linkage
   <222> (1)..(15)
<220>
   <221> Position 1 = LNA and every other thereafter = LNA
   <222> (1)..(16)
<220>
   <221> 5'methyl-cytosine LNA at positions 1, 5 and 9
   <222> (1)..(16)
<400> 243
   caatcagacc taggaa 16
<210> 244
   <211> 16
   <212> DNA
   <213> artificial
<220>
   <223> 16mer LNA Oligomer compound
<220>
   <221> Phosphorothioate linkage
   <222> (1)..(15)
<220>
   <221> Position 1 = LNA and every other thereafter = LNA
   <222> (1)..(15)
<220>
   <221> 5'methyl-cytosine LNA at positions 1 and 13
   <222> (1)..(13)
<400> 244
   ccacaatcag tcctag 16
<210> 245
   <211> 14
   <212> DNA
   <213> artificial
<220>
   <223> 14mer LNA oligomer compound
<220>
   <221> Phosphorothioate linkage
   <222> (1)..(13)
<220>
   <221> Position 1 = LNA and every other thereafter = LNA
   <222> (1)..(13)
<220>
   <221> 5'methyl-cytosine LNA at positions 1, 3, 7, and 11
   <222> (1)..(11)
<400> 245
   cacaatcagt ccta 14
<210> 246
   <211> 12
   <212> DNA
   <213> artificial
<220>
   <223> 12mer LNA oligomer compound
<220>
   <221> Phosphorothioate linkage
   <222> (1)..(11)
<220>
   <221> Position 1 = LNA and every other thereafter = LNA
   <222> (1)..(12)
<220>
   <221> 5'methyl-cytosine LNA
   <222> (11)..(11)
<400> 246
   acaatcagtc ct 12
<210> 247
   <211> 214
   <212> DNA
   <213> homo sapiens
<400> 247
   tcctccctca cccccaccag ctccctctcc ctcccaaagc ccccactcac caatggagta     60
   gagcttggac ttccaccgtt ggtagcgata cattaaacca atgaagagga gggataaaag    120
   gcaaagacca aagaaaatga ccaggggcaa cagcactgtg gtgcctgcag acaaagcagg    180
   tgttggtcag aggagcgggc agaggggggc cgca                                 214
<210> 248
   <211> 129
   <212> DNA
   <213> homo sapiens
<400> 248
   ataaggaatg gtcagggaca tttgggagta actctctcat ttcatctcac ctctttttca 60
   ggtgtcgatt tcccacaaac tgaggaaaaa gaaagaaagc atcataaatt tcacttcctc 120
   tctcagccc 129
<210> 249
   <211> 178
   <212> DNA
   <213> homo sapiens
<400> 249
   acaaaagctt cctgaacaag tggatgaagg aaggaaggat ggactcttac ttttcacctg 60
   ggtcatgatg acacagttca ccactcctat tattagtaga cccaaggctg tcacacccac 120
   aatcagtcct agaaagaaaa gagaagctca gacagctaca gaaaacaaac tcaaatgt 178
<210> 250
   <211> 135
   <212> DNA
   <213> Homo sapiens
<400> 250
   ccgggaagga gaccagggga agagggggag agggcagtgg agacactcac caccttggct 60
   tctctctgca ggcacaaggg cttctctata aagaggagag gggacaagta agagattgac 120
   ttcctcaggc acatc
<210> 251
   <211> 14
   <212> DNA
   <213> Artificial
<220>
   <223> Oligomer compound
<220>
   <221> Phosphorothioate linkage
   <222> (1)..(13)
<220>
   <221> 1st nucleobase = LNA, then every other nucleobase = LNA
   <222> (1)..(13)
<220>
   <221> All LNA cytosine = 5-methyl cytosine
   <222> (1)..(14)
<400> 251
   acaatcagtc ctag 14
<210> 252
   <211> 12
   <212> DNA
   <213> Artificial
<220>
   <223> Oligomer compound
<220>
   <221> Phosphorothioate linkage
   <222> (1)..(11)
<220>
   <221> 1st nucleobase = LNA, then every other nucleobase = LNA
   <222> (1)..(11)
<220>
   <221> All LNA cytosine = 5-methyl cytosine
   <222> (1)..(12)
<400> 252
   aatcagtcct ag 12
<210> 253
   <211> 10
   <212> DNA
   <213> Artificial
<220>
   <223> Oligomer compound
<220>
   <221> Phosphorothioate linkage
   <222> (1)..(9)
<220>
   <221> 1st nucleobase = LNA, then every other nucleobase = LNA
   <222> (1)..(9)
<220>
   <221> All LNA cytosine = 5-methyl cytosine
   <222> (1)..(10)
<400> 253
   tcagtcctag 10
<210> 254
   <211> 14
   <212> DNA
   <213> Artificial
<220>
   <223> Oligomer compound
<220>
   <221> Phosphorothioate linkage
   <222> (1)..(13)
<220>
   <221> 1st nucleobase = LNA, then every other nucleobase = LNA
   <222> (1)..(13)
<220>
   <221> All LNA cytosine = 5-methyl cytosine
   <222> (1)..(14)
<400> 254
   ccacaatcag tcct 14
<210> 255
   <211> 12
   <212> DNA
   <213> Artificial
<220>
   <223> Oligomer compound
<220>
   <221> Phosphorothioate linkage
   <222> (1)..(11)
<220>
   <221> 1st nucleobase = LNA, then every other nucleobase = LNA
   <222> (1)..(11)
<220>
   <221> All LNA cytosine = 5-methyl cytosine
   <222> (1)..(12)
<400> 255
   ccacaatcag tc 12
<210> 256
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 256
   ccacaatcag 10
<210> 257
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 257
   cacaatcagt cc 12
<210> 258
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 258
   caatcagtcc ta 12
<210> 259
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 259
   cacaatcagt 10
<210> 260
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 260
   acaatcagtc 10
<210> 261
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 261
   caatcagtcc 10
<210> 262
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 262
   aatcagtcct 10
<210> 263
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 263
   atcagtccta 10
<210> 264
   <211> 16
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(16)
<400> 264
   ccacaatcag tcctag 16
<210> 265
   <211> 14
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(14)
<400> 265
   gtcctagaaa gaaa 14
<210> 266
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 266
   cctagaaaga aa 12
<210> 267
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 267
   tagaaagaaa 10
<210> 268
   <211> 14
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(14)
<400> 268
   cagtcctaga aaga 14
<210> 269
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 269
   cagtcctaga aa 12
<210> 270
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 270
   cagtcctaga 10
<210> 271
   <211> 14
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(14)
<400> 271
   agtcctagaa agaa 14
<210> 272
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 272
   agtcctagaa ag 12
<210> 273
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 273
   gtcctagaaa ga 12
<210> 274
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 274
   tcctagaaag aa 12
<210> 275
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 275
   agtcctagaa 10
<210> 276
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 276
   gtcctagaaa 10
<210> 277
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 277
   tcctagaaag 10
<210> 278
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 278
   cctagaaaga 10
<210> 279
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 279
   ctagaaagaa 10
<210> 280
   <211> 16
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(16)
<400> 280
   ccacaatcag tcctag 16
<210> 281
   <211> 14
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(14)
<400> 281
   ttttcacctg ggtc 14
<210> 282
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 282
   ttcacctggg tc 12
<210> 283
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 283
   cacctgggtc 10
<210> 284
   <211> 14
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(14)
<400> 284
   acttttcacc tggg 14
<210> 285
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 285
   acttttcacc tg 12
<210> 286
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 286
   acttttcacc 10
<210> 287
   <211> 14
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(14)
<400> 287
   cttttcacct gggt 14
<210> 288
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 288
   cttttcacct gg 12
<210> 289
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 289
   ttttcacctg gg 12
<210> 290
   <211> 12
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(12)
<400> 290
   tttcacctgg gt 12
<210> 291
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 291
   cttttcacct 10
<210> 292
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 292
   ttttcacctg 10
<210> 293
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 293
   tttcacctgg 10
<210> 294
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 294
   ttcacctggg 10
<210> 295
   <211> 10
   <212> DNA
   <213> Artificial
<220>
   <223> Oligomer compound
<220>
   <221> Phosphorothioate linkages between nucleobases, 1st nucleobase = LNA, then every other nucleobase = LNA, All LNA cytosine = 5-methyl cytosine
   <222> (1)..(10)
<400> 295
   tcacctgggt 10

## Description

### FIELD OF THE INVENTION

The present disclosure relates to compositions and methods for preparing splice variants of TNFalpha receptor (TNFR) *in vivo* or *in vitro,* and the resulting TNFR protein variants. Such variants may be prepared by controlling the splicing of pre-mRNA molecules and regulating protein expression with splice switching oligonucleotides or splice switching oligomers (SSOs). The preferred SSOs according to the disclosure target exon 7 or 8 of TNFR1 (TNFRSF1A) or TNFR2 (TNFRSF1B) pre-mRNA, typically resulting in the production of TNFR variants which comprise a deletion in part or the entire exon 7 or 8 respectfully. SSOs targeting exon 7 are found to result in a soluble form of the TNFR, which has thereputic benefit for treatment of inflammatory diseases. The SSO's are characterized in that they are substantially incapable or incapable of recruiting RNaseH.

### BACKGROUND OF THE INVENTION

WO2007/05889 provides a description of the background art relating to pre-mRNA splicing, the role of TNF-alpha in inflammation and inflammatory disorders, and the mediation of TNF-alpha activity via TNF1 and TNF2.

TNF-alpha is a pro-inflammatory cytokine that exists as a membrane-bound homotrimer and is released into the circulation by the protease TNF-alpha converting enzyme (TACE). TNF-alpha is introduced into the circulation as a mediator of the inflammatory response to injury and infection. TNF-alpha activity is implicated in the progression of inflammatory diseases such as rheumatoid arthritis, Crohn's disease, ulcerative colitis, psoriasis and psoriatic arthritis (Palladino, M.A., et al., 2003, Nat. Rev. Drug Discov. 2:736-46). The acute exposure to high levels of TNF-alpha, as experienced during a massive infection, results in sepsis; its symptoms include shock, hypoxia, multiple organ failure, and death. Chronic low doses of TNF-alpha can cause cachexia, a disease characterized by weight loss, dehydration and fat loss, and is associated with malignancies.

TNF-alpha activity is mediated primarily through two receptors coded by two different genes, TNFR1 and TNFR2. TNFR1 is a membrane-bound protein with a molecular weight of approximately 55 kilodaltons (kDa), while TNFR2 is a membrane-bound protein with a molecular weight of 75 kDa. The soluble extracellular domains of both receptors are shed to some extent from the cell membrane by the action of metalloproteases. Moreover, the pre-mRNA of TNFR2 undergoes alternative splicing, creating either a full length, active membrane-bound receptor (mTNFR2), or a secreted decoy receptor (sTNFR2) that lacks exons 7 and 8 which encompasses the coding sequences for the transmembrane (Lainez et al, 2004, Int. Immunol, 16:169). The sTNFR2 binds TNF-alpha but does not elicit a physiological response, thus reducing TNF-alpha activity. Although an endogenous, secreted splice variant of TNFR1 has not yet been identified, the similar gene structures of the two receptors strongly suggest the potential to produce this TNFR1 isoform.

Because of the role played by excessive activity by TNF superfamily members, it is useful to control the alternative splicing of TNFR receptors so that the amount of the secreted form is increased and the amount of the integral membrane form is decreased. The present invention provides a splice switching oligonucleotide or splice switching oligomer (SSO) to achieve this goal. SSOs are similar to antisense oligonucleotides (ASONs). However, in contrast to ASON, SSOs are able to hybridize to a target RNA without causing degradation of the target by RNase H.

SSOs have been used to modify the aberrant splicing found in certain thalassemias (U.S. Pat. No. 5,976,879 to KoIe; Lacerra, G., et al., 2000, Proc. Natl. Acad. Sci. 97:9591). Studies with the IL-5 receptor alpha-chain (IL-5Ralpha) demonstrated that SSOs directed against the membrane-spanning exon increased synthesis of the secreted form and inhibited synthesis of the integral membrane form (U.S. Pat. No. 6,210,892 to Bennett; Karras, J.G., et al., 2000, MoL Pharm, 58:380). WO00/58512 also discloses examples of redirecting the splicing of IL-5R to soluble forms (examples 25 and 30).

SSOs have been used to produce the major CD40 splice variant detected in Tone, in which deletion of exon 6, which is upstream of the transmembrane region, resulted in an altered reading frame of the protein. While the SSO resulted in the expected mRNA splice variant, the translation product of the variant mRNA appeared to be unstable because the secreted receptor could not be detected (Siwkowski, A.M., et al., 2004, Nucleic Acids Res. 32; 2695). Tone et al., PNAS, 2001, 98(4):1751-1756 predicts that the mouse splice variant lacking exon 6 would not be a stable, secreted form of CD40 (see page 1756, right hand column.

WO02/088393 discloses gapmer oligonucleotides having 2'MOE wings and a deoxy gap,, which are targeted to mouse TNFR2 - these oligonucleotides are designed to recruit RNAseH to degrade the TNFR2 mRNA (mRNA down-regulation). The SSO oligonucleotides of the present invention are designed not to recruit RNaseH, but to disrupt the processing of the TNFR pre-mRNA, resulting in stable, secreted, ligand-binding TNFR splice variants.

US2005/202531 teaches that antisense oligonucleotides may be used to alter the alternative splicing pattern of CD40, however, it does not teach or provide any guidance as to splice elements or regions of CD40 that should be targeted by SSOs or any guidance as to which sequences should be used.

### SUMMARY OF THE INVENTION

The present disclosure employs splice switching oligonucleotides or splice switching oligomers (SSOs) to control the alternative splicing of receptors from the TNFR superfamily so that the amount of a soluble, stable, secreted, ligand-binding form is increased and the amount of the integral membrane form is decreased.

The present invention provides an oligomer of 12 - 16 nucleobases in length, comprising of a contiguous nucleobase sequence which consists of SEQ ID No 246:
Aₛ^{o}CₛAₛ^{o}aₛTₛ^{o}cₛAₛ^{o}gₛTₛ^{o}cₛ^{m}Cₛ^{o}t or an oligomer 16 nucleobases in length, comprising of a contiguous nucleobase sequence which consists of SEQ ID No 244:
^{m}Cₛ^{o}CₛAₛ^{o}CₛAₛ^{o}aₛTₛ^{o}CₛAₛ^{o}gₛTₛ^{o}Cₛ^{m}Cₛ^{o}tₛAₛ^{o}g
wherein capital letters represent LNA; superscript "o" represents oxy LNA; subscript "s" represents phosphorothiate linkage; lower case letters represent DNA; ^{m}C represents 5-methylcytosine;
and wherein the LNA is beta-D-oxy LNA.

A conjugate comprising the oligomer according to the invention and at least one non-nucleotide moiety covalently attached to said oligomer.

A pharmaceutical composition comprising the oligomer according to the invention, or the conjugate according to the invention, and a pharmaceutically acceptable carrier.

An oligomer according to the invention or a conjugate according to the invention, for the treatment of an inflammatory disease or condition.

An *in vitro* method of increasing the expression of a soluble form of TNFRSF1B TNFalpha receptor in a mammalian cell which expresses said TNFalpha receptor, said method comprising administering the oligomer, the conjugate, or the pharmaceutical composition according to the invention.

Use of an oligomer or a conjugate according to the invention, in the manufacture of a medicament for the treatment of an inflammatory disease or condition.

The disclosure provides an oligomer of between 8 and 16 nucleobases in length, comprising of a contiguous nucleobase sequence which consists of between 8 and 16 nucleobases in length, wherein said contiguous nucleobase sequence is complementary to a corresponding region of contiguous nucleotides present in SEQ ID NO 1 or SEQ ID NO 2, SEQ ID NO 3, SEQ ID NO 4 and wherein said contiguous nucleobase sequence does not comprise 5 or more contiguous DNA (2'-deoxyribosnucleoside) monomer units, wherein said contiguous nucleobases sequence comprises at least one nucleotide analogue selected from the group consisting of: beta-D-oxy LNA, thio-LNA, amino-LNA and ena-LNA.

Optionally, in the above embodiment the contiguous nucleobase sequence comprises or consists of at least one further nucleotide analogue (X).

In one embodiment, the further nucleotide analogue units, are independently selected from the group consisting of: 2'-OMe-RNA units, 2'-fluoro-DNA units, 2'-MOE RNA unit, and LNA units.

In one embodiment, the oligomer or contiguous nucleobase sequence consists of between 8 and 15 nucleobases in length, such 9, 10, 11, 12, 13 or 14 nucleobases.

In one embodiment, the contiguous nucleobase sequence is identical to or is present in a nucleobase sequence or a nucleobase motif sequence selected from the group consisting of: SEQ ID NO 131 - SEQ ID No 145, SEQ ID NO 147 - SEQ ID NO 161, and SEQ ID NO 163 - 177.

In one embodiment, the oligomer is selected from the group consisting of: SEQ ID NO 245 - SEQ ID NO 246, SEQ ID NO 251 - 263, SEQ ID NO 264 - SEQ ID NO 279, and SEQ ID NO 280 - SEQ ID NO 295.

In one embodiment, said contiguous nucleobase sequence is identical to or is present in a nucleobase sequence or a nucleobase motif sequence selected from the group consisting of: SEQ ID NO 130, SEQ ID NO 146, and SEQ ID NO 162.

In one embodiment, the oligomer is selected from the group consisting of: SEQ ID NO 244, SEQ ID NO 264, and SEQ ID NO 280.

The present disclosure relates to splice switching oligonucleotides or splice switching oligomers (SSOs). The preferred SSOs according to the disclosure target exon 7 of TNFR1 (TNFRSF1A) or TNFR2 (TNFRSF1A) pre-mRNA, typically resulting in the production of TNFR variants which comprise a deletion in part or the entire exon 7 respectfully. SSOs targeting exon 7 are found to result in a soluble form of the TNFR, which has thereputic benefit for treatment of inflammatory diseases. The SSO's are characterized in that they are substantially incapable or incapable of recruiting RNaseH

The disclosure provides an oligomer of between 8 and 50, such as between 8 and 16 nucleobases in length, comprising (or consisting) of a contiguous nucleobase sequence which consists of between 8 and 50 nucleobases in length, wherein said contiguous nucleobase sequence is complementary, preferably perfectly complementary, to a corresponding region of contiguous nucleotides present in SEQ ID NO 1 or SEQ ID NO 2, SEQ ID NO 3, or SEQ ID NO 4 and wherein said contiguous nucleobase sequence does not comprise 5 or more contiguous DNA (2'-deoxyribosnucleoside) monomer units.

SEQ ID NO 1 or SEQ ID NO 2, SEQ ID NO 3, or SEQ ID NO 4 are identical to SEQ ID NO 1 or SEQ ID NO 2, SEQ ID NO 3, or SEQ ID NO 4 of PCT/US2006/043651.

SEQ ID NO 247 is the reverse complement of SEQ ID NO 1. SEQ ID NO 248 is the reverse complement of SEQ ID NO 2, SEQ ID NO 249 is the reverse complement of SEQ ID NO 3, SEQ ID NO 250 is the reverse complement of SEQ ID NO 4.

Therefore, it is preferred that the oligomer of the disclosure comprises or consists of a contiguous nucleobase sequence which is homologous (preferably 100% homologus) to a corresponding region (i.e. part of) of SEQ ID NO 247, SEQ ID NO 248, SEQ ID NO 249, or SEQ ID NO 250.

The disclosure provides an oligomer of between 8 and 50 nucleobases in length, comprising (or consisting) of a contiguous nucleobase sequence which consists of between 8 and 50 nucleobases in length, wherein said contiguous nucleobase sequence is present in a (corresponding) region of contiguous nucleotides present in SEQ ID NO 247 or SEQ ID NO 248, SEQ ID NO 249, or SEQ ID NO 250 and wherein said contiguous nucleobase sequence does not comprise 5 or more contiguous DNA (2'-deoxyribosnucleoside) monomer units.

The disclosure provides an oligomer of between 8 and 50 nucleobases in length, comprising (or consisting) of a contiguous nucleobase sequence which consists of between 8 and 50 nucleobases in length, wherein said contiguous nucleobase sequence is complementary, preferably perfectly complementary, to a corresponding region of contiguous nucleotides present in SEQ ID NO 1 or SEQ ID NO 2, SEQ ID NO 3, or SEQ ID NO 4 and wherein said oligomer is essentially incapable, or incapable, of recruiting RNAseH when formed in a duplex with a complex with a complementary mRNA molecule.

The disclosure provides an oligomer of between 8 and 50 nucleobases in length, comprising (or consisting) of a contiguous nucleobase sequence which consists of between 8 and 50 nucleobases in length, wherein said contiguous nucleobase sequence is present in a (corresponding) region of contiguous nucleotides present in SEQ ID NO 247 or SEQ ID NO 248, SEQ ID NO 249, or SEQ ID NO 250 and wherein said oligomer is essentially incapable, or incapable, of recruiting RNAseH when formed in a duplex with a complex with a complementary mRNA molecule.

The disclosure further provides for a method of altering the splicing of a TNFalpha receptor pre-mRNA mRNA, selected from TNFRSF1A or TNFRSF1A in a mammalian cell which expresses TNFRSF1A TNFalpha receptor or TNFRSF1B TNFalpha receptor, said method comprising administering an oligomer or a conjugate, or a pharmaceutical composition according to the invention to the cell.

The disclosure also refers to a method of preparing a soluble form of TNFRSF1A TNFalpha receptor or TNFRSF1B TNFalpha receptor in a mammalian cell which expresses said TNFalpha receptor, said method comprising administering an oligomer or a conjugate, or a pharmaceutical composition according to the invention to the cell.

The above methods may further comprise the step of purifying the soluble form of the TNFRSF1A TNFalpha receptor or the TNFRSF1B TNFalpha receptor.

The above methods may be performed *in vivo* or *in vitro.*

The disclosure provides for a composition for use in a method of treatment or prevention of an inflammatory disease or condition comprising the steps of administering the pharmaceutical composition according to the invention to a patient who is suffering from, or is likely to suffer from said inflammatory disease.

The disclosure provides for an isolated or purified soluble form of TNFalpha receptor comprises a deletion in the trans-membrane binding domain encoded by exon 7, wherein said TNFalpha receptor is selected from the TNFalpha receptor TNFRSF1A or TNFRSF1B.

The disclosure provides for an isolated or purified soluble form of TNFalpha receptor which lacks the trans-membrane binding domain encoded by exon 7, wherein said TNFalpha receptor is selected from the TNFalpha receptor TNFRSF1A or TNFRSF1B.

The disclosure further provides for a nucleic acid encoding the soluble form of TNFalpha receptor.

The disclosure further provides for a vector comprising the nucleic acid according to the disclosure, such as an expression vector.

The disclosure further provides for a host cell which comprises the nucleic acid or the vector according to the disclosure.

The disclosure further provides for a method for the preparation of a soluble form of TNFalpha receptor, said method comprising the step of culturing the host cell according to the disclosure under conditions which allow the expression of the nucleic acid according to the disclosure, and subsequently isolating said soluble form of TNFalpha receptor from said host cells.

The disclosure further provides for pharmaceutical composition comprising the isolated or purified soluble form of TNFalpha receptor according to the disclosure, or as prepared according to a method of the disclosure, and a pharmaceutically acceptable carrier.

The disclosure further provides for the use of the isolated or purified soluble form of TNFalpha receptor according to the disclosure, or as prepared according to a method of the disclosure, for the preparation of a medicament for the treatment of an inflammatory disease or condition.

The disclosure further provides for an isolated or purified soluble form of TNFalpha receptor according to the disclosure, or as prepared according to a method of the disclosure, for the treatment of an inflammatory disease or condition.

Related cases PCT/US2006/043651, PCT/US2007/10557, US 11/595,485, and US 11/799,117,.

### BRIEF DESCRIPTION OF THE DRAWINGS

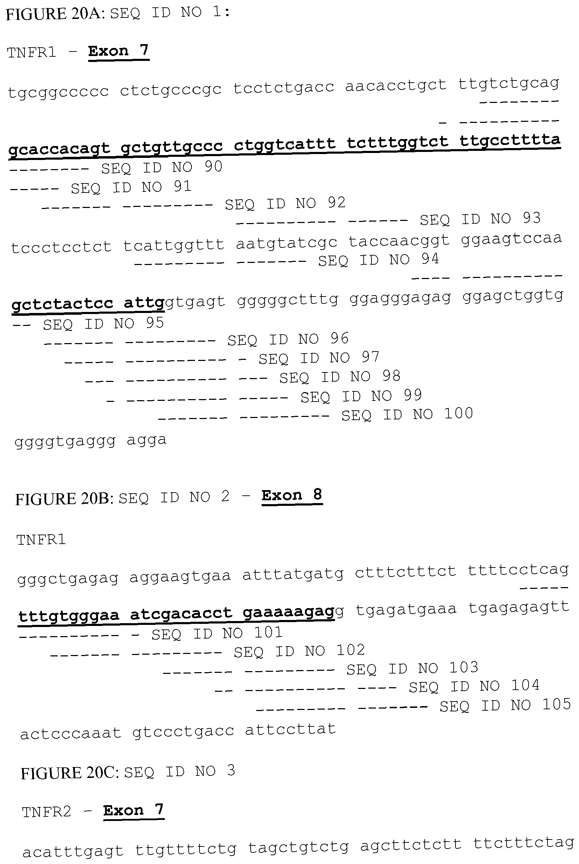

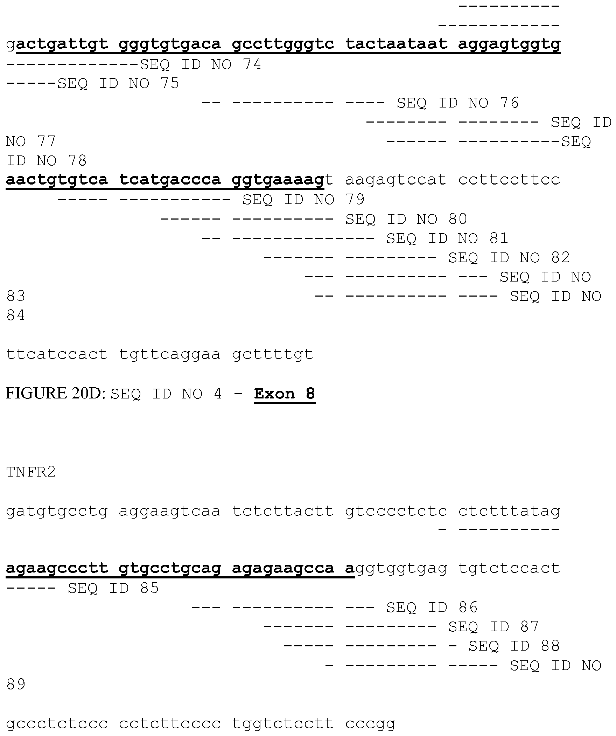

The following figures are identicial to those described in PCT/US2007/10557. Figure 20 is new to the present application.

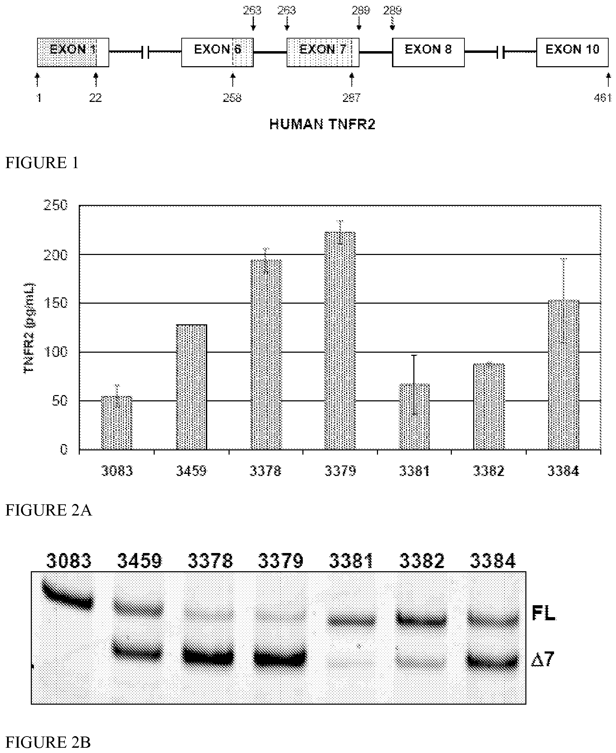

FIGURE 1 schematically depicts the human TNFR2 structure. Relevant exons and introns are represented by boxes and lines, respectively. The signal sequence and the transmembrane region are shaded. Residues that form the boundaries of the signal sequence, the transmembrane region, and the final residue are indicated below the diagram. Exon boundaries are indicated above the diagram; if the 3' end of an exon and the 5' end of the following exon have the same residue number, then the splice junction is located within the codon encoding that residue.
FIGURE 2A graphically illustrates the amount of soluble TNFR2 from SSO treated primary human hepatocytes. The indicated SSO was transfected into primary human hepatocytes at 50 nM. After ∼48 hrs, the extracellular media was analyzed by enzyme linked immunosorbant assay (ELISA) for soluble TNFR2 using the Quantikine® Human sTNF RII ELISA kit from R&D Systems (Minneapolis, MN). Error bars represent the standard deviation for 3 independent experiments.
FIGURE 2B Total RNA was analyzed for TNFR2 splice switching by RT-PCR using primers specific for human TNFR2. SSOs targeted to exon seven led to shifting from full length TNFR2 mRNA (FL) to TNFR2 Δ7 mRNA (Δ7). SSO 3083 is a control SSO with no TNFR2 splice switching ability.

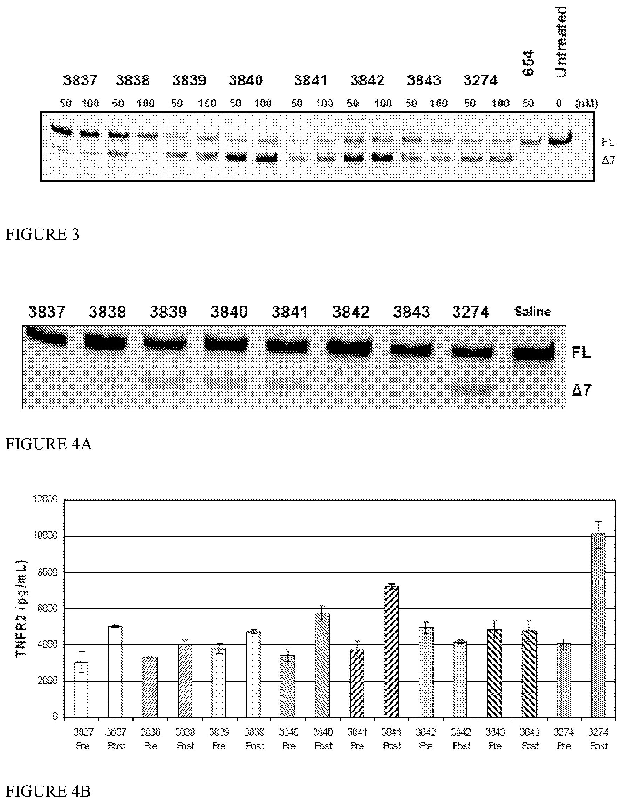

FIGURE 3 shows the splicing products of L929 cells treated with SSO 10-mers targeted to mouse TNFR2 exon 7. L929 cells were transfected with the indicated SSO concentration (50 or 100nM), and evaluated for splice switching of TNFR2 by RT-PCR 24 hrs later. PCR primers were used to amplify from Exon 5 to Exon 9, so that "Full Length" (FL) TNFR2 is represented by a 486 bp band. Transcripts lacking exon 7 (Δ7) is represented by a 408 bp band.
FIGUREs 4A and 4B show the splicing products of mice treated with SSO 10-mers targeted to mouse TNFR2 exon 7. The indicated SSOs were resuspended in saline, and injected i.p. into mice at 25mg/kg/day for 5 days. Mice were prebled before SSO injection, and 10 days after the final SSO injection and sacrificed. At the time of sacrifice, total RNA from livers was analyzed for TNFR2 splice switching by RT-PCR. FL - full length TNFR2; Δ7 - TNFR2 Δ7 (FIGURE 4A). The concentration of TNFR2 Δ7 in the serum taken before (Pre) and after (Post) SSO injection was determined by ELISA using the Quantikine® Mouse sTNF RII ELISA kit from R&D Systems (Minneapolis, MN) (FIGURE 4B). Error bars represent the standard error from 3 independent readings of the same sample.

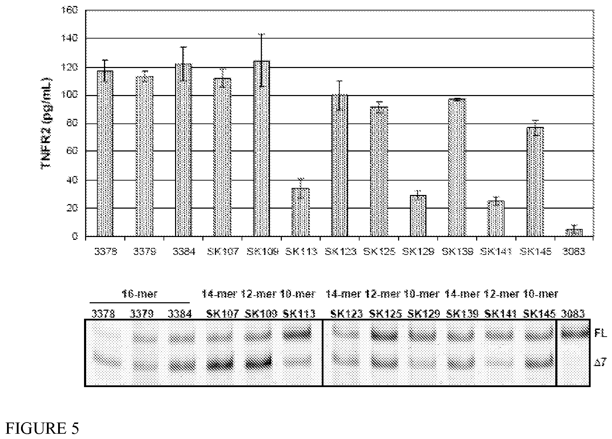

FIGURE 5 depicts the splice switching ability of SSOs of different lengths. Primary human hepatocytes were transfected with the indicated SSO and TNFR2 expression analyzed by RT-PCR (top panel) and ELISA (bottom panel) as in Figure 2. Error bars represent the standard deviation from 2 independent experiments.

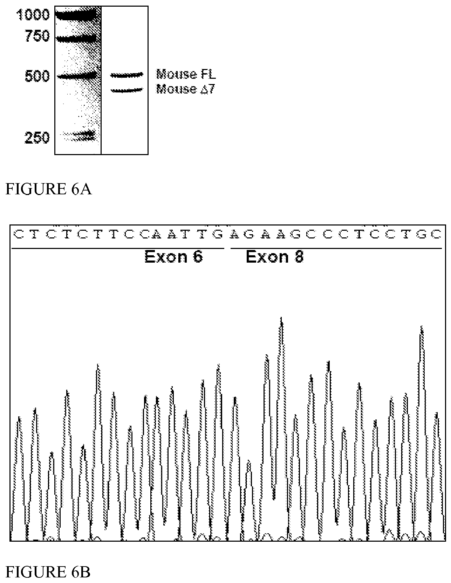

FIGUREs 6A and 26B illustrate TNFR2 Δ7 mRNA induction in the livers of SSO treated mice. Figure. 6A: Total RNA from the livers of SSO 3274 treated mice were subjected to RT-PCR, and the products visualized on a 1.5% agarose gel. The sequence of the exon 6 - exon 8 junction is shown in Figure. 6B.

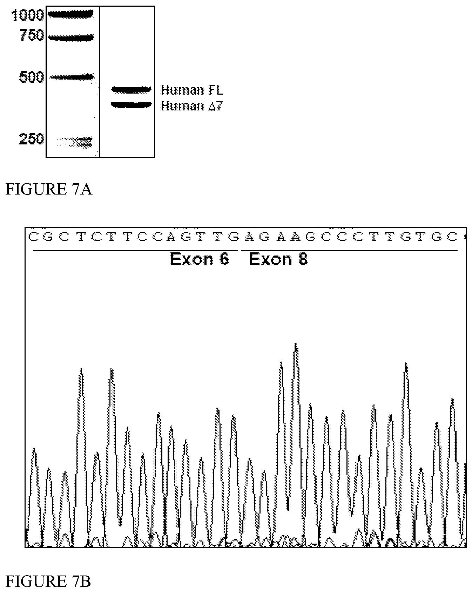

FIGUREs 7A and 7B illustrate TNFR2 Δ7 mRNA induction in SSO treated primary human hepatocytes. Figure. 7A: Total RNA from SSO 3379 treated cells were subjected to RT-PCR, and the products visualized on a 1.5% agarose gel. The sequence of the exon 6 - exon 8 junction is shown inFigure. 7B.

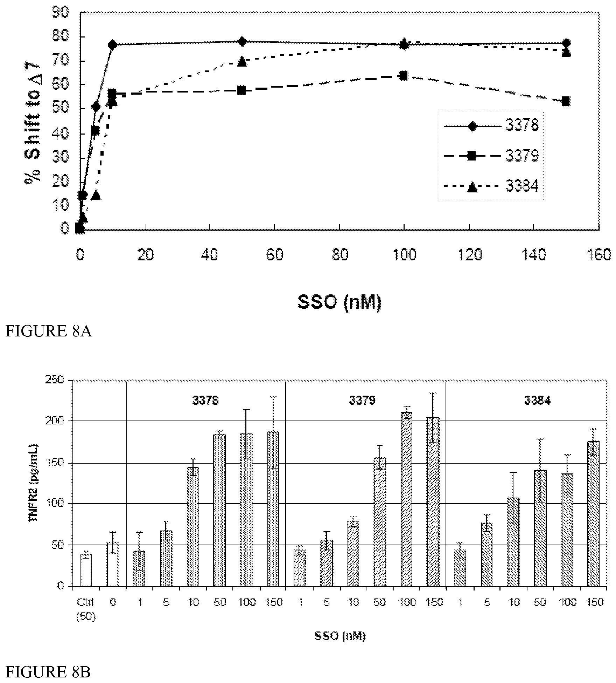

FIGUREs 8A and 8B illustrate the dose dependence of TNFR2 pre-mRNA splicing shifting by SSO 3378, 3379 and 3384. Primary human hepatocytes were transfected with 1-150nM of the indicated SSO. After ∼48 hrs, the cells were harvested for total RNA, and the extracellular media was collected.Figure. 8A: Total RNA was analyzed for TNFR2 splice switching by RT-PCR using primers specific for human TNFR2. For each SSO, amount of splice switching is plotted as a function of SSO concentration. Figure. 8B: The concentration of soluble TNFR2 in the extracellular media was determined by ELISA and plotted as a function of SSO. Error bars represent the standard deviation for at least 2 independent experiments.

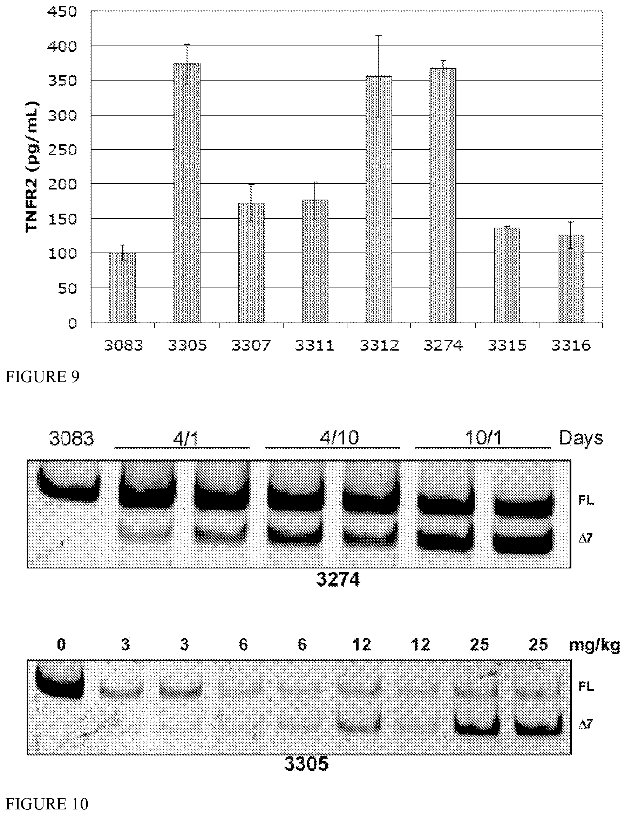

FIGURE 9 graphically illustrates detection of secreted TNFR2 splice variants from L929 cells. Cells were transfected with the indicated SSOs. After 72 hrs, the extracellular media was removed and analyzed by ELISA. The data are expressed as pg soluble TNFR2 per mL.
FIGURE 10 shows the splicing products for intraperitoneal (i.p.) injection of SSO 3274 (top) and 3305 (bottom) in mice. SSO 3274 was injected i.p. at 25 mg/kg/day for either 4 days (4/1 and 4/10) or 10 days (10/1). Mice were sacrificed either 1 day (4/1 and 10/1) or 10 days (4/10) after the last injection and total RNA from liver was analyzed by RT-PCR for TNFR2 splice switching. SSO 3305 was injected at the indicated dose per day for 4 days. Mice were sacrificed the next day and the livers analyzed as with 3274 treated animals.

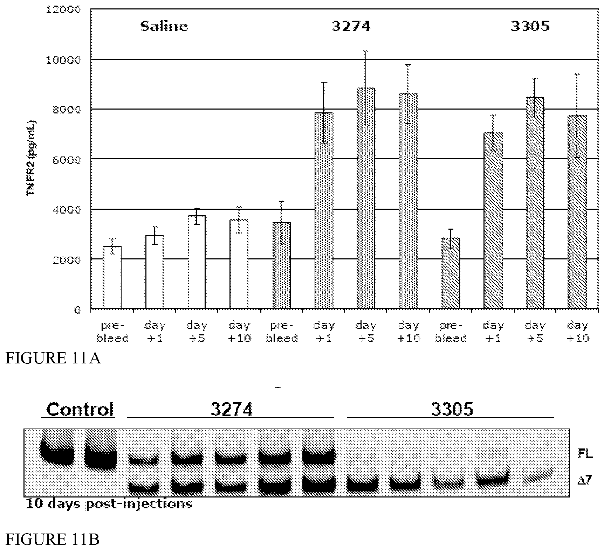

FIGURE 11A graphically illustrates the amount of soluble TNFR2 in mouse serum 10 days after SSO treatment. Mice were injected i.p. with the indicated SSO or saline (n=5 per group) at 25 mg/kg/day for 10 days. Serum was collected 4 days before injections began and on the indicated days after the last injection. Sera was analyzed by ELISA as described in Figure 22 At day 10, mice were sacrificed and livers were analyzed for TNFR2 splice switching by RT-PCR (FIGURE 11B) as described in Figure 30.

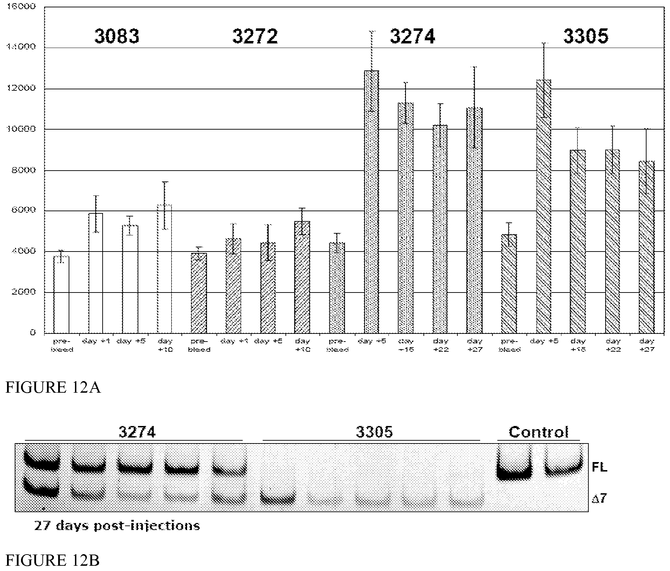

FIGURE 12A graphically illustrates the amount of soluble TNFR2 in mouse serum 27 days after SSO treatment. Mice were treated as described in Figure 11, except that serum samples were collected until day 27 after the last injection. SSOs 3083 and 3272 are control SSOs with no TNFR2 splice switching ability. At day 27, mice were sacrificed and livers were analyzed for TNFR2 splice switching by RT-PCR (FIGURE 12B) as described in Figure 11.

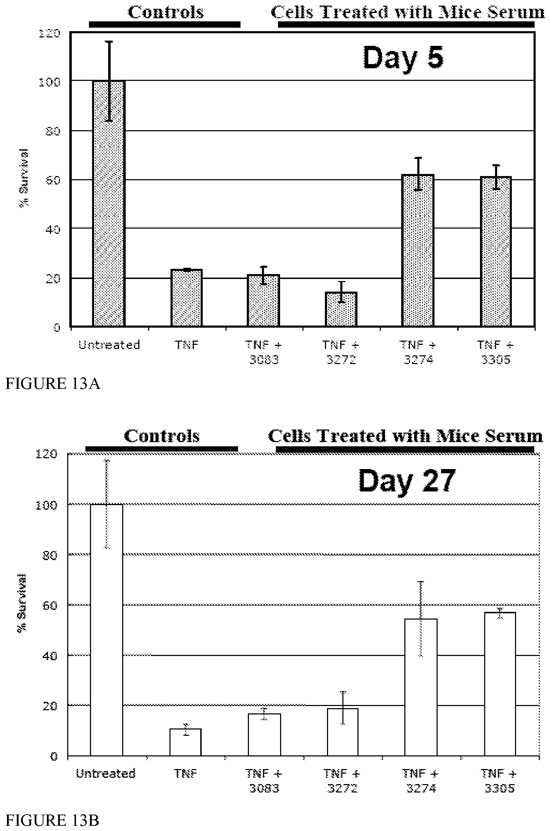

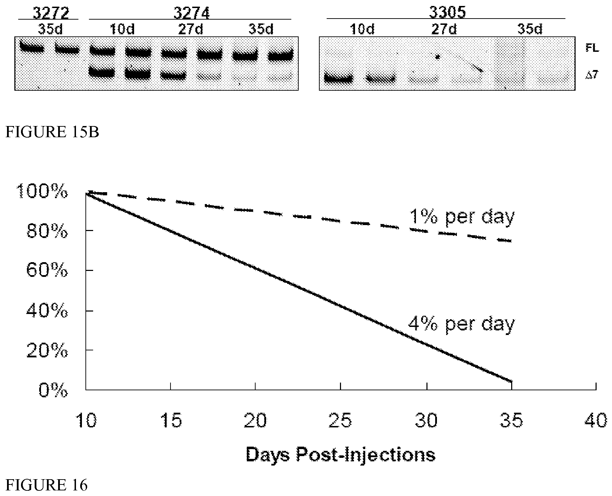

FIGUREs 13A and 13B graphically depict the anti-TNF-a activity in a cell-based assay using serum from SSO treated mice, where serum samples were collected 5 days (FIGURE 16A) and 27 days (FIGURE 16B) after SSO treatment. L929 cells were treated with either 0.1 ng/mL TNF-α, or TNF-α plus 10% serum from mice treated with the indicated SSO. Cell viability was measured 24 hrs later and normalized to untreated cells.

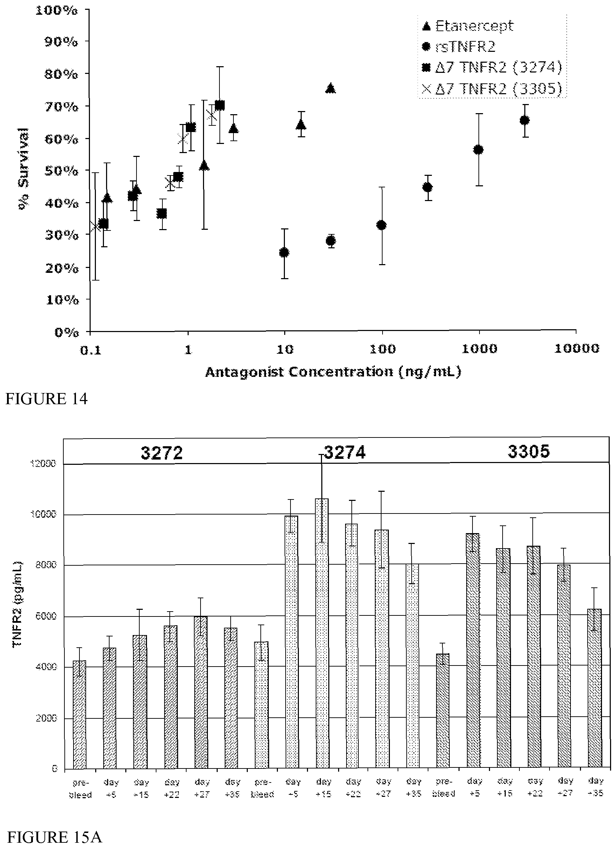

FIGURE 14 graphically compares the anti-TNF-a activity of serum from the indicated SSO oligonucleotide-treated mice to recombinant soluble TNFR2 (rsTNFR2) extracellular domain from Sigma® and to Enbrel® using the cell survival assay described in Figure 13.
FIGUREs 15A and 15B compare the stability of muTNFR2 Δ7 protein (FIGURE 15A) and mRNA (FIGURE 15B). Mice were injected at 25 mg/kg/day daily with either SSO 3272, SSO 3274 or SSO 3305 (n = 5). Mice were bled on the indicated day after the last injection and the serum TNFR2 concentration was measured. Total RNA from mice sacrificed on the indicated day after the last injection of SSO was subjected to RT-PCR as described in Figure 10.
FIGURE 16 plots TNFR2 Δ7 protein (dashed line) and mRNA (solid line) levels over time, as a percentage of the amount of protein or mRNA, respectively, 10 days after the last injection.

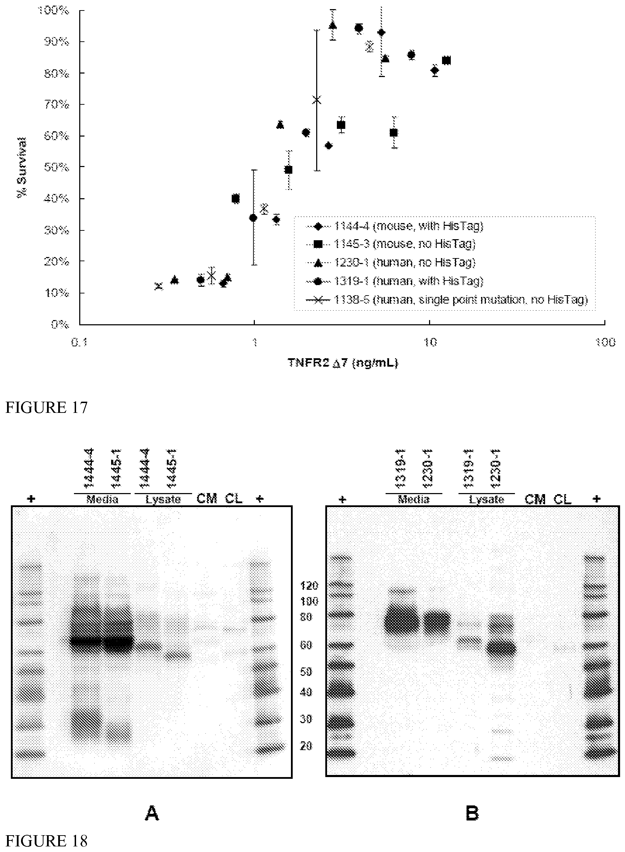

FIGURE 17 graphically illustrates the dose dependant anti-TNF-a activity of TNFR2 Δ7 expressed in HeLa cells after transfection with TNFR2 Δ7 mammalian expression plasmids. HeLa cells were transfected with the indicated mouse or human TNFR2 Δ7 plasmid and extracellular media was collected after 48 hrs. The TNFR2 Δ7 concentration in the media was determined by ELISA and serial dilutions were prepared. These dilutions were assayed for anti-TNF-a activity by the L929 cytoxicity assay as in Figure. 14.
FIGURE 18 shows expressed mouse (A) and human (B) TNFR2 D7 protein isolated by polyacrylamide gel electrophoresis (PAGE). HeLa cells were transfected with the indicated plasmid. After ∼48 hrs, the extracellular media was collected and concentrated, and cells were collected in RIPA lysis buffer. The proteins in the samples were separated by PAGE and a western blot was performed using a C-terminal TNFR2 primary antibody (Abcam) that recognizes both the human and mouse TNFR2 D7 proteins. Media, extracellular media samples from HeLa cells transfected with the indicated plasmid; Lysate, cell lysate from Hela cells transfected with the indicated plasmid. CM, control media from untransfected HeLa cells; CL, control cell lysates from untransfected HeLa cells. +, molecular weight markers (kDal).

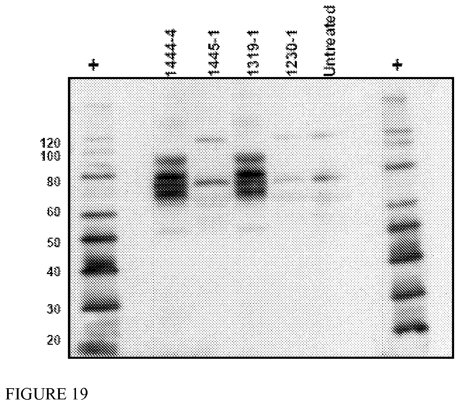

FIGURE 19 shows purified His-tagged human and mouse TNFR2 D7. Unconcentrated extracellular media containing the indicated TNFR2 D7 protein was prepared as in Figure18. Approximately 32 mL of the media was applied to a 1 mL HisPur cobalt spin column (Pierce), and bound proteins were eluted in 1 mL buffer containing 150 mM imidazole. Samples of each were analyzed by PAGE and western blot was performed as in Figure 18. The multiple bands in lanes 1144-4 and 1319-1 represent variably glycosylated forms of TNFR2 D7.
FIGURE 20 Alignment of oligomer motifs according to the disclosure compared against their target sequence - SEQ ID NO 1 (Figure 20A), SEQ ID NO 2 (Figure 20B), SEQ ID NO 3 (Figure 20C), and SEQ ID NO 4 (Figure2).

### DETAILED DESCRIPTION OF THE DISCLOSURE

The present disclosure provides compositions and methods for controlling expression of TNF receptors (TNFR1 and TNFR2) and of other cytokine receptors from the TNFR superfamily by controlling the splicing of pre-mRNA that code for the said receptors. More specifically, the increased expression of the secreted form and the decreased expression of the integral-membrane form. Furthermore, the disclosure can be used in the treatment of diseases associated with excessive cytokine activity.

The exon or exons that are present in the integral membrane form mRNA but are removed from the primary transcript (the "pre-mRNA") to make a secreted form mRNA are termed the "transmembrane exons." The invention involves nucleic acids and nucleic acid analogs that are complementary to either of the transmembrane exons and/or adjacent introns of a receptor pre-mRNA. Complementarity can be based on sequences in the sequence of pre-mRNA that spans the splice site, which would include, but is not limited to, complementarity based on sequences that span the exon-intron junction, or complementarity can be based solely on the sequence of the intron, or complementarity can be based solely on the sequence of the exon.

There are several alternative chemistries available and known to those skilled in the art. One important feature is the ability to hybridize to a target RNA without causing degradation of the target by RNase H as do 2'-deoxy oligonucleotides ("antisense oligonucleotides" hereafter "ASON"). For clarity, such compounds will be termed splice- switching oligomers (SSOs). Those skilled in the art appreciate that SSO include, but are not limited to, 2' O-modified oligonucleotides and ribonucleosidephosphorothioates as well as peptide nucleic acids and other polymers lacking ribofuranosyl-based linkages.

One embodiment of the disclosure is a method of treating an inflammatory disease or condition by administering SSOs to a patient or a live subject. The SSOs that are administered alter the splicing of a pre-mRNA to produce a splice variant that encodes a stable, secreted, ligand-binding form of a receptor of the TNFR superfamily, thereby decreasing the activity of the ligand for that receptor. In another embodiment, the disclosure is a method of producing a stable, secreted, ligand-binding form of a receptor of the TNFR superfamily in a cell by administering SSOs to the cell.

One embodiment of the disclosure provides is a protein, either full length or mature, which can bind TNF, is encoded by a cDNA derived from a mammalian TNFR gene, and in the cDNA exon 6 is followed directly by exon 8 and as a result lacks exon 7 ("TNFR δ7"). In another embodiment, the disclosure provides a pharmaceutical composition comprising a TNFR δ7. In a further embodiment, the disclosure provides a method of treating an inflammatory disease or condition by administering a pharmaceutical composition comprising a TNFR δ7.

In yet another embodiment, the disclosure provides a nucleic acid that encodes a TNFR δ7. In a further embodiment, the disclosure provides a pharmaceutical composition comprising a nucleic acid that encodes a TNFR δ7.

In another embodiment, the disclosure provides an expression vector comprising a nucleic acid that encodes a TNFR δ7. In a further embodiment, the disclosure provides a method of increasing the level of a soluble TNFR in the serum of a mammal by transforming cells of the mammal with an expression vector comprising a nucleic acid that encodes a TNFR δ7.

In another embodiment, the disclosure provides a cell transformed with an expression vector comprising a nucleic acid that encodes a TNFR δ7. In a further embodiment, the disclosure provides a method of producing a TNFR δ7 by culturing, under conditions suitable to express the TNFR δ7, a cell transformed with an expression vector comprising a nucleic acid that encodes a TNFR δ7. In yet another embodiment, the disclosure provides a method of treating an inflammatory disease or condition by administering an expression vector comprising a nucleic acid that encodes a TNFR δ7.

In yet another embodiment, splice-switching oligomers (SSOs) are disclosed that alter the splicing of a mammalian TNFR2 pre-mRNA to produce a mammalian TNFR2 protein, which can bind TNF and where exon 6 is followed directly by exon 8 and as a result lacks exon 7 ("TNFR2 57"). One embodiment of the disclosure is a method of treating an inflammatory disease or condition by administering SSOs to a patient or a live subject. The SSOs that are administered alter the splicing of a mammalian TNFR2 pre-mRNA to produce a TNFR2 δ7. In another embodiment, the disclosure provides a method of producing a TNFR2 δ7 in a cell by administering SSOs to the cell.

The foregoing and other objects and aspects of the present disclosure are discussed in detail in the drawings herein and the specification set forth below.

### The Oligomer

In one embodiment the oligomer consists of the contiguous nucleobase sequence.

However, it is also envisaged that the oligomer may comprise of other nucleobase sequence which typically flank the contiguous nucleobase sequence at either the 5' or 3' end or further nucleobase sequence at both the 5' and 3' ends. Suitably these 5' and or 3' 'flanking' regions may be 1, 2, 3, 4, 5, or 6 nucleobases in length. DNA or RNA nucleobases which are at the termini of the oligomer of the invention are expected to be cleaved from the oligomer when used in vivo by endogenous exo-nucleases - as such the includion of flanking DNA or RNA units may not affect the *in vivo* performance of the oligomer.

In one embodiment, the 3' end of the contiguous nucleobase sequence is flanked by 1, 2 or 3 DNA or RNA units. 3', DNA units are frequently used during solid state synthesis of oligomers.

In one embodiment, the 5' end of the contiguous nucleobase sequence is flanked by 1, 2 or 3 DNA or RNA units.

In one embodiment the disclosure provides an oligomer of between 8 and 50 nucleobases in length, comprising of a contiguous nucleobase sequence which consists of between 8 and 50 nucleobases in length, wherein said contiguous nucleobase sequence is complementary to a corresponding region of contiguous nucleotides present in SEQ ID NO 1 or SEQ ID NO 2, SEQ ID NO 3, or SEQ ID NO 4 (i.e. said contiguous nucleobase sequence is present in a region ('corresponding' - or part of) of contiguous nucleotides present in SEQ ID NO 247 or SEQ ID NO 248, SEQ ID NO 249, or SEQ ID NO 250) and wherein said contiguous nucleobase sequence does not comprise 5 or more contiguous DNA (2'-deoxyribosnucleoside) monomer units.

In one embodiment the oligomer is essentially incapable of recruiting RNAseH when formed in a duplex with a complex with a complementary mRNA molecule.

In one embodiment the contiguous nucleobase sequence comprises or consists of nucleotide analogues (X).

In one embodiment the nucleotide analogues (X) are independently selected form the group consisting of: 2'-O-alkyl-RNA unit, 2'-OMe-RNA unit, 2'-amino-DNA unit, 2'-fluoro-DNA unit, LNA unit, PNA unit, HNA unit, INA unit.

In one embodiment the contiguous nucleobase sequence comprises both nucleotide analogues (X) and nucleotides (x).

In one embodiment the contiguous nucleobase sequence does not comprise a region of more than 7 consecutive nucleotide analogue units (X), such as not more than 6, not more than 5, not more than 4, not more than 3, or not more than 2 consecutive nucleotide analogue units (X).

In one embodiment the 5' most nucleobase of the contiguous nucleobase sequence is a nucleotide analogue (X).

In one embodiment the 5' most nucleobase of the contiguous nucleobase sequence is a nucleotide unit (x), such as a DNA (2'-deoxyribosnucleoside) monomer unit.

In one embodiment the 3' most nucleobase of the contiguous nucleobase sequence is a nucleotide analogue (X).

In one embodiment the 3' most nucleobase of the contiguous nucleobase sequence is a nucleotide unit (x), such as a DNA (2'-deoxyribosnucleoside) monomer unit.

In one embodiment the contiguous nucleobase sequence comprises or consists of an alternating sequence of nucleotides and nucleobases.

In one embodiment the alternating sequence of nucleotides and nucleobases is an, sequence selected from the group consisting of Xx, xX, Xxx, xXx, xxX, XXx, XxX, xXX, XXXx, XXxX, XxXX, xXXX, xxxX, xxXx, xXxx, Xxxx, XXXXx, XXXxX, XXxXX, XxXXX, xXXXX, xxxxX, xxxXx, xxXxx, xXxxx, Xxxxx, wherein said alternating sequence is optionally repeated.

In one embodiment the repeated sequence is repeated for the entire length of the contiguous nucleobase sequence, wherein, optionally the 5' and/or 3' repeat may be truncated.

In one embodiment the single stranded oligonucleotide comprises said at least one LNA analogue unit and at least one further nucleotide analogue unit other than LNA.

In one embodiment the single stranded oligonucleotide consists of at least one sequence X¹X²X¹ or X²X¹X², wherein X¹ is LNA and X² is a nucleotide analogue other than LNA, such as either a 2'-OMe RNA unit and 2'-fluoro DNA unit.

In one embodiment the sequence of nucleobases of the single stranded oligonucleotide consists of alternative X¹ and X² units.

In one embodiment the nucleotide analogue units, such as X, are independently selected form the group consisting of: 2'-OMe-RNA units, 2'-fluoro-DNA units, and LNA units.

In one embodiment the nucleotide analogue units (X) are LNA units.

In one embodiment the LNA units are selected from the group consisting of oxy-LNA, amino-LNA, thio-LNA, and ena-LNA.

In one embodiment the contiguous nucleobase sequence does not comprise a contiguous sub-sequence consisting of 5 or more contiguous nucleobases independently selected from DNA and LNA units, wherein the LNA units present in the contiguous sub-sequence are in the alpha-L-configuration.

In one embodiment the contiguous nucleobase sequence does not comprise a contiguous sub-sequence consisting of 5 or more contiguous nucleobases independently selected from DNA and LNA units, wherein the LNA units present in the contiguous sub-sequence are alpha-L-oxy LNA.

In one embodiment the all the LNA units are in the beta-D configuration.

In one embodiment the contiguous nucleobases sequence consists only of LNA and DNA units.

In one embodiment the contiguous nucleobases sequence consists only of LNA and DNA units. LNA units in the beta-D configuration are preferred, such as beta-D-oxy or beta-D-thio or beta-D-amino.

In one embodiment the LNA may be selected from the group consisting of: beta-D-oxy LNA or beta-D-thio LNA or beta-D-amino LNA, ena-LNA, and optionally including the group consisting of alpha- L-oxy LNA or alpha- L-thio LNA or alpha- L-amino LNA.

In one embodiment the length of the contiguous nucleobase sequence is between 8 and 16, such as 9, 10, 11, 12, 13, 14, 15 or 16 nucleobases, in length, or between 10 - 14 or 11-14 or 12 -14.

In one embodiment the length of the contiguous nucleobase sequence is between 8 and 15, such as 8, 9, 10, 11, 12, 13, 14, or 15 nucleobases, in length.

In one embodiment the contiguous nucleobase sequence comprises a nucleobase sequence which is complementary to a corresponding region of SEQ ID NO 1 or SEQ ID NO 3, i.e is present in a (corresponding) region of contiguous nucleotides present in SEQ ID NO 247 or SEQ ID NO 249.

In one embodiment the contiguous nucleobase sequence is complementary to a corresponding region of contiguous nucleotides present in a sequence selected from the group consisting of: 51-164 of SEQ ID NO 1, 51-79 of SEQ ID NO 2, 51-127 of SEQ ID NO 3, and 51-85 of SEQ ID NO 4.

In one embodiment the contiguous nucleobase sequence is complementary to a corresponding region of contiguous nucleotides present in a sequence selected from the group consisting of: 1 - 50 of SEQ ID NO 1, 165-215 of SEQ ID NO 1, 1 - 50 of SEQ ID NO 2, 80 -130 of SEQ ID NO 2, 1 - 50 of SEQ ID NO 3, 128 - 178 of SEQ ID NO 3, 1 - 50 of SEQ ID NO 4, and 86 - 136 of SEQ ID NO 4.

In one embodiment the contiguous nucleobase sequence comprises a nucleobase sequence which is complementary to an 5' exon/intron 3' or 3' intron/exon 5' border.

In one embodiment the 5' exon/intron 3' or 3' intron/exon 5' border is selected from the group consisting of nucleobases 50-51 of SEQ ID NO 1, 164-165 of SEQ ID NO 1, 50-51 of SEQ ID NO 2, 79-80 of SEQ ID NO 2, 51-52 of SEQ ID NO 3, 129-139 of SEQ ID NO 3, 50-51 of SEQ ID NO 4, 81-82 of SEQ ID No 4.

In one embodiment the contiguous nucleobase sequence is identical to or is present in a nucleobase sequence present in a sequence selected from the group consisting of SEQ ID NO 74 to SEQ ID NO 105.

In one embodiment the contiguous nucleobase sequence is identical to or is present in a nucleobase sequence selected from the group consisting of: SEQ ID NO 74, SEQ ID NO 75, SEQ ID NO 77, SEQ ID NO 78, SEQ ID NO 80, SEQ ID NO 82, and SEQ ID NO 84.

In one embodiment the contiguous nucleobase sequence is identical to or is present in a nucleobase sequence selected from the group consisting of: SEQ ID NO 85, SEQ ID NO 86, SEQ ID NO 87, SEQ ID NO 88, and SEQ ID NO 89.

In one embodiment the oligomer is selected from the group consisting of: SEQ ID NO 74, SEQ ID NO 75, SEQ ID NO 77, SEQ ID NO 78, SEQ ID NO 80, SEQ ID NO 82, and SEQ ID NO 84.

In one embodiment the oligomer is selected from the group consisting of: SEQ ID NO 86, SEQ ID NO 87, SEQ ID NO 88, and SEQ ID NO 89.

In one embodiment the contiguous nucleobase sequence comprises a nucleobase sequence which is complementary to a region of SEQ ID No 3 selected from nucleotides: 47-49, 54 - 56, and 122-124.

In one embodiment the contiguous nucleobase sequence is identical to or is present in a nucleobase sequence or nucleobase sequence motif selected from the group consisting of: SEQ ID NO 130 - SEQ ID No 145, SEQ ID NO 146 - SEQ ID NO 161, and SEQ ID NO 162 - 177.

In one embodiment the contiguous nucleobase sequence is identical to or is present in a nucleobase sequence or nucleobase sequence motif selected from the group consisting of: SEQ ID NO 131 - SEQ ID No 145, SEQ ID NO 147 - SEQ ID NO 161, and SEQ ID NO 163 - 177.

In one embodiment the oligomer is selected from the group consisting of: SEQ ID NO 243, SEQ ID NO 244, SEQ ID NO 245 or SEQ ID NO 246.

In one embodiment the oligomer comprises at least one non-nucleotide moiety covalently attached to said oligomer.

### Splice-switching oligomers (SSOs):

In another aspect, the present invention employs splice switching oligonucleotides or splice switching oligomers (SSOs) to control the alternative splicing of TNFR2 so that the amount of a soluble, ligand-binding form that lacks exon 7 is increased and the amount of the integral membrane form is decreased. The methods and compositions of the present disclosure can be used in the treatment of diseases associated with excessive tnf activity.

Accordingly, one embodiment of the disclosure is a method of treating an inflammatory disease or condition by administering SSOs to a patient. The SSOs that are administered alter the splicing of a pre-mRNA to produce a mammalian TNFR2 protein that lacks exon 7.

In another embodiment, the disclosure provides a method of producing a mammalian TNFR2 protein that lacks exon 7 in a cell by administering SSOs to the cell.

The length of the SSO (i.e. The number of monomers in the oligomer) is similar to an antisense oligonucleotide (ASON), typically between about 8 and 30 nucleotides. In preferred embodiments, the SSO will be between about 10 to 16 nucleotides. The disclosure can be practiced with SSOs of several chemistries that hybridize to RNA, but that do not activate the destruction of the RNA by RNAseH, as do conventional antisense 2'-deoxy oligonucleotides. The disclosure can be practiced using 2'o modified nucleic acid oligomers, such as where the 2'O is replaced with -O-CH₃, -O-CH₂-CH₂-O-CH₃, -O-CH₂-CH₂-CH₂-NH₂, -O-CH₂-CH₂-CH₂-OH or -F, where 2'O-methyl or 2'O-methyloxyethyl is preferred. The nucleobases do not need to be linked to sugars; so-called peptide nucleic acid oligomers or morpholine-based oligomers can be used. A comparison of these different linking chemistries is found in Sazani, p. et al., 2001, nucleic acids res. 29:3695. The term splice-switching oligonucleotide is intended to cover the above forms. Those skilled in the art will appreciate the relationship between antisense oligonucleotide gapmers and SSOs. Gapmers are ASON that contain an RNAse H activating region (typically a 2'-deoxyribonucleoside phosphorothioate) which is flanked by non-activating nuclease resistant oligomers. In general, any chemistry suitable for the flanking sequences in a gapmer ASON can be used in an SSO.

The SSOs of this invention may be made through the well-known technique of solid phase synthesis. Any other means for such synthesis known in the art may additionally or alternatively be used. It is well known to use similar techniques to prepare oligonucleotides such as the phosphorothioates and alkylated derivatives.

The bases of the SSO may be the conventional cytosine, guanine, adenine and uracil or thymidine. Alternatively, modified bases can be used. Of particular interest are modified bases that increase binding affinity. One non-limiting example of preferred modified bases are the so-called g-clamp or 9-(aminoethoxy)phenoxazine nucleotides, cytosine analogues that form 4 hydrogen bonds with guanosine. (Flanagan, W.M., et al., 1999, proc. Natl. Acad. Sci. 96:3513; Holmes, S.C., 2003, Nucleic Acids Res. 31:2759). Specific examples of other bases include, but are not limited to, 5-methylcytosine (^{me}C), isocytosine, pseudoisocytosine, 5-bromouracil, 5-propynyluracil, 5-propyny-6, 5-methylthiazoleuracil, 6-aminopurine, 2-aminopurine, inosine, 2,6-diaminopurine, 7-propyne-7-deazaadenine, 7-propyne-7-deazaguanine and 2-chloro-6-aminopurine.

When LNA nucleotides are employed in an SSO it is preferred that non-LNA nucleotides also be present. LNA nucleotides have such high affinities of hybridization that there can be significant non-specific binding, which may reduce the effective concentration of the free-SSO. When LNA nucleotides are used they may be alternated conveniently with 2'-deoxynucleotides. Alternating nucleotides, alternating dinucleotides or mixed patterns, e.g., LDLDLD or LLDLLD or LDDLDD can be used. For example in one embodiment, contains a sequence of nucleotides selected from the group consisting of: LDLDDLLDDLDLDLL, LDLDLLLDDLLLDLL, LMLMMLLMMLMLMLL, LMLMLLLMMLLLMLL, LFLFFLLFFLFLFLL, LFLFLLLFFLLLFLL, LDDLDDLDDL, DLDDLDDLDD, DDLDDLDDLD, LMMLMMLMML, MLMMLMMLMM, MMLMMLMMLM, LFFLFFLFFL, FLFFLFFLFF, FFLFFLFFLF, DLDLDLDLDL, LDLDLDLDL, MLMLMLMLML, LMLMLMLML, FLFLFLFLFL, LFLFLFLFL, where L is a LNA unit, D is a DNA unit, M is 2'Moe, F is 2'fluoro.

When 2'-deoxynucleotides or 2'-deoxynucleoside phosphorothioates are mixed with LNA nucleotides it is important to avoid RNAse H activation. It is expected that between about one third and two thirds of the LNA nucleotides of an SSO will be suitable. When affinity-enhancing modifications are used, including but not limited to LNA or g-clamp nucleotides, the skilled person recognizes it can be necessary to increase the proportion of such affinity-enhancing modifications.

Numerous alternative chemistries which do not activate RNAse H are available. For example, suitable SSOs can be oligonucleotides wherein at least one of the internucleotide bridging phosphate residues is a modified phosphate, such as methyl phosphonate, methyl phosphonothioate, phosphoromorpholidate, phosphoropiperazidate, and phosphoroamidate. For example, every other one of the internucleotide bridging phosphate residues may be modified as described. In another non-limiting example, such SSO are oligonucleotides wherein at least one of the nucleotides contains a 2' lower alkyl moiety (e.g., c₁-c₄, linear or branched, saturated or unsaturated alkyl, such as methyl, ethyl, ethenyl, propyl, 1-propenyl, 2-propenyl, and isopropyl). For example, every other one of the nucleotides may be modified as described. (see references in u.s. pat. 5,976,879 col. 4). For *in vivo* use, phosphorothioate linkages are preferred.

The length of the SSO will be from about 8 to about 30 bases in length. Those skilled in the art appreciate that when affinity-increasing chemical modifications are used, the SSO can be shorter and still retain specificity. Those skilled in the art will further appreciate that an upper limit on the size of the SSO is imposed by the need to maintain specific recognition of the target sequence, and to avoid secondary-structure forming self hybridization of the SSO and by the limitations of gaining cell entry. These limitations imply that an SSO of increasing length (above and beyond a certain length which will depend on the affinity of the SSO) will be more frequently found to be less specific, inactive or poorly active.

SSOs of the disclosure include, but are not limited to, modifications of the SSO involving chemically linking to the SSO one or more moieties or conjugates which enhance the activity, cellular distribution or cellular uptake of the SSO. Such moieties include, but are not limited to, lipid moieties such as a cholesterol moiety, cholic acid, a thioether, e.g. Hexyl-s-tritylthiol, a thiocholesterol, an aliphatic chain, e.g., dodecandiol or undecyl residues, a phospholipids, e.g., di-hexadecyl-rac-glycerol or triethylammonium 1,2-di-o-hexadecyl-rac-glycero-3-h-phosphonate, a polyamine or a polyethylene glycol chain, an adamantane acetic acid, a palmityl moiety, an octadecylamine or hexylamino-carbonyl-oxycholesterol moiety.

It is not necessary for all positions in a given SSO to be uniformly modified, and in fact more than one of the aforementioned modifications may be incorporated in a single compound or even at a single nucleoside within an SSO.

The SSOs may be admixed, encapsulated, conjugated, or otherwise associated with other molecules, molecule structures, or mixtures of compounds, as for example liposomes, receptor targeted molecules, oral, rectal, topical or other formulation, for assisting in uptake, distribution, and/or absorption.

Those skilled in the art appreciate that cellular differentiation includes, but is not limited to, differentiation of the spliceosome. Accordingly, the activity of any particular SSO can depend upon the cell type into which they are introduced. For example, SSOs which are effective in one cell type may be ineffective in another cell type.

The methods, oligonucleotides, and formulations of the present disclosure are also useful as *in vitro* or *in vivo* tools to examine splicing in human or animal genes. Such methods can be carried out by the procedures described herein, or modifications thereof which will be apparent to skilled persons.

The SSOs disclosed herein can be used to treat any condition in which the medical practitioner intends to limit the effect of tnf or the signalling pathway activated by tnf. In particular, the invention can be used to treat an inflammatory disease. In one embodiment, the condition is an inflammatory systemic disease, e.g., rheumatoid arthritis or psoriatic arthritis. In another embodiment, the disease is an inflammatory liver disease. Examples of inflammatory liver diseases include, but are not limited to, hepatitis associated with the hepatitis a, b, or c viruses, alcoholic liver disease, and non-alcoholic steatosis. In yet another embodiment, the inflammatory disease is a skin condition such as psoriasis.

### RNAseH Recruitment

The oligomer according to the invention does not mediate RNAseH based cleavage of a complementary single stranded RNA molecule. A stretch of at least 5 consecutive DNA nucleobases are required for an oligonucleotide to be effective in recruitment of RNAseH.

EP 1 222 309 provides in vitro methods for determining RNaseH activity, which may be used to determine the ability to recruit RNaseH. A compound is deemed capable of recruiting RNase H if, when provided with the complementary RNA target, it has an initial rate, as measured in pmol/l/min, of at least 1 %, such as at least 5%, such as at least 10% or less than 20% of the equivalent DNA only oligonucleotide, with no 2' substitutions, with phosphorothiote linkage groups between all nucleotides in the oligonucleotide, using the methodology provided by Example 91 - 95 of EP 1 222 309.

A compound is deemed essentially incapable of recruiting RNaseH if, when provided with the complementary RNA target, and RNaseH, the RNaseH initial rate, as measured in pmol/l/min, is less than 20% such as less than 10% such as less than 5%, or preferably less than 1%, (or even less than 0.1%) of the initial rate determined using the equivalent DNA only oligonucleotide, with no 2' substitutions, with phosphorothiote linkage groups between all nucleotides in the oligonucleotide, using the methodology provided by Example 91 - 95 of EP 1 222 309.

### Nucleotide Analogues

It will be recognised that when referring to a preferred nucleotide sequence motif or nucleotide sequence, which consists of only nucleotides, the oligomers of the invention which are defined by that sequence may comprise a corresponding nucleotide analogues in place of one or more of the nucleotides present in said sequence, such as LNA units or other nucleotide analogues, which raise the duplex stability/Tₘ of the oligomer/target duplex (*i.e.* affinity enhancing nucleotide analogues).

Furthermore, the nucleotide analogues may enhance the stability of the oligomer *in vivo.*

In one embodiment, the nucleotide analogues (X) are independently selected form the group consisting of: 2'-O-alkyl-RNA unit, 2'-OMe-RNA unit, 2' MOE RNA unit, 2'-amino-DNA unit, 2'-fluoro-DNA unit, LNA unit, PNA unit, HNA unit, INA unit.

In one embodiment, the contiguous nucleobases sequence does not comprise 2'OMe ribonucleotide analogues or 2'-MOE ribonucleotide analogues..

In one embodiment the nucleotide analogue is 2'-MOE, *i.e.* 2'O-2methoxyethyl RNA. Therefore in one embodiment X² or M as referred to in nucleobases motifsherein may be 2'-MOE.

Incorporation of affinity-enhancing nucleotide analogues in the oligomer, such as LNA or 2'-substituted sugars, can allow the size of the specifically binding oligomer to be reduced, and may also reduce the upper limit to the size of the oligomer before non-specific or aberrant binding takes place.

Suitably, when the nucleobase sequence of the oligomer, or the contiguous nucleobase sequence, is not fully complementary to the corresponding region of the TNFR target sequence, in one embodiment, when the oligomer comprises affinity enhancing nucleotide analogues, such nucleotide analogues form a complement with their corresponding nucleotide in the TNFR target.

The oligomer may thus comprise or consist of a simple sequence of natural nucleotides - preferably 2'-deoxynucleotides (referred to here generally as "DNA"), but also possibly ribonucleotides (referred to here generally as "RNA") - or it could comprise one or more (and possibly consist completely of) nucleotide "analogues".

Nucleotide "analogues" are variants of natural DNA or RNA nucleotides by virtue of modifications in the sugar and/or base and/or phosphate portions. The term "nucleobase" will be used to encompass natural (DNA- or RNA-type) nucleotides as well as such "analogues" thereof. Analogues could in principle be merely "silent" or "equivalent" to the natural nucleotides in the context of the oligonucleotide, *i.e.* have no functional effect on the way the oligonucleotide works to inhibit beta-catenin expression. Such "equivalent" analogues may nevertheless be useful if, for example, they are easier or cheaper to manufacture, or are more stable to storage or manufacturing conditions, or represent a tag or label. Preferably, however, the analogues will have a functional effect on the way in which the oligomer works to inhibit expression; for example by producing increased binding affinity to the target and/or increased resistance to intracellular nucleases and/or increased ease of transport into the cell.

Examples of such modification of the nucleotide include modifying the sugar moiety to provide a 2'-substituent group or to produce a bridged (locked nucleic acid) structure which enhances binding affinity and probably also provides some increased nuclease resistance; modifying the internucleotide linkage from its normal phosphodiester to one that is more resistant to nuclease attack, such as phosphorothioate or boranophosphate.

A preferred nucleotide analogue is LNA, such as beta-D-oxy-LNA, alpha-L-oxy-LNA, beta-D-amino-LNA and beta-D-thio-LNA, most preferred beta-D-oxy-LNA.

In some embodiments, the oligomer comprises from 3-8 nucleotide analogues, e.g. 6 or 7 nucleotide analogues. In the by far most preferred embodiments, at least one of said nucleotide analogues is a locked nucleic acid (LNA); for example at least 3 or at least 4, or at least 5, or at least 6, or at least 7, or 8, of the nucleotide analogues may be LNA. In some embodiments all the nucleotides analogues may be LNA.

In some embodiments the nucleotide analogues present within the oligomer of the invention are independently selected from, for example: 2'-O-alkyl-RNA units, 2'-amino-DNA units, 2'-fluoro-DNA units, LNA units, arabino nucleic acid (ANA) units, 2'-fluoro-ANA units, HNA units, INA (intercalating nucleic acid) units and 2'MOE units.

2'-O-methoxyethyl-RNA (2'MOE), 2'-fluoro-DNA monomers and LNA are preferred nucleotide analogues, and as such the oligonucleotide of the invention may comprise nucleotide analogues which are independently selected from these three types of analogue, or may comprise only one type of analogue selected from the three types.

Preferably, the oligomer according to the disclosure comprises at least one Locked Nucleic Acid (LNA) unit, such as 1, 2, 3, 4, 5, 6, 7, or 8 LNA units, preferably between 4 to 8 LNA units, most preferably 4, 5 or 6 LNA units. Suitably, the oligomer may comprise both beta-D-oxy-LNA, and one or more of the following LNA units: thio-LNA, amino-LNA, oxy-LNA, ena-LNA and/or alpha-LNA in either the D-beta or L-alpha configurations or combinations thereof.

In one embodiment of the disclosure, the oligomer may comprise both LNA and DNA units. Preferably the combined total of LNA and DNA units is 8 - 24, such as 8 - 15 or 10-25, or 10-20, or 12-16.

In one embodiment of the disclosure, the nucleobase sequence of the oligomer, such as the contiguous nucleobase sequence consists of at least one LNA and the remaining nucleobase units are DNA units.

In some embodiments of oligomer according to the disclosure, such as an antisense oligonucleotide which comprises LNA, all LNA C units are 5'methyl-Cytosine. In some embodiments, all the nucleotide analogues are LNA.

In most preferred embodiments the oligomer comprises only LNA nucleotide analogues and nucleotides (RNA or DNA, most preferably DNA nucleotides, optionally with modified internucleobase linkages such as phosphorothioate).

In some embodiments at least one of said nucleotide analogues is 2'-MOE-RNA, such as 2, 3, 4, 5, 6, 7 or 8 2'-MOE-RNA nucleobase units.

In some embodiments at least one of said nucleotide analogues is 2'-fluoro DNA, such as 2, 3, 4, 5, 6, 7 or 8 2'-fluoro-DNA nucleobase units.

Specific examples of nucleoside analogues are described by e.g. Freier & Altmann; Nucl. Acid Res., 1997, 25, 4429-4443 and Uhlmann; Curr. Opinion in Drug Development, 2000, 3(2), 293-213, and in Scheme 1:

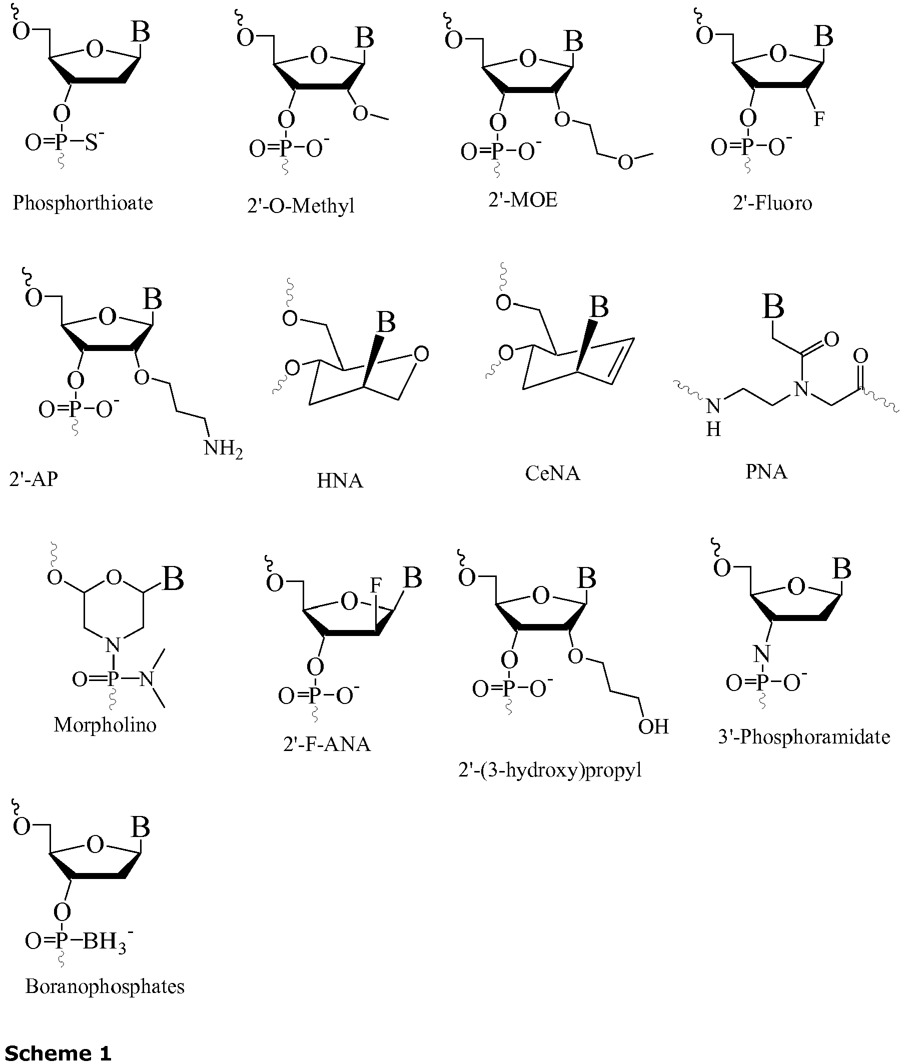

The term "LNA" refers to a bicyclic nucleotide analogue, known as "Locked Nucleic Acid". It may refer to an LNA monomer, or, when used in the context of an "LNA oligonucleotide" refers to an oligonucleotide containing one or more such bicyclic nucleotide analogues.

A particularly preferred chemistry is provided by locked nucleic acids (LNA) (Koshkin, A.A., et al., 1998, Tetrahedron 54:3607; Obika, S., et al., 1998, Tetrahedron Lett. 39:5401). As used herein, the terms "LNA unit", "LNA monomer", "LNA residue", "locked nucleic acid unit", "locked nucleic acid monomer" or "locked nucleic acid residue", refer to a bicyclic nucleoside analogue. LNA units and methods of their synthesis are described in *inter alia* WO 99/14226, WO 00/56746, WO 00/56748, WO 01/25248, WO 02/28875, WO 03/006475 and WO 03/095467. The LNA unit may also be defined with respect to its chemical formula. Thus, an "LNA unit", as used herein, has the chemical structure shown in Formula 1 below: 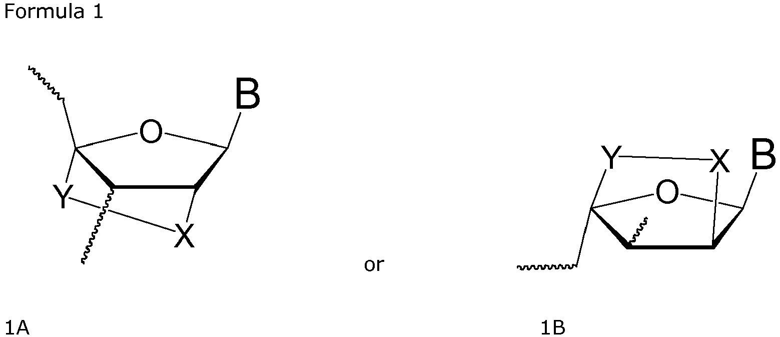 wherein,
X is selected from the group consisting of O, S and NRH, where R is H or C₁-C₄-alkyl;
Y is (-CH₂)ᵣ, where r is an integer of 1-4; and
B is a base of natural or non-natural origin as described above.

In a preferred embodiment, r is 1 or 2, and in a more preferred embodiment r is 1

The LNA used in the oligonucleotide compounds of the invention preferably has the structure of the general formula 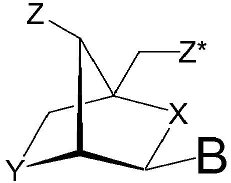
where X and Y are independently selected among the groups -O-,
-S-, -N(H)-, N(R)-, -CH₂- or -CH- (if part of a double bond),
-CH₂-O-, -CH₂-S-, -CH₂-N(H)-, -CH₂-N(R)-, -CH₂-CH₂- or -CH₂-CH- (if part of a double bond), -CH=CH-, where R is selected from hydrogen and C₁₋₄-alkyl; Z and Z* are independently selected among an internucleoside linkage, a terminal group or a protecting group; B constitutes a natural or non-natural nucleotide base moiety; and the asymmetric groups may be found in either orientation.

Preferably, the LNA used in the oligomer of the disclosure comprises at least one LNA unit according any of the formulas 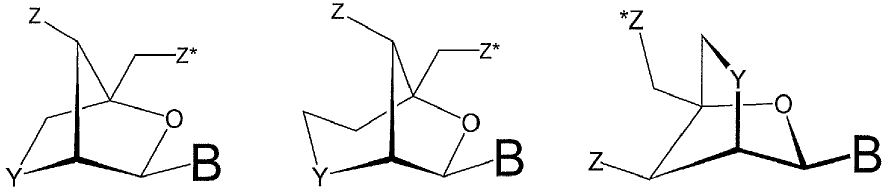 wherein Y is -O-, -S-, -NH-, or N(R^{H}); Z and Z* are independently selected among an internucleoside linkage, a terminal group or a protecting group; B constitutes a natural or non-natural nucleotide base moiety, and R^{H} is selected from hydrogen and C₁₋₄-alkyl.

Preferably, the LNA used in the oligomer of the disclosure comprises internucleoside linkages selected from -O-P(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R^{H})-O-, O-PO(OCH₃)-O-, -O-PO(NR^{H})-O-, -O-PO(OCH₂CH₂S-R)-O-, -O-PO(BH₃)-O-, -O-PO(NHR^{H})-O-, -O-P(O)₂-NR^{H}-, -NR^{H}-P(O)₂-O-, -NR^{H}-CO-O-, where R^{H} is selected form hydrogen and C₁₋₄-alkyl.

Specifically preferred LNA units are shown in scheme 2:

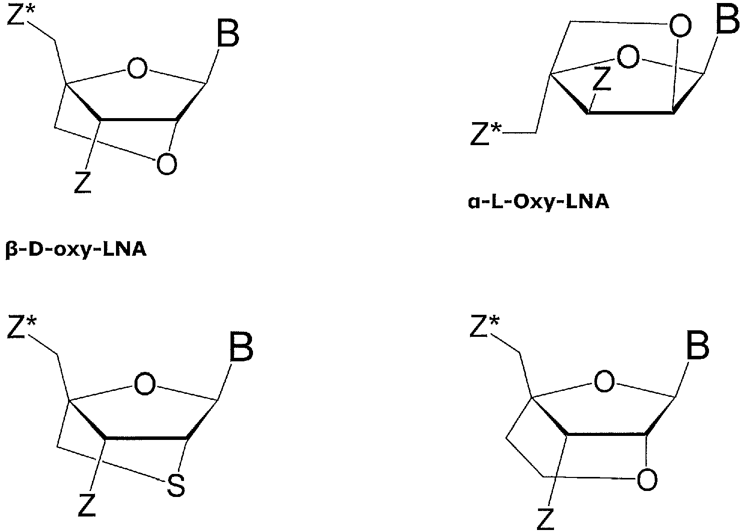

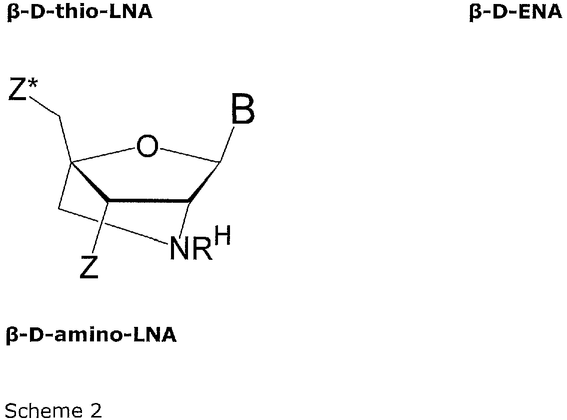

The term "thio-LNA" comprises a locked nucleotide in which at least one of X or Y in the general formula above is selected from S or -CH₂-S-. Thio-LNA can be in both beta-D and alpha-L-configuration.

The term "amino-LNA" comprises a locked nucleotide in which at least one of X or Y in the general formula above is selected from -N(H)-, N(R)-, CH₂-N(H)-, and -CH₂-N(R)- where R is selected from hydrogen and C₁₋₄-alkyl. Amino-LNA can be in both beta-D and alpha-L-configuration.

The term "oxy-LNA" comprises a locked nucleotide in which at least one of X or Y in the general formula above represents -O- or -CH₂-O-. Oxy-LNA can be in both beta-D and alpha-L-configuration.

The term "ena-LNA" comprises a locked nucleotide in which Y in the general formula above is -CH₂-O- (where the oxygen atom of -CH₂-O- is attached to the 2'-position relative to the base B).

In a preferred embodiment LNA is selected from beta-D-oxy-LNA, alpha-L-oxy-LNA, beta-D-amino-LNA and beta-D-thio-LNA, in particular beta-D-oxy-LNA.

Preferably, the oligomer according to the disclosure comprises at least one nucleotide analogue, such as Locked Nucleic Acid (LNA) unit, such as 1, 2, 3, 4, 5, 6, 7, 8, 9, or 10 nucleotide analogues, such as Locked Nucleic Acid (LNA) units, preferably between 3 to 9 nucleotide analogues, such as LNA units, such as 4 - 8, nucleotide analogues, such as LNA units, such as 6-9 nucleotide analogues, such as LNA units, preferably 6, 7 or 8 nucleotide analogues, such as LNA units.

The oligomer according to the disclosure, such as an antisense oligonucleotide, may comprises of 1, 2, 3, 4, 5, 6, 7, 8, 9, 10, 11, 12, 13, 14 or 15 nucleotide analogues, such as LNA units, in particular 3, 4, 5, 6, 7, 8, 9 or 10 nucleotide analogues, such as LNA units, such as between 1 and 10 nucleotide analogues, such as LNA units such as between 2 and 8 nucleotide analogues such as LNA units.

Preferably the LNA units comprise at least one beta-D-oxy-LNA unit(s) such as 2, 3, 4, 5, 6, 7, 8, 9, or 10 beta-D-oxy-LNA units.

The oligomer of the invention, such as the antisense oligonucleotide, may comprise more than one type of LNA unit. Suitably, the compound may comprise both beta-D-oxy-LNA, and one or more of the following LNA units: thio-LNA, amino-LNA, oxy-LNA, ena-LNA and/or alpha-LNA in either the D-beta or L-alpha configurations or combinations thereof.

Preferably, the oligomer, such as an antisense oligonucleotide, may comprise or consist of both nucleotide analogues, such as LNA units, and DNA units.

LNA and DNA are preferred, but MOE, 2'-O-Me, and other 2'-substituted analogues and RNA could also be used.

Preferred DNA analogues includes DNA analogues where the 2'-H group is substituted with a substitution other than -OH (RNA) e.g. by substitution with -O-CH₃, -O-CH2-CH2-O-CH3, -O-CH2-CH2-CH2-NH2, -O-CH2-CH2-CH2-OH or -F.

Preferred RNA analogues includes RNA analogues which have been modified in its 2'-OH group, e.g. by substitution with a group other than -H (DNA), for example -O-CH₃, -O-CH2-CH2-O-CH3, -O-CH2-CH2-CH2-NH2, -O-CH2-CH2-CH2-OH or -F.

In one embodiment the nucleotide analogue is "ENA".

In one embodiment, the oligomer of the disclosure does not comprise any RNA units.

High affintiy nucleotide analogues are nucleotide analogues which result in oligonucleotide which has a higher thermal duplex stability with a complementary RNA nucleotide than the binding affinity of an equivalent DNA nucleotide. This is typically determined by measuring the Tₘ.

Nucleotide analogues which increase the Tₘ of the oligomer/target nucleic acid target, as compared to the equivalent nucleotide are preferred (affinity enhancing nucleotide analogues). The oligomers may suitably be capable of hybridising against the target nucleic acid, such as a TNFR mRNA, to form a duplex with a Tₘ of at least 30°C, such as 37°C, such as at least 40°C, at least 50°C, at least 55°C, or at least 60°C. In one aspect, for example, the Tₘ is between 30°C and 80°C, such as between 40°C and 70°C.

In one embodiment at least 30%, such as at least 33%, such as at least 40%, such as at least 50%, such as at least 60%, such as at least 66%, such as at least 70%, such as at least 80%, such as at least 90% of the nucleobases of the oligomer of the invention are nucleotide analogues nucleobases, such as LNA. In one embodiment, all of the nucleobases of the oligomer of the disclosure are nucleotide analogues nucleobases, such as LNA.

It will be recognized that for shorter oligonucleotides it may be necessary to increase the proportion of (high affinity) nucleotide analogues, such as LNA.

The term "oligonucleotide" (or simply "oligo") which is used interchangeably with the term "oligomer" refers, in the context of the present disclosure, to a molecule formed by covalent linkage of two or more nucleobases. When used in the context of the oligonucleotide of the disclosure (also referred to the single stranded oligonucleotide), the term "oligonucleotide" may have, in one embodiment, for example between 8 -26 nucleobases, such as between 12 to 26 nucleobases. In a preferable embodiment, as detailed herein, the oligonucleotide of the invention has a length of between 10 - 16 nucleobases or 8-15 nucleobases.

### Variation of the length of the Oligomer

The length of the oligonucleotides of the disclosure may vary. Indeed it is considered advantageous to have short oligonucleotides, such as between 10 - 17 or 10 - 15 nucleobases.

In such an embodiment, the oligonucleotide of the disclosure may have a length of 10, 11, 12, 13, 14, 15, or 16 nucleobases.

In one embodiment, the oligonucleotide according to the present disclosure has a length of from 8 to 24 nucleotides, such as 10 to 24, between 12 to 24 nucleotides, such as a length of 8, 9, 10, 11, 12, 13, 14, 15, 16, 17, 18, 19, 20, 21, 22, 23 or 24 nucleotides, preferably a length of from 10 - 22, such as between 12 to 22 nucleotides, such as a length of 10, 11, 12, 13, 14, 15, 16, 17, 18, 19, 20, 21 or 22 nucleotides, more preferably a length of from 10 - 20, such as between 12 to 20 nucleotides, such as a length of 10, 11, 12, 13, 14, 15, 16, 17, 18, 19 or 20 nucleotides, even more preferably a length of from 10 to 19, such as between 12 to 19 nucleotides, such as a length of 10, 11, 12, 13, 14, 15, 16, 17, 18 or 19 nucleotides, e.g. a length of from 10 to 18, such as between 12 to 18 nucleotides, such as a length of 10, 11, 12, 13, 14, 15, 16, 17 or 18 nucleotides, more preferably a length of from 10 - 17, such as from 12 to 17 nucleotides, such as a length of 10, 11, 12, 13, 14, 15, 16 or 17 nucleotides, most preferably a length of from 10 to 16, such as between 12 to 16 nucleotides, such as a length of 10, 11, 12, 13, 14, 15 or 16 nucleotides.

### Internucleoside linkage group

The term "internucleoside linkage group" is intended to mean a group capable of covalently coupling together two nucleobases, such as between DNA units, between DNA units and nucleotide analogues, between two non-LNA units, between a non-LNA unit and an LNA unit, and between two LNA units, etc. Preferred examples include phosphate, phpshodiester groups and phosphorothioate groups.

The internucleoside linkage may be selected form the group consisting of: -O-P(O)2-O-, -O-P(O,S)-O-, -O-P(S)2-O-, -S-P(O)2-O-, -S-P(O,S)-O-, -S-P(S)2-O-, -O-P(O)2-S-, -O-P(O,S)-S-, -S-P(O)2-S-, -O-PO(RH)-O-, O-PO(OCH3)-O-, -O-PO(NRH)-O-, -O-PO(OCH2CH2S-R)-O-, -O-PO(BH3)-O-, -O-PO(NHRH)-O-, -O-P(O)2-NRH-, -NRH-P(O)2-O-, -NRH-CO-O-, -NRH-CO-NRH-, and/or the internucleoside linkage may be selected form the group consisting of: -O-CO-O-, -O-CO-NRH-, -NRH-CO-CH2-, -O-CH2-CO-NRH-, -O-CH2-CH2-NRH-, -CO-NRH-CH2-, -CH2-NRH-CO-, -O-CH2-CH2-S-, -S-CH2-CH2-O-, -S-CH2-CH2-S-, -CH2-SO2-CH2-, -CH2-CO-NRH-, -O-CH2-CH2-NRH-CO-, -CH2-NCH3-O-CH2-, where RH is selected from hydrogen and C1-4-alkyl. Suitably, in some embodiments, sulphur (S) containing internucleoside linkages as provided above may be preferred.

### Modification of the internucleoside linkage group

Typical internucleoside linkage groups in oligonucleotides are phosphate groups, but these may be replaced by internucleoside linkage groups differing from phosphate. In a further interesting embodiment of the disclosure, the oligonucleotide of the disclosure is modified in its internucleoside linkage group structure, i.e. the modified oligonucleotide comprises an internucleoside linkage group which differs from phosphate. Accordingly, in a preferred embodiment, the oligonucleotide according to the present invention comprises at least one internucleoside linkage group which differs from phosphate.

### Specific examples of internucleoside linkage groups which differ from phosphate

(-O-P(O)2-O-) include -O-P(O,S)-O-, -O-P(S)2-O-, -S-P(O)2-O-, -S-P(O,S)-O-, -S-P(S)2-O-, -O-P(O)2-S-, -O-P(O,S)-S-, -S-P(O)2-S-, -O-PO(RH)-O-, O-PO(OCH3)-O-, -O-PO(NRH)-O-,-O-PO(OCH2CH2S-R)-O-, -O-PO(BH3)-O-, -O-PO(NHRH)-O-, -O-P(O)2-NRH-, -NRH-P(O)2-O-, -NRH-CO-O-, -NRH-CO-NRH-, -O-CO-O-, -O-CO-NRH-, -NRH-CO-CH2-, -O-CH2-CO-NRH-,-O-CH2-CH2-NRH-, -CO-NRH-CH2-, -CH2-NRH-CO-, -O-CH2-CH2-S-, -S-CH2-CH2-O-, -S-CH2-CH2-S-, -CH2-SO2-CH2-, -CH2-CO-NRH-, -O-CH2-CH2-NRH-CO-, -CH2-NCH3-O-CH2-, where RH is hydrogen or C1-4-alkyl.

When the internucleoside linkage group is modified, the internucleoside linkage group is preferably a phosphorothioate group (-O-P(O,S)-O-). In a preferred embodiment, all internucleoside linkage groups of the oligonucleotides according to the present invention are phosphorothioate.

It is preferable for most therapeutic uses that the oligonucleotide is fully phosphorothiolated - the exception being for therapeutic oligonucleotides for use in the CNS, such as in the brain or spine where phosphorothioation can be toxic, and due to the absence of nucleases, phosphodiester bonds may be used, even between consecutive DNA units.

In one embodiment, the oligomer comprises alternating LNA and DNA units (Xx) or (xX).

In one embodiment, the oligomer comprises a motif of alternating LNA followed by 2 DNA units (Xxx), xXx or xxX.

In one embodiment, at least one of the DNA or non-LNA nucleotide analogue units are replaced with a LNA nucleobase in a position selected from the positions identified as LNA nucleobase units in any one of the embodiments referred to above.

In one embodiment,"X" donates an LNA unit.

In one embodiment, the oligomer comprises at least 3 nucleotide analogue units, such as at least 4 nucleotide analogue units, such as at least 5 nucleotide analogue units, such as at least 6 nucleotide analogue units, such as at least 7 nucleotide analogue units, such as at least 8 nucleotide analogue units, such as at least 9 nucleotide analogue units, such as at least 10, such as at least 11, such as at least 12 nucleotide analogue units.

In one embodiment, the oligomer comprises at least 3 LNA units, such as at least 4 LNA units, such as at least 5 LNA units, such as at least 6 LNA units, such as at least 7 LNA units, such as at least 8 LNA units, such as at least 9 LNA units, such as at least 10 LNA units, such as at least 11 LNA units, such as at least 12 LNA units.

In one embodiment wherein at least one of the nucleotide analogues, such as LNA units, is either cytosine or guanine, such as between 1 - 10 of the of the nucleotide analogues, such as LNA units, is either cytosine or guanine, such as 2, 3, 4, 5, 6, 7, 8, or 9 of the of the nucleotide analogues, such as LNA units, is either cytosine or guanine.

In one embodiment at least two of the nucleotide analogues such as LNA units is either cytosine or guanine. In one embodiment at least three of the nucleotide analogues such as LNA units is either cytosine or guanine. In one embodiment at least four of the nucleotide analogues such as LNA units is either cytosine or guanine. In one embodiment at least five of the nucleotide analogues such as LNA units is either cytosine or guanine. In one embodiment at least six of the nucleotide analogues such as LNA units is either cytosine or guanine. In one embodiment at least seven of the nucleotide analogues such as LNA units is either cytosine or guanine. In one embodiment at least eight of the nucleotide analogues such as LNA units is either cytosine or guanine.

In a preferred embodiment the nucleotide analogues have a higher thermal duplex stability a complementary RNA nucleotide than the binding affinity of an equivalent DNA nucleotide to said complementary RNA nucleotide.

In one embodiment, the nucleotide analogues confer enhanced serum stability to the single stranded oligonucleotide.

### Further Designs for Oligomers of the disclosure

In one embodiment, the first nucleobase of the oligomer according to the disclosure, counting from the 3' end, is a nucleotide analogue, such as an LNA unit.

In one embodiment, the second nucleobase of the oligomer according to the invention, counting from the 3' end, is a nucleotide analogue, such as an LNA unit.

In one embodiment, x" denotes a DNA unit.

In one embodiment, the oligomer comprises a nucleotide analogue unit, such as an LNA unit, at the 5' end.

In one embodiment, the nucleotide analogue units, such as X, are independently selected form the group consisting of: 2'-O-alkyl-RNA unit, 2'-OMe-RNA unit, 2'-amino-DNA unit, 2'-fluoro-DNA unit, 2'-MOE-RNA unit, LNA unit, PNA unit, HNA unit, INA unit.

In one embodiment, all the nucleobases of the oligomer of the disclosure are nucleotide analogue units.

In one embodiment, the nucleotide analogue units, such as X, are independently selected form the group consisting of: 2'-OMe-RNA units, 2'-fluoro-DNA units, and LNA units,

In one embodiment, the oligomer comprises said at least one LNA analogue unit and at least one further nucleotide analogue unit other than LNA.

In one embodiment, the non-LNA nucleotide analogue unit or units are independently selected from 2'-OMe RNA units and 2'-fluoro DNA units.

In one embodiment, the oligomer consists of at least one sequence X¹X²X¹ or X²X¹X², wherein X¹ is LNA and X² is either a 2'-OMe RNA unit and 2'-fluoro DNA unit.

In one embodiment, the sequence of nucleobases of the oligomer consists of alternative X¹ and X² units.

In one embodiment, the oligomer according to the disclosure does not comprise a region of more than 5 consecutive DNA nucleotide units. In one embodiment, the oligomer according to the disclosure does not comprise a region of more than 6 consecutive DNA nucleotide units. In one embodiment, the oligomer according to the disclosure does not comprise a region of more than 7 consecutive DNA nucleotide units. In one embodiment, the oligomer according to the disclosure does not comprise a region of more than 8 consecutive DNA nucleotide units. In one embodiment, the oligomer according to the disclosure does not comprise a region of more than 3 consecutive DNA nucleotide units. In one embodiment, the oligomer according to the disclosure does not comprise a region of more than 2 consecutive DNA nucleotide units.

In one embodiment, the oligomer comprises at least region consisting of at least two consecutive nucleotide analogue units, such as at least two consecutive LNA units.

In one embodiment, the oligomer comprises at least region consisting of at least three consecutive nucleotide analogue units, such as at least three consecutive LNA units.

In one embodiment, the oligomer of the disclosure does not comprise a region of more than 7 consecutive nucleotide analogue units, such as LNA units. In one embodiment, the oligomer of the disclosure does not comprise a region of more than 6 consecutive nucleotide analogue units, such as LNA units. In one embodiment, the oligomer of the disclosure does not comprise a region of more than 5 consecutive nucleotide analogue units, such as LNA units. In one embodiment, the oligomer of the disclosure does not comprise a region of more than 4 consecutive nucleotide analogue units, such as LNA units. In one embodiment, the oligomer of the disclosure does not comprise a region of more than 3 consecutive nucleotide analogue units, such as LNA units. In one embodiment, the oligomer of the disclosure does not comprise a region of more than 2 consecutive nucleotide analogue units, such as LNA units.

In one embodiment, the oligonucleotide of the disclosure comprises at least 50%, such as 55%, 60%, 65%, 70%, 75%, 80%, 85%, 90%, 95% or such as 100% of the nucleobase units of the oligomer are (preferably high affinity) nucleotide analogues, such as a Locked Nucleic Acid (LNA) nucleobase unit,

Table 3 and 4 below provides non-limiting examples of short microRNA sequences that could advantageously be targeted with an oligonucleotide of the present disclosure.

The oligonucleotides according to the disclosure may, in one embodiment, have a sequence of nucleobases 5' - 3' selected form the group consisting of the following motifs:
LxLxxLLxxLL
LxLxLLLxxLL
LxxLxxLxxL
xLxxLxxLxx 'Every third'
xxLxxLxxLx 'Every third'
xLxLxLxLxL 'Every second'
LxLxLxLxL 'Every second'
LdLddLLddLL
LdLdLLLddLLL
LMLMMLLMMLL
LMLMLLLMMLL
LFLFFLLFFLL
LFLFLLLFFLLL
LLLLLL
LLLLLLL
LLLLLLLL
LLLLLLLLL
LLLLLLLLLL
LLLLLLLLLLL
LLLLLLLLLLLL
LMMLMMLMML
MLMMLMMLMM 'Every third'
MMLMMLMMLM 'Every third'
LFFLFFLFFL 'Every third'
FLFFLFFLFF 'Every third'
FFLFFLFFLF 'Every third'
dLdLdLdLdL 'Every second'
LdLdLdLdL 'Every second'
MLMLMLMLML 'Every second'
LMLMLMLML 'Every second'
FLFLFLFLFL 'Every second'
LFLFLFLFL 'Every second'
LdLddLLddLdLdLL
LdLdLLLddLLLdLL
LMLMMLLMMLMLMLL
LMLMLLLMMLLLMLL
LFLFFLLFFLFLFLL
LFLFLLLFFLLLFLL
LddLddLddL(d)(d)(L)(d)(d)(L)(d)
dLddLddLdd(L)(d)(d)(L)(d)(d)(L)
ddLddLddLd(d)(L)(d)(d)(L)(d)(d)
LMMLMMLMML(M)(M)(L)(M)(M)(L)(M)
MLMMLMMLMM(L)(M)(M)(L)(M)(M)(L)
MMLMMLMMLM(M)(L)(M)(M)(L)(M)(M)
LFFLFFLFFL(F)(F)(L)(F)(F)(L)(F)
FLFFLFFLFF(L)(F)(F)(L)(F)(F)(L)
FFLFFLFFLF(F)(L)(F)(F)(L)(F)(F)
dLdLdLdLdL(d)(L)(d)(L)(d)(L)(d)
LdLdLdLdL(d)(L)(d)(L)(d)(L)(d)(L)
MLMLMLMLML(M)(L)(M)(L)(M)(L)(M)
LMLMLMLML(M)(L)(M)(L)(M)(L)(M)(L)
FLFLFLFLFL(F)(L)(F)(L)(F)(L)(F)
LFLFLFLFL(F)(L)(F)(L)(F)(L)(F)(L)
Wherein L = LNA unit, d= DNA units, M = 2'MOE RNA, F = 2'Fluoro and 'x' = as defined herein. It will be recognized that for longer oligomers the above patterns may be repeated, and for shorter, a corresponding fraction of the above motifs may be used - begining from the 5' end, or from the 3' end and residues in brackets are optional

In one embodiment, the disclosure further provides for a oligomer wherein said oligomer (or contiguous nucleobase sequence) comprises either at least one phosphorothioate linkage and/or at least one 3' terminal LNA unit, and/or at least one 5' teriminal LNA unit.

### Proteins

The disclosure further provides for an isolated, or purified, soluble form of TNFalpha receptor comprises a deletion in the trans-membrane binding domain encoded by exon 7, wherein said TNFalpha receptor is selected from the TNFalpha receptor TNFRSF1A or TNFRSF1B, or a variant, fragment or homologue thereof.

In one embodiment, the isolated, or purified, soluble form of TNFalpha receptor according to the disclosure lacks the trans-membrane binding domain encoded by exon 7.

In one embodiment, the isolated, or purified, soluble form of TNFalpha receptor is the human TNFR1 TNFalpha receptor (residues 1-455, or residues 30-455 of SEQ ID NO 123, or a variant, fragment or homologue thereof.), wherein said deletion is between residues 209 and 246 (or region which corresponds to residues 209 and 246 of SEQ ID No 123).

In one embodiment, the isolated, or purified, soluble form of TNFalpha has a sequence consisting of residues 1-208 or residues 30-208 of SEQ ID NO 119, or is a variant, fragment or homologue thereof.

In one embodiment, the isolated, or purified, soluble form of TNFalpha receptor is the human TNFR2 TNFalpha receptor (residues 1-435, or residues 23-435 of SEQ ID NO 127, or a variant, fragment or homologue thereof, wherein said deletion is between residues 263 and 289 (or region which corresponds to residues 209 and 246 of SEQ ID No 123).

In one embodiment, the isolated, or purified, soluble form of TNFalpha receptor has a sequence consisting of residues 1-262 or 23-262 of SEQ ID NO 127, or is a variant, fragment or homologue thereof.

In one preferred embodiment, the soluble form of the TNFalpha receptor is both isolated and purified.

One embodiment of the present disclosure is a protein, either full length or mature, which is encoded by a cDNA derived from a mammalian TNFR gene, and in the cDNA exon 6 is followed directly by exon 8 and as a result lacks exon 7. Furthermore the protein can bind TNF, preferably TNF-α, and can act as a TNF, preferably TNF-α, antagonist. Preferably, TNFR of the present disclosure is capable of inhibition of TNF-mediated cytotoxicity to a greater extent than the soluble extracellular domain alone, and more preferably, to an extent comparable to or greater than TNFR:Fc. Mammalian TNFR according to the present disclosure includes, but is not limited to, human, primate, murine, canine, feline, bovine, ovine, equine, and porcine TNFR. Furthermore, mammalian TNFR according to the present disclosure includes, but is not limited to, a protein sequence that results from one or more single nucleotide polymorphisms, such as for example those disclosed in EP Pat. Appl. 1,172,444, as long as the protein retains a comparable biological activity to the reference sequence with which it is being compared.

In one embodiment, the mammalian TNFR is a mammalian TNFR1, preferably a human TNFR1. For human TNFR1 two non-limiting examples of this embodiment are given by huTNFR1 Δ7 which includes the signal sequence as shown in SEQ ID No: 122 and mature huTNFR1 Δ7 (amino acids 30-417 of SEQ ID No: 122) which lacks the signal sequence. The sequences of these huTNFR1 Δ7 proteins are either amino acids 1-208 of wild type human TNFR1 (SEQ ID No: 118) which includes the signal sequence or 30-208 of wild type human TNFR1 for mature huTNFR1 Δ7 which lacks the signal sequence, and in either case is followed immediately by amino acids 247-455 of wild type human TNFR1.

In another preferred embodiment, the mammalian TNFR is a mammalian TNFR2, most preferably a human TNFR2. For human TNFR2 two non-limiting examples of this embodiment are given by huTNFR2 Δ7 which includes the signal sequence as shown in SEQ ID No: 126 or mature huTNFR2 Δ7 (amino acids 23-435 of SEQ ID No: 126) which lacks the signal sequence. The sequences of these huTNFR2 Δ7 proteins are either amino acids 1-262 of wild type human TNFR2 (SEQ ID No: 120) which includes the signal sequence or 23-262 of wild type human TNFR2 for mature huTNFR2 Δ7 which lacks the signal sequence, followed in either case by the amino acid glutamate, because of the creation of a unique codon at the exon 6-8 junction, which is followed by amino acids 290-461 of wild type human TNFR2.

The proteins of the present disclosure also include those proteins that are chemically modified. Chemical modification of a protein refers to a protein where at least one of its amino acid residues is modified by either natural processes, such as processing or other post-translational modifications, or by chemical modification techniques known in the art. Such modifications include, but are not limited to, acetylation, acylation, amidation, ADP-ribosylation, glycosylation, methylation, pegylation, prenylation, phosphorylation, or cholesterol conjugation.

The proteins of the present disclosure may, in one embodiment, also include variants, fragments and homolgoues of the proteins of the disclosure. However, such proteins comprise a deletion in the amino acid sequence which is encoded by exon 7 or exon 8, as explained herein.

### Nucleic Acids

The disclosure further provides a nucleic acid encoding the soluble form of TNFalpha receptor according to the disclosure.

In one embodiment, the nucleic acid is selected from the group consisting of: nucleotides 1-1251 of SEQ ID NO 121, 88-1251 of SEQ ID NO 121, 1-1305 of SEQ ID NO 125 and 67-1305 of SEQ ID NO 125, or variant, homologue or fragment thereof, including a nucleic acid which encodes the same primary amino acid sequence as the nucleic acid, i.e. due to the degeneracy of the genetic code.

One embodiment of the present disclosure provides a nucleic acid that encodes a protein, either full length or mature, which is encoded by a cDNA derived from a mammalian TNFR gene, and in the cDNA exon 6 is followed directly by exon 8 and as a result lacks exon 7.

Such sequences are preferably provided in the form of an open reading frame uninterrupted by internal nontranslated sequences, or introns, which are typically present in eukaryotic genes. Genomic DNA containing the relevant sequences can also be used. In one embodiment, the nucleic acid is either an mRNA or a cDNA. In another embodiment, it is genomic DNA.

In one embodiment, the mammalian TNFR is a mammalian TNFR1. For this embodiment, the mammalian TNFR1 is preferably a human TNFR1. For human TNFR1, two non-limiting examples of this embodiment are nucleic acids which encode the huTNFR1 Δ7 which includes the signal sequence as shown in SEQ ID No: 122 and mature huTNFR1 Δ7 (amino acids 30-417 of SEQ ID No: 122) which lacks the signal sequence. Preferably, the sequences of these huTNFR1 Δ7 nucleic acids are nucleotides 1-1251 of SEQ ID No: 121, which includes the signal sequence and nucleotides 88-1251 of SEQ ID No: 121 which lacks the signal sequence. The sequences of these huTNFR1 Δ7 nucleic acids are either nucleotides 1-625 of wild type human TNFR1 (SEQ ID No: 117) which includes the signal sequence or 88-625 of wild type human TNFR1 for mature huTNFR2 Δ7 which lacks the signal sequence, and in either case is followed immediately by amino acids 740-1368 of wild type human TNFR1.

In another preferred embodiment, the mammalian TNFR is a mammalian TNFR2, most preferably a human TNFR2. For human TNFR2, two non-limiting examples of this embodiment are nucleic acids which encode the huTNFR2 Δ7 which includes the signal sequence as shown in SEQ ID No: 126 or mature huTNFR2 Δ7 (amino acids 23-435 of SEQ ID No: 126) which lacks the signal sequence. Preferably, the sequences of these huTNFR2 Δ7 nucleic acids are nucleotides 1-1305 of SEQ ID No: 115 which includes the signal sequence and nucleotides 67-1305 of SEQ ID No: 115 which lacks the signal sequence. The sequences of these huTNFR2 Δ7 nucleic acids are either nucleotides 1-787 of wild type human TNFR2 (SEQ ID No: 119) which includes the signal sequence or 67-787 of wild type human TNFR2 for mature huTNFR2 Δ7 which lacks the signal sequence, and in either case is followed immediately by amino acids 866-1386 of wild type human TNFR2.

The bases of the nucleic acids of the present disclosure can be the conventional bases cytosine, guanine, adenine and uracil or thymidine. Alternatively, modified bases can be used. Other suitable bases include, but are not limited to, 5-methylcytosine (^{Me}C), isocytosine, pseudoisocytosine, 5-bromouracil, 5-propynyluracil, 5-propyny-6, 5-methylthiazoleuracil, 6-aminopurine, 2-aminopurine, inosine, 2,6-diaminopurine, 7-propyne-7-deazaadenine, 7-propyne-7-deazaguanine, 2-chloro-6-aminopurine and 9-(aminoethoxy)phenoxazine.

Suitable nucleic acids of the present disclosure include numerous alternative chemistries. For example, suitable nucleic acids of the present invention disclosure, but are not limited to, those wherein at least one of the internucleotide bridging phosphate residues is a modified phosphate, such as phosphorothioate, methyl phosphonate, methyl phosphonothioate, phosphoromorpholidate, phosphoropiperazidate, and phosphoroamidate. In another non-limiting example, suitable nucleic acids of the present invention include those wherein at least one of the nucleotides contain a 2' lower alkyl moiety (e.g., C₁-C₄, linear or branched, saturated or unsaturated alkyl, such as methyl, ethyl, ethenyl, propyl, 1-propenyl, 2-propenyl, and isopropyl).

Nucleic acids of the present disclosure also include, but are not limited to, those wherein at least one, of the nucleotides is a nucleic acid analogue. Examples of such analogues include, but are not limited to, hexitol (HNA) nucleotides, 2'O-4'C-linked bicyclic ribofuranosyl (LNA) nucleotides, peptide nucleic acid (PNA) analogues, N3'→P5' phosphoramidate analogues, phosphorodiamidate morpholino nucleotide analogues, and combinations thereof.

Nucleic acids of the present disclosure include, but are not limited to, modifications of the nucleic acids involving chemically linking to the nucleic acids one or more moieties or conjugates. Such moieties include, but are not limited to, lipid moieties such as a cholesterol moiety, cholic acid, a thioether, e.g. hexyl-S-tritylthiol, a thiocholesterol, an aliphatic chain, e.g., dodecandiol or undecyl residues, a phospholipids, e.g., di-hexadecyl-rac-glycerol or triethylammonium 1,2-di-O-hexadecyl-rac-glycero-3-H-phosphonate, a polyamine or a polyethylene glycol chain, an adamantane acetic acid, a palmityl moiety, an octadecylamine or hexylamino-carbonyl-oxycholesterol moiety.

### Expression Vectors and Host Cells

The disclosure also provides for a vector comprising the nucleic acid of the disclosure.

In one embodiment, the vector comprises an expression cassette capable of driving the expression of said nucleic acid in a host cell.

The disclosure also provides for a host cell comprising the nucleic acid or the vector according to the disclosure.

The disclosure also provides for a method for the preparation of a soluble form of TNFalpha receptor, said method comprising the step of culturing the host cell according to the disclosure under conditions which allow the expression of said nucleic acid, and subsequently isolating said soluble form of TNFalpha receptor from said host cells.

The present disclosure provides expression vectors to amplify or express DNA encoding mammalian TNFR of the current disclosure. The present disclosure also provides host cells transformed with the foregoing expression vectors. Expression vectors are replicable DNA constructs which have synthetic or cDNA-derived DNA fragments encoding mammalian TNFR or bioequivalent analogues operably linked to suitable transcriptional or translational regulatory elements derived from mammalian, microbial, viral, or insect genes. A transcriptional unit generally comprises an assembly of (a) a genetic element or elements having a regulatory role in gene expression, such as, transcriptional promoters or enhancers, (b) a structural or coding sequence which is transcribed into mRNA and translated into protein, and (c) appropriate transcription and translation initiation and termination sequences. Such regulatory elements can include an operator sequence to control transcription, and a sequence encoding suitable mRNA ribosomal binding sites. The ability to replicate in a host, usually conferred by an origin of replication, and a selection gene to facilitate recognition of transformants, can additionally be incorporated.

DNA regions are operably linked when they are functionally related to each other. For example, DNA for a signal peptide (secretory leader) is operably linked to DNA for a polypeptide if it is expressed as a precursor which participates in the secretion of the polypeptide; a promoter is operably linked to a coding sequence if it controls the transcription of the sequence; or a ribosome binding site is operably linked to a coding sequence if it is positioned so as to permit translation. Generally, operably linked means contiguous and, in the case of secretory leaders, contiguous and in reading frame. Structural elements intended for use in yeast expression systems preferably include a leader sequence enabling extracellular secretion of translated protein by a host cell. Alternatively, where recombinant protein is expressed without a leader or transport sequence, it may include an N-terminal methionine residue. This residue may optionally be subsequently cleaved from the expressed protein to provide a final product.

Mammalian TNFR DNA is expressed or amplified in a recombinant expression system comprising a substantially homogeneous monoculture of suitable host microorganisms, for example, bacteria such as E. coli or yeast such as S. cerevisiae, which have stably integrated (by transformation or transfection) a recombinant transcriptional unit into chromosomal DNA or carry the recombinant transcriptional unit as a component of a resident plasmid. Recombinant expression systems as defined herein will express heterologous protein either constitutively or upon induction of the regulatory elements linked to the DNA sequence or synthetic gene to be expressed.

Transformed host cells are cells which have been transformed or transfected with mammalian TNFR vectors constructed using recombinant DNA techniques. Transformed host cells ordinarily express TNFR, but host cells transformed for purposes of cloning or amplifying TNFR DNA do not need to express TNFR. Suitable host cells for expression of mammalian TNFR include prokaryotes, yeast, fungi, or higher eukaryotic cells. Prokaryotes include gram negative or gram positive organisms, for example E. coli or bacilli. Higher eukaryotic cells include, but are not limited to, established insect and mammalian cell lines. Cell-free translation systems can also be employed to produce mammalian TNFR using RNAs derived from the DNA constructs of the present disclosure. Appropriate cloning and expression vectors for use with bacterial, fungal, yeast, and mammalian cellular hosts are well known in the art.

Prokaryotic expression hosts may be used for expression of TNFR that do not require extensive proteolytic and disulfide processing. Prokaryotic expression vectors generally comprise one or more phenotypic selectable markers, for example a gene encoding proteins conferring antibiotic resistance or supplying an autotrophic requirement, and an origin of replication recognized by the host to ensure amplification within the host. Suitable prokaryotic hosts for transformation include E. coli, Bacillus subtilis, Salmonella typhimurium, and various species within the genera Pseudomonas, Streptomyces, and Staphyolococcus, although others can also be employed as a matter of choice.

Useful expression vectors for bacterial use can comprise a selectable marker and bacterial origin of replication derived from commercially available plasmids comprising genetic elements of the well known cloning vector pBR322 (ATCC 37017). These pBR322 "backbone" sections are combined with an appropriate promoter and the structural sequence to be expressed. pBR322 contains genes for ampicillin and tetracycline resistance and thus provides simple means for identifying transformed cells. Such commercial vectors include, for example, the series of Novagen® pET vectors (EMD Biosciences, Inc., Madison, Wis.).

Promoters commonly used in recombinant microbial expression vectors include the lactose promoter system, and the λ P_{L} promoter, the T7 promoter, and the T7 lac promoter. A particularly useful bacterial expression system, Novagen® pET system (EMD Biosciences, Inc., Madison, Wis.) employs a T7 or T7 lac promoter and E. coli strain, such as BL21(DE3) which contain a chromosomal copy of the T7 RNA polymerase gene.

TNFR proteins can also be expressed in yeast and fungal hosts, preferably from the genus Saccharomyces, such as S. cerevisiae. Yeast of other genera, such as Pichia or Kluyveromyces can also be employed. Yeast vectors will generally contain an origin of replication from the 2µ yeast plasmid or an autonomously replicating sequence (ARS), promoter, DNA encoding TNFR, sequences for polyadenylation and transcription termination and a selection gene. Preferably, yeast vectors will include an origin of replication and selectable marker permitting transformation of both yeast and E. coli, e.g., the ampicillin resistance gene of E. coli and S. cerevisiae TRP1 or URA3 gene, which provides a selection marker for a mutant strain of yeast lacking the ability to grow in tryptophan or uracil, respectively, and a promoter derived from a highly expressed yeast gene to induce transcription of a structural sequence downstream. The presence of the TRP1 or URA3 lesion in the yeast host cell genome then provides an effective environment for detecting transformation by growth in the absence of tryptophan or uracil, respectively.

Suitable promoter sequences in yeast vectors include the promoters for metallothionein, 3-phosphoglycerate kinase or other glycolytic enzymes , such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Suitable vectors and promoters for use in yeast expression are well known in the art.

Preferred yeast vectors can be assembled using DNA sequences from pUC18 for selection and replication in E. coli (Amp^{r} gene and origin of replication) and yeast DNA sequences including a glucose-repressible ADH2 promoter and α-factor secretion leader. The yeast α-factor leader, which directs secretion of heterologous proteins, can be inserted between the promoter and the structural gene to be expressed. The leader sequence can be modified to contain, near its 3' end, one or more useful restriction sites to facilitate fusion of the leader sequence to foreign genes. Suitable yeast transformation protocols are known to those of skill in the art.

Host strains transformed by vectors comprising the ADH2 promoter may be grown for expression in a rich medium consisting of 1% yeast extract, 2% peptone, and 1% or 4% glucose supplemented with 80 mg/ml adenine and 80 mg/ml uracil. Derepression of the ADH2 promoter occurs upon exhaustion of medium glucose. Crude yeast supernatants are harvested by filtration and held at 4°C. prior to further purification.

Various mammalian or insect cell culture systems are also advantageously employed to express TNFR protein. Expression of recombinant proteins in mammalian cells is particularly preferred because such proteins are generally correctly folded, appropriately modified and completely functional. Examples of suitable mammalian host cell lines include the COS-7 lines of monkey kidney cells, and other cell lines capable of expressing an appropriate vector including, for example, L cells, such as L929, C127, 3T3, Chinese hamster ovary (CHO), HeLa and BHK cell lines. Mammalian expression vectors can comprise nontranscribed elements such as an origin of replication, a suitable promoter, for example, the CMVie promoter, the chicken beta-actin promoter, or the composite hEF1-HTLV promoter, and enhancer linked to the gene to be expressed, and other 5' or 3' flanking nontranscribed sequences, and 5' or 3' nontranslated sequences, such as necessary ribosome binding sites, a polyadenylation site, splice donor and acceptor sites, and transcriptional termination sequences. Baculovirus systems for production of heterologous proteins in insect cells are known to those of skill in the art.

The transcriptional and translational control sequences in expression vectors to be used in transforming vertebrate cells can be provided by viral sources. For example, commonly used promoters and enhancers are derived from Polyoma, Adenovirus 2, Simian Virus 40 (SV40), human cytomegalovirus, such as the CMVie promoter, HTLV, such as the composite hEF1-HTLV promoter. DNA sequences derived from the SV40 viral genome, for example, SV40 origin, early and late promoter, enhancer, splice, and polyadenylation sites can be used to provide the other genetic elements required for expression of a heterologous DNA sequence.

Further, mammalian genomic TNFR promoter, such as control and/or signal sequences can be utilized, provided such control sequences are compatible with the host cell chosen.

In preferred aspects of the present disclosure, recombinant expression vectors comprising TNFR cDNAs are stably integrated into a host cell's DNA.

### Protein Expression and Purification:

When mammalian or insect cells are used, properly expressed TNFR protein will be secreted into the extracellular media. The protein is recovered from the media, and is concentrated and is purified using standard biochemical techniques. After expression in mammalian cells by lentiviral or AAV transduction, plasmid transfection, or any similar procedure, or in insect cells after baculoviral transduction, the extracellular media of these cells is concentrated using concentration filters with an appropriate molecular weight cutoff, such as Amicon® filtration units. To avoid loss of TNFR protein, the filter should allow proteins to flow through that are at or below 50 kDal.

When TNFR protein is expressed in bacterial culture it can be purified by standard biochemical techniques. Bacteria are lysed, and the cellular extract containing the TNFR is desalted and is concentrated.

In either case, the TNFR protein is preferably purified by affinity chromatography. The use of column chromatography with an affinity matrix comprising TNF-α is preferred. Alternatively, an affinity purification tag can be added to either the N- or the C-terminus of the TNFR protein. For example, a polyhistidine-tag (His-tag), which is an amino acid motif with at least six histidines, can be used for this purpose (Hengen, P., 1995, Trends Biochem. Sci. 20:285-86). The addition of a His-tag can be achieved by the in-frame addition of a nucleotide sequence encoding the His-tag directly to either the 5' or 3' end of the TNFR open reading frame in an expression vector. One such nucleotide sequence for the addition of a C-terminal His-tag is given in SEQ ID No: 126. When a His-tag is incorporated into the protein, a nickel or cobalt affinity column is employed to purify the tagged TNFR, and the His-tag can optionally then be cleaved. Other suitable affinity purification tags and methods of purification of proteins with those tags are well known in the art.

Alternatively, a non-affinity based purification scheme can be used, involving fractionation of the TNFR extracts on a series of columns that separate the protein based on size (size exclusion chromatography), charge (anion and cation exchange chromatography) and hydrophobicity (reverse phase chromatography). High performance liquid chromatography can be used to facilitate these steps.

Other methods for the expression and purification of TNFR proteins are well known (*See, e.g.,* U.S. Pat. No. 5,605,690 to Jacobs).

### Definitions

The term "internucleoside linkage group" is intended to mean a group capable of covalently coupling together two nucleobases, such as between DNA units, between DNA units and nucleotide analogues, between two non-LNA units, between a non-LNA unit and an LNA unit, and between two LNA units, etc. Preferred examples include phosphate, phpshodiester groups and phosphorothioate groups.

Herein, the term "nitrogenous base" is intended to cover purines and pyrimidines, such as the DNA nucleobases A, C, T and G, the RNA nucleobases A, C, U and G, as well as non-DNA/RNA nucleobases, such as 5-methylcytosine (^{Me}C), isocytosine, pseudoisocytosine, 5-bromouracil, 5-propynyluracil, 5-propyny-6-fluoroluracil, 5-methylthiazoleuracil, 6-aminopurine, 2-aminopurine, inosine, 2,6-diaminopurine, 7-propyne-7-deazaadenine, 7-propyne-7-deazaguanine and 2-chloro-6-aminopurine, in particular ^{Me}C. It will be understood that the actual selection of the non-DNA/RNA nucleobase will depend on the corresponding (or matching) nucleotide present in the microRNA strand which the oligonucleotide is intended to target. For example, in case the corresponding nucleotide is G it will normally be necessary to select a non-DNA/RNA nucleobase which is capable of establishing hydrogen bonds to G. In this specific case, where the corresponding nucleotide is G, a typical example of a preferred non-DNA/RNA nucleobase is ^{Me}C.

As used herein, the terms "tumor necrosis factor receptor", "TNF receptor", and "TNFR" refer to proteins having amino acid sequences of or which are substantially similar to native mammalian TNF receptor sequences, and which are capable of binding TNF molecules. In this context, a "native" receptor or gene for such a receptor, means a receptor or gene that occurs in nature, as well as the naturally-occurring allelic variations of such receptors and genes.

The term "mature" as used in connection with a TNFR means a protein expressed in a form lacking a leader or signal sequence as may be present in full-length transcripts of a native gene.

The nomenclature for TNFR proteins as used herein follows the convention of naming the protein (e.g., TNFR2) preceded by a species designation, e.g., hu (for human) or mu (for murine), followed by a Δ (to designate a deletion) and the number of the exon(s) deleted. For example, huTNFR2 Δ7 refers to human TNFR2 lacking exon 7. In the absence of any species designation, TNFR refers generically to mammalian TNFR.

The term "secreted" means that the protein is soluble, i.e., that it is not bound to the cell membrane. In this context, a form will be soluble if using conventional assays known to one of skill in the art most of this form can be detected in fractions that are not associated with the membrane, e.g., in cellular supernatants or serum.

The term "stable" means that the secreted TNFR form is detectable using conventional assays by one of skill in the art, such as, western blots, ELISA assays in harvested cells, cellular supernatants, or serum.

As used herein, the terms "tumor necrosis factor" and "TNF" refer to the naturally-occuring protein ligands that bind to TNF receptors. TNF includes, but is not limited to, TNF-α and TNF-β.

As used herein, the term "an inflammatory disease or condition" refers to a disease, disorder, or other medical condition that at least in part results from or is aggravated by the binding of TNF to its receptor. Such diseases or conditions include, but are not limited to, those associated with increased levels of TNF, increased levels of TNF receptor, or increased sensitization or deregulation of the corresponding signaling pathway. The term also encompasses diseases and conditions for which known TNF antagonists have been shown useful. Examples of inflammatory diseases or conditions include, but are not limited to, rheumatoid arthritis, juvenile rheumatoid arthritis, psoriasis, psoriatic arthritis, ankylosing spondylitis, inflammatory bowel disease (including Crohn's disease and ulcerative colitis), hepatitis, sepsis, alcoholic liver disease, and non-alcoholic steatosis.

As used herein, the term "hepatitis" refers to a gastroenterological disease, condition, or disorder that is characterized, at least in part, by inflammation of the liver. Examples of hepatitis include, but are not limited to, hepatitis associated with hepatitis A virus, hepatitis B virus, hepatitis C virus, or liver inflammation associated with ischemia/reperfusion.

As used herein, the term "TNF antagonist" means that the protein is capable of measurable inhibition of TNF-mediated cytotoxicity using standard assays as are well known in the art . (*See, e.g* L929 cytotoxicity assay as described in the Examples below).

The term "binds TNF" means that the protein can bind detectable levels of TNF, preferably TNF-α, as measured by standard binding assays as are well known in the art (*See, e.g.,* U.S. Pat. No. 5,945,397 to Smith, cols. 16-17). Preferably, receptors of the present disclosure are capable of binding greater than 0.1 nmoles TNF-α/nmole receptor, and more preferably, greater than 0.5 nmoles TNF-α/nmole receptor using standard binding assays.

As used herein, the term "regulatory element" refers to a nucleotide sequence involved in an interaction of molecules that contributes to the functional regulation of a nucleic acid, including but not limited to, replication, duplication, transcription, splicing, translation, or degradation of the nucleic acid. The regulation may be enhancing or inhibitory in nature. Regulatory elements known in the art include, for example, transcriptional regulatory sequences such as promoters and enhancers. A promoter is a DNA region that is capable under certain conditions of aiding the initiation of transcription of a coding region usually located downstream (in the 3' direction) from the promoter. An expression vector typically comprises such regulatory elements operably linked to the nucleic acid of the disclosure.

The terms "oligomer" and "splice switching oligomer" and "oligonucleotide" are used interchangeably herein.

As used herein, the term "operably linked" refers to a juxtaposition of genetic elements, wherein the elements are in a relationship permitting them to operate in the expected manner. For example, a promoter is operably linked to a coding region if the promoter helps initiate transcription of the coding sequence (such as in an expression vector). As long as this functional relationship is maintained, there can be intervening residues between the promoter and the coding region.

As used herein, the terms "transformation" or "transfection" refer to the insertion of an exogenous nucleic acid into a cell, irrespective of the method used for the insertion, for example, lipofection, transduction, infection or electroporation. The exogenous nucleic acid can be maintained as a non-integrated vector, for example, a plasmid, or alternatively, can be integrated into the cell's genome.

As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids. or viral vectors (e.g., replication defective retroviruses, adenoviruses and adeno-associated viruses).

As used herein, the term "isolated protein" refers to a protein or polypeptide that is not naturally-occurring and/or is separated from one or more components that are naturally associated with it.

As used herein, the term "isolated nucleic acid" refers to a nucleic acid that is not naturally-occurring and/or is in the form of a separate fragment or as a component of a larger construct, which has been derived from a nucleic acid isolated at least once in substantially pure form, i.e., free of contaminating endogenous materials, and in a quantity or concentration enabling identification and manipulation by standard biochemical methods, for example, using a cloning vector.

As used herein the term "purified protein" refers to a protein that is present in the substantial absence of other protein. However, such purified proteins can contain other proteins added as stabilizers, carriers, excipients, or co-therapeutics. The term "purified" as used herein preferably means at least 50% such as at least 80% by dry weight, more preferably in the range of 95-99% by weight, and most preferably at least 99.8% by weight, of protein present, excluding proteins added as stabilizers, carriers, excipients, or co-therapeutics.

As used herein, the term "altering the splicing of a pre-mRNA" refers to altering the splicing of a cellular pre-mRNA target resulting in an altered ratio of splice products. Such an alteration of splicing can be detected by a variety of techniques well known to one of skill in the art. For example, RT-PCR on total cellular RNA can be used to detect the ratio of splice products in the presence and the absence of an SSO.

As used herein, the term "complementary" is used to indicate a sufficient degree of complementarity or precise pairing such that stable and specific binding occurs between an oligonucleotide and a DNA or RNA containing the target sequence. It is understood in the art that the sequence of an oligonucleotide need not be 100% complementary to that of its target. For example, for an SSO there is a sufficient degree of complementarity when, under conditions which permit splicing, binding to the target will occur and non-specific binding will be avoided. ¨However, it is preferred that the oligonucloeitde or contiguous nucleobase sequence is fully (i.e. perfectly) complementary to the target sequence (such as the region of SEQ ID NO 1 - 4, refered to herein).

The terms "corresponding to" and "corresponds to" as used in the context of oligonucleotides refers to the comparison between either a nucleobase sequence of the compound of the invention, and the reverse complement thereof, or in one embodiment between a nucleobase sequence and an equivalent (identical) nucleobase sequence which may for example comprise other nucleobases but retains the same base sequence, or complement thereof. Nucleotide analogues are compared directly to their equivalent or corresponding natural nucleotides. Sequences which form the reverse complement of a sequence are referred to as the complement sequence of the sequence.

When referring to the length of a nucleotide molecule as referred to herein, the length corresponds to the number of monomer units, *i.e.* nucleobases, irrespective as to whether those monomer units are nucleotides or nucleotide analogues. With respect to nucleobases, the terms monomer and unit are used interchangeably herein.

It should be understood that when the term "about" is used in the context of specific values or ranges of values, the disclosure should be read as to include the specific value or range referred to.

The term "variant" as used in herein in the context of a protein or polypeptide (sequence), refers to a polypeptide which is prepared from the original (parent) polypeptide, or using the sequence information from the polypeptide, by insertion, deletion or substitution of one or more amino acids in said sequence, i.e. at least one amino acids, but preferably less than 50 amino acids, such as less than 40, less than 30, less than 20, or less than 10 amino acids, such as 1 amino acid, 1-2 amino acids, 1-3 amino acids, 1-4 amino acids, 1-5 amino acids.

The term "homologue" as used herein in the context of a protein or polypeptide (sequence), refers to a polypeptide which is at least 70% homologous, such as at least 80% homologous, such as at least 85% homologous, or at least 90% homologous, such as at least 95%, 96%, 97%, 98% or 99% homologous to said polypeptide sequence. Homology between two polypeptide sequences may be determined using ClustalW alignment algorithm using the Blosum 62 algorithm, with Gap Extent = 0.5, Gap open = 10 (see http://www.ebi.ac.uk/emboss/align/index.html). The alignment may, in one embodiment be a local alignment (water) or a separate embodiment be a global alignment (needle). As the homolgoues of the exon deletion TNFR proteins referred to herein also comprise deletion in the respective exon, a global alignment may be preferred.

The term "fragment" as used herein in the context of a protein or polypeptide (sequence), refers to a polypeptide which consists of only a part of the polypeptide sequence. A fragment may therefore comprise at least 5% such as at least 10% of said polypeptide sequence, including at least 20%, 30%, 40%, 50%, 60%, 70%, 80%, 90% or 95% of said polypeptide sequence.

The above definitions of variant, fragment and homologue also apply to nucleic acid sequences, although the homology algorithm used is DNAfull. Obviously, when referring to nucleic acid variant, fragment or homologue, the terms protein, polypeptide and amino acid should be replaced with nucleic acid, polynucleotide or nucleobase/nucleotide accordingly.

As used herein, the terms "membrane bound form" or "integral membrane form" refer to proteins having amino acid sequences that span a cell membrane, with amino acid sequences on each side of the membrane.

As used herein, the term "stable, secreted, ligand-binding form" or as it is sometimes known "stable, soluble, ligand-binding form." (where the terms "secreted" and "soluble" are synonymous and interchangeable herein) refer to proteins that are related to the native membrane bound form receptors, in such a way that they are secreted and stable and still capable of binding to the corresponding ligand. It should be noted that these forms are not defined by whether or not such secreted forms are physiological, only that the products of such splice variants would be secreted, stable, and still capable of ligand-binding when produced.

The term "secreted" means that the form is soluble, i.e., that it is no longer bound to the cell membrane. In this context, a form will be soluble if using conventional assays known to one of skill in the art most of this form can be detected in fractions that are not associated with the membrane, e.g., in cellular supernatants or serum.

The term "stable" means that the secreted form is detectable using conventional assays by one of skill in the art. For example, western blots, ELISA assays can be used to detect the form from harvested cells, cellular supernatants, or serum from patients.

The term "ligand-binding" means that the form retains at least some significant level, although not necessarily all, of the specific ligand-binding activity of the corresponding integral membrane form.

As used herein, the term "to reduce the activity of a ligand" refers to any action that leads to a decrease in transmission of an intracellular signal resulting from the ligand binding to or interaction with the receptor. For example, activity can be reduced by binding of the ligand to a soluble form of its receptor or by decreasing the quantity of the membrane form of its receptor available to bind the ligand.

### Pharmaceutical Compositions and Preparations

Other embodiments of the invention are pharmaceutical compositions comprising the oligomers according to the invention.

The oligomers of the present invention may be admixed, encapsulated, conjugated, or otherwise associated with other molecules, molecule structures, or mixtures of compounds, as for example liposomes, receptor targeted molecules, oral, rectal, topical or other formulations, for assisting in uptake, distribution, and/or absorption.

Formulations of the present invention comprise the oligomers according to the invention in a physiologically or pharmaceutically acceptable carrier, such as an aqueous carrier. Thus formulations for use in the present invention include, but are not limited to, those suitable for parenteral administration including intra-articular, intraperitoneal, intravenous, intraarterial, subcutaneous, or intramuscular injection or infusion, as well as those suitable for topical, ophthalmic, vaginal, oral, rectal or pulmonary administration (including inhalation or insufflation of powders or aerosols, including by nebulizer, intratracheal, and intranasal delivery). The formulations may conveniently be presented in unit dosage form and may be prepared by any of the methods well known in the art. The most suitable route of administration in any given case may depend upon the subject, the nature and severity of the condition being treated, and the particular active compound which is being used.

Pharmaceutical compositions of the present invention include, but are not limited to, physiologically and pharmaceutically acceptable salts, i.e., salts that retain the desired biological activity of the parent compound and do not impart undesired toxicological properties. Examples of such salts are (a) salts formed with cations such as sodium, potassium, NH₄⁺, magnesium, calcium, polyamines such as spermine and spermidine, etc.; (b) acid addition salts formed with inorganic acids, for example, hydrochloric acid, hydrobromic acid, sulfuric acid, phosphoric acid, nitric acid and the like; and (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, napthalenesulfonic acid, methanesulfonic acid, p-toluenesulfonic acid, napthalenedisulfonic acid, polygalacturonic acid, and the like.

The present invention provides for the use of the oligomers as set forth above for the preparation of a medicament for treating a patient afflicted with an inflammatory disorder involving excessive activity of TNF, as discussed below. In the manufacture of a medicament according to the invention, the oligomers of the present invention are typically admixed with, inter alia, an acceptable carrier. The carrier must, of course, be acceptable in the sense of being compatible with other ingredients in the formulation and must not be deleterious to the patient. The carrier may be a solid or liquid. Oligomers, nucleic acids and proteins of the present invention are incorporated in formulations, which may be prepared by any of the well known techniques of pharmacy consisting essentially of admixing the components, optionally including one or more accessory therapeutic ingredients.

Formulations of the present invention may comprise sterile aqueous and non-aqueous injection solutions of the active compounds, which preparations are preferably isotonic with the blood of the intended recipient and essentially pyrogen free. These preparations may contain anti-oxidants, buffers, bacteriostats, and solutes which render the formulation isotonic with the blood of the intended recipient. Aqueous and non-aqueous sterile suspensions can include, but are not limited to, suspending agents and thickening agents. The formulations may be presented in unit dose or multi-dose containers, for example, sealed ampoules and vials, and may be stored in freeze-dried (lyophilized) condition requiring only the addition of the sterile liquid carrier, for example, saline or water-for-injection immediately prior to use.

In the formulation the oligomers of the present invention may be contained within a particle or vesicle, such as a liposome or microcrystal, which may be suitable for parenteral administration. The particles may be of any suitable structure, such as unilamellar or plurilameller, so long as the oligomers of the present invention are contained therein. Positively charged lipids such as N-[1-(2,3-dioleoyloxy)propyl]-N,N,N-trimethyl-ammoniummethylsulfate, or "DOTAP," are particularly preferred for such particles and vesicles. The preparation of such lipid particles is well known (See references in U.S. Pat. No. 5,976,879 col. 6).

Accordingly one embodiment of the disclosure is a method of treating an inflammatory disease or condition by administering a stable, secreted, ligand-binding form of a TNF receptor, thereby decreasing the activity of TNF for the receptor. In another embodiment, the disclosure provides a method of treating an inflammatory disease or condition by administering an oligonucleotide that encodes a stable, secreted, ligand-binding form of a TNF receptor, thereby decreasing the activity of TNF for the receptor. In another embodiment, the disclosure provides a method of producing a stable, secreted, ligand-binding form of a TNF receptor.

The following aspects of the present disclosure discussed below apply to the foregoing embodiments.

The methods, nucleic acids, proteins, and formulations of the present disclosure are also useful as *in vitro* or *in vivo* tools.

Embodiments of the invention can be used to treat any condition in which the medical practitioner intends to limit the effect of TNF or a signalling pathway activated by it. In particular, the invention can be used to treat an inflammatory disease. In one embodiment, the condition is an inflammatory systemic disease, e.g., rheumatoid arthritis or psoriatic arthritis. In another embodiment, the disease is an inflammatory liver disease. Examples of inflammatory liver diseases include, but are not limited to, hepatitis associated with the hepatitis A, B, or C viruses, alcoholic liver disease, and non-alcoholic steatosis. In yet another embodiment, the inflammatory disease is a skin condition such as psoriasis.

The uses of the present invention include, but are not limited to, treatment of diseases for which known TNF antagonists have been shown useful. Three specific TNF antagonists are currently FDA-approved. The drugs are etanercept (Enbrel®), infliximab (Remicade®) and adalimumab (Humira®). One or more of these drugs is approved for the treatment of rheumatoid arthritis, juvenile rheumatoid arthritis, psoriasis, psoriatic arthritis, ankylosing spondylitis, and inflammatory bowel disease (Crohn's disease or ulcerative colitis).

### Use of proteins for the treatment of inflammatory diseases:

Accordingly one embodiment of the disclosure is a method of treating an inflammatory disease or condition by administering SSOs to a patient, The SSOs that are administered alter the splicing of a pre-mRNA to produce a splice variant that encodes a stable, secreted, ligand-binding form of a receptor of the TNFR superfamily, thereby decreasing the activity of the ligand for that receptor. In another embodiment, the disclosure provides a method of producing a stable, secreted, ligand-binding form of a receptor of the TNFR superfamily in a cell by administering SSOs to the cell.

For therapeutic use, purified TNFR proteins of the present disclosure are administered to a patient, preferably a human, for treating TNF-dependent inflammatory diseases, such as arthritis. In the treatment of humans, the use of huTNFRs is preferred. The TNFR proteins of the present disclosure can be administered by bolus injection, continuous infusion, sustained release from implants, or other suitable techniques. Typically, TNFR therapeutic proteins will be administered in the form of a composition comprising purified protein in conjunction with physiologically acceptable carriers, excipients or diluents. Such carriers will be nontoxic to recipients at the dosages and concentrations employed. Ordinarily, the preparation of such compositions entails combining the TNFR with buffers, antioxidants such as ascorbic acid, polypeptides, proteins, amino acids, carbohydrates including glucose, sucrose or dextrins, chelating agents such as EDTA, glutathione and other stabilizers and excipients. Neutral buffered saline or saline mixed with conspecific serum albumin are exemplary appropriate diluents. Preferably, product is formulated as a lyophilizate using appropriate excipient solutions, for example, sucrose, as diluents. Preservatives, such as benzyl alcohol may also be added. The amount and frequency of administration will depend of course, on such factors as the nature and the severity of the indication being treated, the desired response, the condition of the patient and so forth.

TNFR proteins of the present disclosure are administered systemically in therapeutically effective amounts preferably ranging from about 0.1 mg/kg/week to about 100 mg/kg/week. In preferred embodiments, TNFR is administered in amounts ranging from about 0.5 mg/kg/week to about 50 mg/kg/week. For local administration, dosages preferably range from about 0.01 mg/kg to about 1.0 mg/kg per injection.

### Use of expression vectors to increase the levels of a TNF antagonist in a mammal

The present disclosure provides a process of increasing the levels of a TNF antagonist in a mammal. The process includes the step of transforming cells of the mammal with an expression vector described herein, which drives expression of a TNFR as described herein.

The process is particularly useful in large mammals such as domestic pets, those used for food production, and primates. Exemplary large mammals are dogs, cats, horses cows, sheep, deer, and pigs. Exemplary primates are monkeys, apes, and humans.

The mammalian cells can be transformed either *in vivo* or *ex vivo.* When transformed *in vivo,* the expression vector are administered directly to the mammal, such as by injection. Means for transforming cells *in vivo* are well known in the art. When transformed *ex vivo,* cells are removed from the mammal, transformed *ex vivo,* and the transformed cells are reimplanted into the mammal.

The uses of the present invention include, but are not limited to, treatment of diseases for which known TNF antagonists have been shown useful. Three specific TNF antagonists are currently FDA-approved. The drugs are etanercept (Enbrel®), infliximab (Remicade®) and adalimumab (Humira®). One or more of these drugs is approved for the treatment of rheumatoid arthritis, juvenile rheumatoid arthritis, psoriasis, psoriatic arthritis, ankylosing spondylitis, and inflammatory bowel disease (Crohn's disease or ulcerative colitis).

The administration of the SSO to subjects can be accomplished using procedures developed for ASON. ASON have been successfully administered to experimental animals and human subjects by intravenous administration in saline in doses as high as 6 mg/kg three times a week (Yacysyhn, B.R., et al., 2002, Gut 51:30 (anti-ICAM-1 ASON for treatment of Crohn's disease); Stevenson, J., et al., 1999, J. Clinical Oncology 17:2227 (anti-RAF-1 ASON targeted to PBMC)). The pharmacokinetics of 2'O-MOE phosphorothioate ASON, directed towards TNF-α has been reported (Geary, R.S., et al., 2003, Drug Metabolism and Disposition 31:1419). The systemic efficacy of mixed LNA/DNA molecules has also been reported (Fluiter, K., et al., 2003, Nucleic Acids Res. 31:953).

The systemic activity of SSO in a mouse model system was investigated using 2'O-MOE phosphorothioates and PNA chemistries. Significant activity was observed in all tissues investigated except brain, stomach and dermis (Sazani, P., et al., 2002, Nature Biotechnology 20, 1228).

In general any method of administration that is useful in conventional antisense treatments can be used to administer the SSO of the invention. For testing of the SSO in cultured cells, any of the techniques that have been developed to test ASON or SSO may be used.

Formulations of the present invention comprise SSOs in a physiologically or pharmaceutically acceptable carrier, such as an aqueous carrier. Thus formulations for use in the present invention include, but are not limited to, those suitable for parenteral administration including intraperitoneal, intraarticular, intravenous, intraarterial, subcutaneous, or intramuscular injection or infusion, as well as those suitable for topical, ophthalmic, vaginal, oral, rectal or pulmonary (including inhalation or insufflation of powders or aerosols, including by nebulizer, intratracheal, intranasal delivery) administration. The formulations may conveniently be presented in unit dosage form and may be prepared by any of the methods well known in the art. The most suitable route of administration in any given case may depend upon the subject, the nature and severity of the condition being treated, and the particular active compound which is being used.

Pharmaceutical compositions of the present invention include, but are not limited to, physiologically and pharmaceutically acceptable salts ,i.e, salts that retain the desired biological activity of the parent compound and do not impart undesired toxicological properties. Examples of such salts are (a) salts formed with cations such as sodium, potassium, NH₄⁺, magnesium, calcium, polyamines such as spermine and spermidine, etc.; (b) acid addition salts formed with inorganic acids, for example, hydrochloric acid, hydrobromic acid, sulfuric acid, phosphoric acid, nitric acid and the like; and (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, napthalenesulfonic acid, methanesulfonic acid, p-toluenesulfonic acid, napthalenedisulfonic acid, polygalacturonic acid, and the like.

The present disclosure provides for the use of SSOs having the characteristics set forth above for the preparation of a medicament for increasing the ratio of a mammalian TNFR2 protein that lacks exon 7 to its corresponding membrane bound form, in a patient afflicted with an inflammatory disorder involving TNF-α, as discussed above. In the manufacture of a medicament according to the invention, the SSOs are typically admixed with, inter alia, an acceptable carrier. The carrier must, of course, be acceptable in the sense of being compatible with any other ingredients in the formulation and must not be deleterious to the patient. The carrier may be a solid or liquid. SSOs are incorporated in the formulations of the invention, which may be prepared by any of the well known techniques of pharmacy consisting essentially of admixing the components, optionally including one or more accessory therapeutic ingredients.

Formulations of the present invention may comprise sterile aqueous and non-aqueous injection solutions of the active compounds, which preparations are preferably isotonic with the blood of the intended recipient and essentially pyrogen free. These preparations may contain anti-oxidants, buffers, bacteriostats, and solutes which render the formulation isotonic with the blood of the intended recipient. Aqueous and non-aqueous sterile suspensions can include, but are not limited to, suspending agents and thickening agents. The formulations may be presented in unit dose or multi-dose containers, for example, sealed ampoules and vials, and may be stored in freeze-dried (lyophilized) condition requiring only the addition of the sterile liquid carrier, for example, saline or water-for-injection immediately prior to use.

In the formulation the SSOs may be contained within a particle or vesicle, such as a liposome, or microcrystal, which may be suitable for parenteral administration. The particles may be of any suitable structure, such as unilamellar or plurilameller, so long as the SSOs are contained therein. Positively charged lipids such as N-[1-(2,3-dioleoyloxy)propyl]-N,N,N-trimethyl-ammoniummethylsulfate, or "DOTAP," are particularly preferred for such particles and vesicles. The preparation of such lipid particles is well known. [See references in U.S. Pat. 5,976,879 col. 6]

The SSO can be targeted to any element or combination of elements that regulate splicing, including the 3'splice site, the 5' splice site, the branch point, the polypyrimidine tract, exonic splicing ehancers, exonic splicing silencers, intronic splicing enhancers, and intronic splicing silencers.

Those skilled in the art can appreciate that the disclosure as directed toward human TNFR2 can be practiced using SSO having a sequence that is complementary to at least 8, to at least 9, to at least 10, to at least 11, to at least 12, to at least 13, to at least 14, to at least 15, preferably between 10 and 16 nucleotides of the portions of the TNFR1 or TNFR2 gene comprising exons 7 and its adjacent introns.

SEQ ID No: 3 contains the sequence of exon 7 of TNFR2 and 50 adjacent nucleotides of the flanking introns. For example, SSO targeted to human TNFR2 can have a nucleobase sequence selected from the sequences listed in Table 4. When affinity-enhancing modifications are used, including but not limited to LNA or G-clamp nucleotides, the skilled person recognizes the length of the SSO can be correspondingly reduced.

Those skilled in the art will also recognize that the selection of SSO sequences must be made with care to avoid a self-complementary SSO, which may lead to the formation of partial "hairpin" duplex structures. In addition, high GC content should be avoided to minimize the possibility of non-specific base pairing. Furthermore, SSOs matching off-target genes, as revealed for example by BLAST, should also be avoided.

In some situations, it may be preferred to select an SSO sequence that can target a human and at least one other species. These SSOs can be used to test and to optimize the invention in said other species before being used in humans, thereby being useful for regulatory approval and drug development purposes. For example, SSOs with sequences selected from SEQ ID Nos: 14, 30, 46, 70 and 71 which target human TNFR2 are also 100% complementary to the corresponding Macaca Mullata sequences. As a result these sequences can be used to test treatments in monkeys, before being used in humans.

The following aspects of the present disclosure discussed below apply to the foregoing embodiments.

The length of the SSO is similar to an antisense oligonucleotide (ASON), typically between about 10 and 24 nucleotides. The disclosure can be practiced with SSOs of several chemistries that hybridize to RNA, but that do not activate the destruction of the RNA by RNase H, as do conventional antisense 2^{r}-deoxy oligonucleotides. The disclosure can be practiced using 2'0 modified nucleic acid oligomers, such as 2'O-methyl or 2'O-methyloxyethyl phosphorothioate. The nucleobases do not need to be linked to sugars; so-called peptide nucleic acid oligomers or morpholine-based oligomers can be used. A comparison of these different linking chemistries is found in Sazani, P. et al, 2001, Nucleic Acids Res. 29:3695. The term splice-switching oligonucleotide is intended to cover the above forms. Those skilled in the art will appreciate the relationship between antisense oligonucleotide gapmers and SSOs. Gapmers are ASON that contain an RNase H activating region (typically a 2'-deoxyribonucleoside phosphorothioate) which is flanked by non-activating nuclease resistant oligomers. In general, any chemistry suitable for the flanking sequences in a gapmer ASON can be used in an SSO.

The SSOs of this invention may be made through the well-known technique of solid phase synthesis. Any other means for such synthesis known in the art may additionally or alternatively be used. It is well known to use similar techniques to prepare oligonucleotides such as the phosphorothioates and alkylated derivatives.

A particularly preferred chemistry is provided by locked nucleic acids (LNA) (Koshkin, A. A., et al., 1998, Tetrahedron 54:3607; Obika, S., et al., 1998, Tetrahedron Lett. 39:5401). LNA are conventional phosphodiester-linked ribonucleotides, except the ribofuranosyl moiety is made bicyclic by a bridge between the 2'0 and the 4¹C. This bridge constrains the conformation of ribofuranosyl ring into the conformation, the V-endo conformation, which is adopted when a oligonucleotide hybridizes to a complementary RNA. Recent advances in the synthesis of LNA are described in WO 03/095467. The bridge is most typically a methylene or an ethylene. The synthesis of 2'0,4'C-ethylene-bridged nucleic acids (ENA), as well as other LNA, is described in Morita, et al., 2003, Bioorg. and Med. Chem. 11:2211. However, alternative chemistries can be used and the 2'O may be replaced by a 2'N. LNA and conventional nucleotides can be mixed to form a chimeric SSO. For example, chimeric SSO of alternating LNA and 2'deoxynucleotides or alternating LNA and 2'O-Me or 2'O-MOE can be employed. An alternative to any of these chemistries, not merely the 2'-deoxynucleotides, is a phosphorothioatediester linkage replacing phosphodiester. For in vivo use, phosphorothioate linkages are preferred.

When LNA nucleotides are employed in an SSO it is preferred that non-LNA nucleotides also be present. LNA nucleotides have such high affinities of hybridization that there can be significant non-specific binding, which may reduce the effective concentration of the free-SSO. When LNA nucleotides are used they may be alternated conveniently with 2'-deoxynucleotides. Alternating nucleotides, alternating dinucleotides or mixed patterns, e.g., LDLDLD or LLDLLD or LDDLDD can be used, When 2'-deoxynucleotides or 2'-deoxynucleoside phosphorothioates are mixed with LNA nucleotides it is important to avoid RNase H activation. It is expected that between about one third and two thirds of the LNA nucleotides of an SSO will be suitable. For example if the SSO is a 12-mer, then at least four LNA nucleotides and four conventional nucleotides will be present.

The bases of the SSO may be the conventional cytosine, guanine, adenine and uracil or thymidine. Alternatively modified bases can also be used. Of particular interest are modified bases that increase binding affinity. One non-limiting example of preferred modified bases are the so-called G-clamp or 9-(aminoethoxy)phenoxazine nucleotides, cytosine analogs that form 4 hydrogen bonds with guanosine. (Flanagan, W.M., et al., 1999, Proc. Natl. Acad. Sci. 96:3513; Holmes, S.C., 2003, Nucleic Acids Res. 31:2759).

Numerous alternative chemistries which do not activate RNase H are available. For example, suitable SSOs may be oligonucleotides wherein at least one, or all, of the internucleotide bridging phosphate residues are modified phosphates, such as methyl phosphonates, methyl phosphonothioates, phosphoromorpholidates, phosphoropiperazidates, and phosphoroamidates. For example, every other one of the internucleotide bridging phosphate residues may be modified as described. In another non-limiting example, such SSO are oligonucleotides wherein at least one, or all, of the nucleotides contain a 2' loweralkyl moiety (e.g., C1-C4, linear or branched, saturated or unsaturated alkyl, such as methyl, ethyl, ethenyl, propyl, 1-propenyl, 2-propenyl, and isopropyl). For example, every other one of the nucleotides may be modified as described. [See references in U.S. Pat. 5,976,879 col. 4].

The length of the SSO (i.e. the number of monomers in the oligomer) will be from about 10 to about 30 bases in length. In one embodiment, 20 bases of 2'O-Me-ribonucleosides phosphorothioates are effective. Those skilled in the art appreciate that when affinity-increasing chemical modifications are used, the SSO can be shorter and still retain specificity. Those skilled in the art will further appreciate that an upper limit on the size of the SSO is imposed by the need to maintain specific recognition of the target sequence, and to avoid secondary-structure forming self hybridization of the SSO and by the limitations of gaining cell entry. These limitations imply that an SSO of increasing length (above and beyond a certain length which will depend on the affinity of the SSO) will be more frequently found to be less specific, inactive or poorly active.

SSOs of the invention include, but are not limited to, modifications of the SSO involving chemically linking to the SSO one or more moieties or conjugates which enhance the activity, cellular distribution or cellular uptake of the SSO. Such moieties include, but are not limited to, lipid moieties such as a cholesterol moiety, cholic acid, a thioether, e.g. hexyl- S-tritylthiol, a thiocholesterol, an aliphatic chain, e.g., dodecandiol or undecyl residues, a phospholipids, e.g., di-hexadecyl-rac-glycerol or triethylammonium 1,2-di-O-hexadecyl-rac-glycero-3-H-phosphonate, a polyamine or a polyethylene glycol chain, an adamantane acetic acid, a palmityl moiety, an octadecylamine or hexylamino-carbonyl-oxycholesterol moiety.

It is not necessary for all positions in a given SSO to be uniformly modified, and in fact more than one of the aforementioned modifications may be incorporated in a single compound or even at a single nucleoside within an SSO.

The SSOs may be admixed, encapsulated, conjugated, or otherwise associated with other molecules, molecule structures, or mixtures of compounds, as for example liposomes, receptor targeted molecules, oral, rectal, topical or other formulation, for assisting in uptake, distribution, and/or absorption.

Those skilled in the art appreciate that cellular differentiation includes, but is not limited to, differentiation of the spliceosome. Accordingly, the activity of any particular SSO of the invention can depend upon the cell type into which they are introduced. For example, SSOs which are effective in cell type may be ineffective in another cell type.

The methods, oligonucleotides, and formulations of the present disclosure are also useful as in vitro or in vivo tools to examine splicing in human or animal genes. Such methods can be carried out by the procedures described herein, or modifications thereof which will be apparent to skilled persons.

The disclosure can be used to treat any condition in which the medical practitioner intends to limit the effect of a TNF superfamily ligand or the signalling pathway activated by such ligand. In particular, the invention can be used to treat an inflammatory disease. In one embodiment, the condition is an inflammatory systemic disease, e.g., rheumatoid arthritis or psoriatic arthritis. In another embodiment, the disease is an inflammatory liver disease. Examples of inflammatory liver diseases include, but are not limited to, hepatitis associated with the hepatitis A, B, or C viruses, alcoholic liver disease, and non-alcoholic steatosis. In yet another embodiment, the inflammatory disease is a skin condition such as psoriasis.

The uses of the present invention include, but are not limited to, treatment of diseases for which known TNF antagonists have been shown useful. Three specific TNF antagonists are currently FDA-approved. The drugs are etanercept (Enbrel®), infliximab (Remicade®) and adalimumab (Humira®). One or more of these drugs is approved for the treatment of rheumatoid arthritis, juvenile rheumatoid arthritis, psoriasis, psoriatic arthritis, ankylosing spondylitis, and inflammatory bowel disease (Crohn's disease or ulcerative colitis).

In a preferred embodiment, the receptor is either the TNFR1 or TNFR2 receptors. In other embodiments, the receptor is a member of the TNFR superfamily that is sufficiently homologous to TNFR1 and TNFR2, e.g., TNFRSF3, TNFRSF5, or TNFRSFI IA, so that deletion of either or both exons homologous to exons 7 and 8 results in a secreted form. Those skilled in the art appreciate that the operability of the invention is not determined by whether or not such secreted forms are physiological, only that the products of such splice variants are secreted, stable, and capable of ligand-binding.

The administration of the SSO to subjects can be accomplished using procedures developed for ASON. ASON have been successfully administered to experimental animals and human subjects by intravenous administration in saline in doses as high as 6 mg/kg three times a week (Yacysyhn, B.R., et al, 2002, Gut 51:30 (anti-ICAM-1 ASON for treatment of Crohn's disease); Stevenson, J., et al., 1999, J. Clinical Oncology 17:2227 (anti-RAF-1 ASON targeted to PBMC)). The pharmacokinetics of 2'O-MOE phosphorothioate ASON, directed towards TNF-alpha has been reported (Geary, R.S., et al., 2003, Drug Metabolism and Disposition 31:1419). The systemic efficacy of mixed LNA/DNA molecules has also been reported (Fluiter, K., et al., 2003, Nucleic Acids Res. 31:953).

The systemic activity of SSO in a mouse model system was investigated using 2'O-MOE phosphorothioates and PNA chemistries. Significant activity was observed in all tissues investigated except brain, stomach and dermis (Sazani, P., et al., 2002, Nature Biotechnology 20, 1228).

In general any method of administration that is useful in conventional antisense treatments can be used to administer the SSO of the invention. For testing of the SSO in cultured cells, any of the techniques that have been developed to test ASON or SSO may be used.

Formulations of the present invention comprise SSOs in a physiologically or pharmaceutically acceptable carrier, such as an aqueous carrier. Thus formulations for use in the present invention include, but are not limited to, those suitable for parenteral administration including intraperitoneal, intravenous, intraarterial, subcutaneous, or intramuscular injection or infusion., as well as those suitable topical (including ophthalmic and to mucous membranes including vaginal delivery), oral, rectal or pulmonary (including inhalation or insufflation of powders or aerosols, including by nebulizer, intratracheal, intranasal delivery) administration. The formulations may conveniently be presented in unit dosage form and may be prepared by any of the methods well known in the art. The most suitable route of administration in any given case may depend upon the subject, the nature and severity of the condition being treated, and the particular active compound which is being used.

Pharmaceutical compositions of the present invention include, but are not limited to, the physiologically and pharmaceutically acceptable salts thereof: i.e, salts that retain the desired biological activity of the parent compound and do not impart undesired toxicological effects thereto. Examples of such salts are (a) salts formed with cations such as sodium, potassium, NH₄⁺, magnesium, calcium, polyamines such as spermine and spermidine, etc.; (b) acid addition salts formed with inorganic acids, for example, hydrochloric acid, hydrobromic acid, sulfuric acid, phosphoric acid, nitric acid and the like; (c) salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, napthalenesulfonic acid, methanesulfonic acid, p-toluenesulfonic acid, napthalenedisulfonic acid, polygalacturonic acid, and the like; and (d) salts formed from elemental anions such as chlorine, bromine, and iodine.

The present disclosure provides for the use of SSOs having the characteristics set forth above for the preparation of a medicament for increasing the ratio of a soluble form of a TNFR superfamily member to its corresponding membrane bound form, in a patient afflicted with an inflammatory disorder involving excessive activity of a cytokine, such as TNF-α, as discussed above. In the manufacture of a medicament according to the invention, the SSOs are typically admixed with, inter alia, an acceptable carrier. The carrier must, of course, be acceptable in the sense of being compatible with any other ingredients in the formulation and must not be deleterious to the patient. The carrier may be a solid or liquid. SSOs are incorporated in the formulations of the invention, which may be prepared by any of the well known techniques of pharmacy consisting essentially of admixing the components, optionally including one or more accessory therapeutic ingredients.

Formulations of the present invention may comprise sterile aqueous and nonaqueous injection solutions of the active compounds, which preparations are preferably isotonic with the blood of the intended recipient and essentially pyrogen free. These preparations may contain anti-oxidants, buffers, bacteriostats, and solutes which render the formulation isotonic with the blood of the intended recipient. Aqueous and non-aqueous sterile suspensions can include, but are not limited to, suspending agents and thickening agents. The formulations may be presented in unit dose or multi-dose containers, for example, sealed ampoules and vials, and may be stored in freeze-dried (lyophilized) condition requiring only the addition of the sterile liquid carrier, for example, saline or water- for-injection immediately prior to use.

In the formulation the SSOs may be contained within a lipid particle or vesicle, such as a liposome or microcrystal, which may be suitable for parenteral administration. The particles may be of any suitable structure, such as unilamellar or plurilameller, so long as the SSOs are contained therein. Positively charged lipids such as N-[I-(2,3-dioleoyloxi)propyl]- N,N,N-trimethyl-ammoniummethylsulfate, or "DOTAP," are particularly preferred for such particles and vesicles. The preparation of such lipid particles is well known. [See references in U.S. Pat. 5,976,879 col. 6]

The SSO can be targeted to any element or combination of elements that regulate splicing, including the 3 'splice site, the 5' splice site, the branch point, the polypyrimidine tract, exonic splicing ehancers, exonic splicing silencers, intronic splicing enhancers, and intronic splicing silencers. The determination of the sequence of the SSO can be guided by the following tables that shows the activities of the SSOs whose sequences and locations are found as depicted in Figure 20. The person skilled in the art will note that: 1) SSOs complementary to the exon need not be complementary to either the splice acceptor or splice donor sites, note SSOs A7-10, B7-7 and B7-9, Table 1; 2) SSOs complementary to sequences of the intron and as few as one nucleotide of the exon can be operative, note A8-5 and B7-6,

Table 1; 3) SSOs complementary to the intron immediately adjacent to the exon can also be effective, note 3312, Table 2; and 4) efficacy of an oligonucleotide alone is usually predictive of the efficacy of the SSO in combination with other SSOs.

Those skilled in the art can appreciate that the disclosure as directed toward human TNF-alpha receptors can be practiced using SSO having a sequence that is complementary to at least 10, preferably between 15 and 20 nucleotides of the portions of the TNFR1 or TNFR2 genes comprising exons 7 or 8 and their adjacent introns. It is further preferred that at least one nucleotide of the exon itself is included within the complementary sequence. SEQ ID Nos: 1-4 contain the sequence of Exons 7 and 8 of the TNFR1 (SEQ ID Nos: 1 and 2) and TNFR2 (SEQ ID Nos; 3 and 4) and 50 adjacent nucleotides of the flanking introns. When affinity-enhancing modifications are used, including but not limited to LNA or G-clamp nucleotides, the skilled person recognizes the length of the SSO can be correspondingly reduced. When alternating conventional and LNA nucleotides are used a length of 16 is effective.

Those skilled in the art will also recognize that the selection of SSO sequences must be made with care to avoid self-complementary SSO, which may lead to the formation of partial "hairpin" duplex structures. In addition, high GC content should be avoided to minimize the possibility of non-specific base pairing. Furthermore, SSOs matching off-target genes, as revealed for example by BLAST, should also be avoided.

In some situations, it may be preferred to select an SSO sequence that can target a human and at least one other species. These SSOs can be used to test and to optimize the disclosure in said other species before being used in humans, thereby being useful for regulatory approval and drug development purposes. For example, SEQ ID Nos: 74, 75, 77, 78, 80, and 89, which target human TNFR2 are also 100% complementary to the corresponding Macaca Mullata sequences. As a result these sequences can be used to test treatments in monkeys, before being used in humans.

In the sequence listing below, SEQ ID NOs 1 - 116 are as disclosed in WO2007/058894. SEQ IDs NOs 117 - 242 are as disclosed as SEQ ID NOs 1 - 126 of PCT/US2007/10557. SEQ IDs NOs 243 - 246 are new to the present application.

**Table 4: Splice Switching Oligomers targeting human TNFR2: Capital letters = LNA, small letters = DNA) - Note SEQ ID No 243 targets themouse TNFR2.**

| **3378** | | | | |
|---|---|---|---|---|
| SEQ ID | Name | Sequence (5'-3') | Description | Nucleobase Motif |
| 130 | SK100 | CcA cAa TcA gTc CtA g | 3378 Full Length | CCA CAA TCA GTC CTA G |
| 131 | SK101 | AcAa TcA gTc CtA g | -2nt 5' (14mer) | A CAA TCA GTC CTA G |
| 132 | SK102 | Aa TcA gTc CtA g | -4nt 5' (12mer) | AA TCA GTC CTA G |
| 133 | SK103 | TcA gTc CtA g | -6nt 5' (10mer) | TCA GTC CTA G |
| 134 | SK104 | CcA cAa TcA gTc Ct | -2nt 3' (14mer) | CCA CAA TCA GTC CT |
| 135 | SK105 | CcA cAa TcA gTc | -4nt 3' (12mer) | CCA CAA TCA GTC |
| 136 | SK106 | CcA cAa TcA g | -6nt 3' (10mer) | CCA CAA TCA G |
| 137 | SK107 | Ca CaA tCa GtC cTa | -1nt 5';-1nt 3' (14mer) | CA CAA TCA GTC CTA |
| 138 | SK108 | Ca CaA tCa GtC c | -1nt 5';-3nt 3' (12mer) | CA CAA TCA GTC C |
| 139 | SK109 | A cAa TcA gTc Ct | -2nt 5';-2nt 3' (12mer) | A CAA TCA GTC CT |
| 140 | SK110 | CaA tCa GtC cTa | -3nt 5';-1nt 3' (12mer) | CAA TCA GTC CTA |
| 141 | SK111 | Ca CaA tCa Gt | -1nt 5';-5nt 3' (10mer) | CA CAA TCA GT |
| 142 | SK112 | A cAa TcA gTc | -2nt 5';-4nt 3' (10mer) | A CAA TCA GTC |
| 143 | SK113 | CaA tCa GtC c | -3nt 5';-3nt 3' (10mer) | CAA TCA GTC C |
| 144 | SK114 | Aa TcA gTc Ct | -4nt 5';-2nt 3' (10mer) | AA TCA GTC CT |
| 145 | SK115 | A tCa GtC cTa | -5nt 5';-1nt 3' (10mer) | A TCA GTC CTA |

| **3379** | | | | |
|---|---|---|---|---|
| SEQ ID | Name | Sequence (5'-3') | Description | Nucleobase Motif |
| 146 | SK116 | CaG tCc TaG aAa GaA a | 3379 Full Length | CCA CAA TCA GTC CTA G |
| 147 | SK117 | G tCc TaG aAa GaA a | -2nt 5' (14mer) | G TCC TAG AAA GAA A |
| 148 | SK118 | CcTaG aAa GaA | -4nt 5' (12mer) | CC TAG AAA GAA A |
| 149 | SK119 | TaGaAa GaA a | -6nt 5' (10mer) | TAG AAA GAA A |
| 150 | SK120 | CaG tCc TaG aAa Ga | -2nt 3' (14mer) | CAG TCC TAG AAA GA |
| 151 | SK121 | CaG tCc TaG aAa | -4nt 3' (12mer) | CAG TCC TAG AAA |
| 152 | SK122 | CaG tCc TaG a | -6nt 3' (10mer) | CAG TCC TAG A |
| 153 | SK123 | Ag TcC tAg AaA gAa | -1nt 5';-1nt 3' (14mer) | AG TCC TAG AAA GAA |
| 154 | SK124 | Ag TcC tAg AaA g | -1nt 5';-3nt 3' (12mer) | AG TCC TAG AAA G |
| 155 | SK125 | G tCc TaG aAa Ga | -2nt 5';-2nt 3' (12mer) | G TCC TAG AAA GA |
| 156 | SK126 | TcC tAg AaA gAa | -3nt 5';-1nt 3' (12mer) | TCC TAG AAA GAA |
| 157 | SK127 | Ag TcC tAg Aa | -1nt 5';-5nt 3' (10mer) | AG TCC TAG AA |
| 158 | SK128 | G tCc TaG aAa | -2nt 5';-4nt 3' (10mer) | G TCC TAG AAA |
| 159 | SK129 | TcC tAg AaA g | -3nt 5';-3nt 3' (10mer) | TCC TAG AAA G |
| 160 | SK130 | Cc TaG aAa Ga | -4nt 5';-2nt 3' (10mer) | CC TAG AAA GA |
| 161 | SK131 | C tAg AaA gAa | -5nt 5';-1nt 3' (10mer) | C TAG AAA GAA |

| **3384** | | | | |
|---|---|---|---|---|
| SEQ ID | Name | Sequence (5'-3') | Description | Nucleobase Motif |
| 162 | SK132 | AcT tTt CaC cTg GgT c | 3384 Full Length | CCA CAA TCA GTC CTA G |
| 163 | SK133 | T tTt CaC cTg GgT c | -2nt 5' (14mer) | T TTT CAC CTG GGT C |
| 164 | SK134 | Tt CaC cTg GgT c | -4nt 5' (12mer) | TT CAC CTG GGT C |
| 165 | SK135 | CaC cTg GgT c | -6nt 5' (10mer) | CAC CTG GGT C |
| 166 | SK136 | AcT tTt CaC cTg Gg | -2nt 3' (14mer) | ACT TTT CAC CTG GG |
| 167 | SK137 | AcT tTt CaC cTg | -4nt 3' (12mer) | ACT TTT CAC CTG |
| 168 | SK138 | AcT tTt CaC c | -6nt 3' (10mer) | ACT TTT CAC C |
| 169 | SK139 | Ct TtT cAc CtG gGt | -1nt 5';-1nt 3' (14mer) | CT TTT CAC CTG GGT |
| 170 | SK140 | Ct TtT cAc CtG g | -1nt 5';-3nt 3' (12mer) | CT TTT CAC CTG G |
| 171 | SK141 | T tTt CaC cTg Gg | -2nt 5';-2nt 3' (12mer) | T TTT CAC CTG GG |
| 172 | SK142 | TtT cAc CtG gGt | -3nt 5';-1nt 3' (12mer) | TTT CAC CTG GGT |
| 173 | SK143 | Ct TtT cAc Ct | -1nt 5';-5nt 3' (10mer) | CT TTT CAC CT |
| 174 | SK144 | T tTt CaC cTg | -2nt 5';-4nt 3' (10mer) | T TTT CAC CTG |
| 175 | SK145 | TtT cAc CtG g | -3nt 5';-3nt 3' (10mer) | TTT CAC CTG G |
| 176 | SK146 | Tt CaC cTg Gg | -4nt 5';-2nt 3' (10mer) | TT CAC CTG GG |
| 177 | SK147 | T cAc CtG gGt | -5nt 5';-1nt 3' (10mer) | T CAC CTG GGT |

| SEQ ID No | Sequence | Length | | |
|---|---|---|---|---|
| SEQ ID 243 | | 16 | | |
| SEQ ID 244 SK100 | | 16 | | |
| SEQ ID 245 SK107 | | 14 | | |
| SEQ ID 246 SK109 | | 12 | | |
| SEQ ID 251 SK101 | | 14 | | |
| SEQ ID 252 SK102 | | 12 | | |
| SEQ ID 253 SK103 | | 10 | | |
| SEQ ID 254 SK104 | | 14 | | |
| SEQ ID 255 SK105 | | 12 | | |
| SEQ ID 256 SK106 | | 10 | | |
| SEQ ID 257 SK108 | | 12 | | |
| SEQ ID 258 SK110 | | 12 | | |
| SEQ ID 259 SK111 | | 10 | | |
| SEQ ID 260 SK112 | | 10 | | |
| SEQ ID 261 SK113 | | 10 | | |
| SEQ ID 262 SK114 | | 10 | | |
| SEQ ID 263 SK115 | | 10 | | |
| SEQ ID 264 SK116 | | 16 | | |
| SEQ ID 265 SK117 | | 14 | | |
| SEQ ID 266 SK118 | | 12 | | |
| SEQ ID 267 SK119 | | 10 | | |
| SEQ ID 268 SK120 | | 14 | | |
| SEQ ID 269 SK121 | | 12 | | |
| SEQ ID 270 SK122 | | 10 | | |
| SEQ ID 271 SK123 | | 14 | | |
| SEQ ID 272 SK124 | | 12 | | |
| SEQ ID 273 SK125 | | 12 | | |
| SEQ ID 274 SK126 | | 12 | | |
| SEQ ID 275 SK127 | | 10 | | |
| SEQ ID 276 SK128 | | 10 | | |
| SEQ ID 277 SK129 | | 10 | | |
| SEQ ID 278 SK130 | | 10 | | |
| SEQ ID 279 SK131 | | 10 | | |
| SEQ ID 280 SK132 | | 16 | | |
| SEQ ID 281 SK133 | | 14 | | |
| SEQ ID 282 SK134 | | 12 | | |
| SEQ ID 283 SK135 | | 10 | | |
| SEQ ID 284 SK136 | | 14 | | |
| SEQ ID 285 SK137 | | 12 | | |
| SEQ ID 286 SK138 | | 10 | | |
| SEQ ID 287 SK139 | | 14 | | |
| SEQ ID 288 SK140 | | 12 | | |
| SEQ ID 289 SK141 | | 12 | | |
| SEQ ID 290 SK142 | | 12 | | |
| SEQ ID 291 SK143 | | 10 | | |
| SEQ ID 292 SK144 | | 10 | | |
| SEQ ID 293 SK145 | | 10 | | |
| SEQ ID 294 SK146 | | 10 | | |
| SEQ ID 295 SK147 | | 10 | | |

| | | | | |
|---|---|---|---|---|
| Capital letters = LNA, preferably oxy LNA (superscript o), preferably phosphorothioate linkages = subscript s, small letters = DNA). ^{m}C = preferably, 5-methylcytosine. | | | | |

Tables 1 - 3 are as according to tables 1 - 3 of WO 2007/058894.

Further embodiments of the disclosure:
The disclosure provides for a method of treating an inflammatory disease or condition which comprises administering one or more splice switching oligomers (SSOs) to a subject for a time and in an amount to reduce the activity of a ligand for a receptor of the tumor necrosis factor receptor (TNFR) superfamily, wherein said one or more SSOs are capable of altering the splicing of a pre-mRNA encoding said receptor to increase production of a stable, secreted, ligand-binding form of said receptor.

In one embodiment the mammalian receptor selected from the group consisting of TNFRSF1A, TNFRSF1B, TNFRSF3, TNFRSF5, TNFRSF8, and TNFRSF11A.

In one embodiment the receptor is a human TNFRSF1A or a human TNFRSF1B.

In one embodiment the receptor is a human TNFRSF1B.

In one embodiment the ligand is TNF-.alpha., RANKL, CD40L, LT-.alpha., or LT-.beta.

In one embodiment the disease or condition is selected from the group consisting of rheumatoid arthritis, juvenile rheumatoid arthritis, psoriasis, psoriatic arthritis, ankylosing spondylitis, inflammatory bowel disease (Crohn's disease or ulcerative colitis), hepatitis, sepsis, alcoholic liver disease, and non-alcoholic steatosis.

In one embodiment of the method of treating an inflammatory disease or condition the two or more SSOs are administered.

In one embodiment the receptor is TNFRSF1A, TNFRSF1B, TNFRSF3, TNFRSF5, or TNFRSF11A, and said altering the splicing of said pre-mRNA comprises excising exon 7, exon 8, or both from said pre-mRNA.

In one embodiment said altering the splicing of said pre-mRNA comprises excising exon 7.

In one embodiment said receptor is a human TNFRSF1A or a human TNFRSF1B, and said SSO comprises from at least 10 to at least 20 nucleotides which are complementary to a contiguous sequence from SEQ ID Nos: 1, 2, 3 or 4.

In one embodiment the sequence of said SSO comprises a sequence selected from the group consisting of SEQ ID Nos: 74, 75, 77, 78, 80, 82, 84, and 86-89.

In one embodiment said SSOs comprise one or more nucleotides or nucleosides independently selected from the group consisting of 2'-deoxyribonucleotides, 2'O-Me ribonucleotides, 2'O-MOE ribonucleotides, hexitol (HNA) nucleotides or nucleosides, 2'O-4'Clinked bicyclic ribofuranosyl (LNA) nucleotides or nucleosides, phosphorothioate analogs of any of the foregoing, peptide nucleic acid (PNA) analogs of any of the foregoing; methylphosponate analogs of any of the foregoing, peptide nucleic acid analogs of any of the foregoing, N3'.fwdarw.P5' phosphoramidate analogs of any of the foregoing, and phosphorodiamidate morpholino nucleotide analogs of any of the foregoing, and combinations thereof.

In one embodiment said SSOs comprise one or more nucleotides or nucleosides independently selected from the group consisting of 2'O-Me ribonucleotides and 2'O-4'Clinked bicyclic ribofuranosyl (LNA) nucleotides or nucleosides.

In one embodiment said administration is parenteral, topical, oral, rectal, or pulmonary.

In one embodiment the disclosure provides for a method of increasing the production of a stable, secreted, ligand-binding form of a receptor from the TNFR superfamily in a cell, which comprises administering one or more splice switching oligomers (SSOs) to said cell, wherein said one or more SSOs are capable of altering the splicing of a pre-mRNA encoding said receptor to increase production of a stable, secreted, ligand-binding form of said receptor.

In one embodiment the method is performed in vivo.

In one embodiment said receptor is a mammalian receptor selected from the group consisting of TNFRSF1A, TNFRSF1B, TNFRSF3, TNFRSF5, TNFRSF8, and TNFRSF1A.

In one embodiment said receptor is a human TNFRSF1A or a human TNFRSF1B.

In one embodiment said receptor is a human TNFRSF1B.

In one embodiment said SSO comprises from at least 10 to at least 20 nucleotides which are complementary to a contiguous sequence from SEQ ID Nos: 1, 2, 3 or 4.

In one embodiment the disclosure provides for a splice switching oligomer (SSO) comprising from at least 10 to at least 20 nucleotides, said SSO capable of altering the splicing of a pre-mRNA encoding a receptor from the TNFR superfamily to produce a stable, secreted, ligand-binding form of said receptor.

In one embodiment said receptor is a mammalian receptor selected from the group consisting of TNFRSF1A, TNFRSF1B, TNFRSF3, TNFRSF5, TNFRSF8, and TNFRSF11A.

In one embodiment said receptor is a human TNFSF1A or a human TNFRSF1B.

In one embodiment said receptor is a human TNFRSF1B.

In one embodiment the SSO comprises from at least 10 to at least 20 nucleotides which are complementary to a contiguous sequence from SEQ ID Nos: 1, 2, 3 or 4.

In one embodiment the SSO comprises one or more nucleotides or nucleosides independently selected from the group consisting of 2'-deoxyribonucleotides, 2'O-Me ribonucleotides, 2'O-MOE ribonucleotides, hexitol (HNA) nucleotides or nucleosides, 2'O-4'C-linked bicyclic ribofuranosyl (LNA) nucleotides or nucleosides, phosphorothioate analogs of any of the foregoing, peptide nucleic acid (PNA) analogs of any of the foregoing; methylphosponate analogs of any of the foregoing, peptide nucleic acid analogs of any of the foregoing, N3'.fwdarw.P5' phosphoramidate analogs of any of the foregoing, and phosphorodiamidate morpholino nucleotide analogs of any of the foregoing, and combinations thereof.

In one embodiment said 2'O-4'C-linked bicyclic ribofuranosyl (LNA) nucleotides or nucleosides are 2'0-4'C-(methylene)-ribofuranosyl nucleotides or nucleosides, respectively, or 2'O-4'C-(ethylene)-ribofuranosyl nucleotides or nucleosides, respectively.

In one embodiment said SSOs comprise one or more nucleotides or nucleosides independently selected from the group consisting of 2'O-Me ribonucleotides and 2'O-4'Clinked bicyclic ribofuranosyl (LNA) nucleotides or nucleosides.

In one embodiment the sequence of said SSO comprises a sequence selected from the group consisting of SEQ ID Nos: 8, 9, 14, 17-21, 24-29, 32, 33, 38-42, 44-46, 50-52, 55-57, 60, 68-71, 74, 75, 77, 78, 80, 82, 84, and 86-89.

In one embodiment the disclosure provides a pharmaceutical composition comprising the SSO and a pharmaceutically acceptable carrier.

In one embodiment said SSO comprises from at least 10 to at least 20 nucleotides which are complementary to a contiguous sequence from SEQ ID Nos: 1, 2, 3 or 4.

In one embodiment the disclosure provides an isolated protein capable of binding tumor necrosis factor (TNF), said protein having a sequence comprising the amino acids encoded by a cDNA derived from a mammalian tumor necrosis factor receptor (TNFR) gene, wherein the cDNA comprises in 5'to 3' contiguous order, the codon encoding the first amino acid after the cleavage point of the signal sequence of said gene through exon 6 of said gene and exon 8 of said gene through exon 10 of said gene; or the codon encoding the first amino acid of the open reading frame of said gene through exon 6 of said gene and exon 8 of said gene through exon 10 of said gene.

In one embodiment said TNF is TNF-α.

In one embodiment said protein contains at least one processing, chemical, or post-translational modification, and wherein said modification is selected from the group consisting of acetylation, acylation, amidation, ADP-ribosylation, glycosylation, methylation, pegylation, prenylation, phosphorylation, or cholesterol conjugation.

In one embodiment said receptor is TNFR1, such as human TNFR1, In one embodiment, said receptor is TNFR2, such as human TNFR2. In one embodiment said protein comprises a sequence selected from the group consisting of SEQ ID No: 6, amino acids 30-417 of SEQ ID No: 6, SEQ ID No: 8, amino acids 30-416 of SEQ ID No: 8, SEQ ID No: 10, amino acids 23-435 of SEQ ID No: 10, SEQ ID No: 12, and amino acids 23-448 of SEQ ID No: 12. In one embodiment, the disclosure provides a pharmaceutical composition comprising the protein according to the disclosure in admixture with a pharmaceutically acceptable carrier. In one embodiment, the disclosure provides a composition comprising the purified protein according to the disclosure.

In one embodiment, the disclosure provides a method of treating an inflammatory disease or condition which comprises administering the pharmaceutical composition according to the disclosure to a subject for a time and in an amount effective to reduce the activity of TNF. In one embodiment, said disease or condition is selected from the group consisting of rheumatoid arthritis, juvenile rheumatoid arthritis, psoriasis, psoriatic arthritis, ankylosing spondylitis, inflammatory bowel disease (Crohn's disease or ulcerative colitis), hepatitis associated with hepatitis A virus, hepatitis associated with hepatitis B virus, hepatitis associated with hepatitis C virus, hepatitis associated with ischemia/reperfusion, sepsis, alcoholic liver disease, and non-alcoholic steatosis. In one embodiment, the disclosure provides an isolated nucleic acid derived from a mammalian tumor necrosis factor receptor (TNFR) gene and encoding a protein capable of binding tumor necrosis factor (TNF), wherein the cDNA of said protein comprises in 5' to 3' contiguous order, the codon encoding the first amino acid after the cleavage point of the signal sequence of said gene through exon 6 of said gene and exon 8 of said gene through exon 10 of said gene; or the codon encoding the first amino acid of the open reading frame of said gene through exon 6 of said gene and exon 8 of said gene through exon 10 of said gene. In such as embodiment, the sequence of said protein comprises a sequence selected from the group consisting of SEQ ID No: 6, amino acids 30-417 of SEQ ID No: 6, SEQ ID No: 8, amino acids 30-416 of SEQ ID No: 8, SEQ ID No: 10, amino acids 23-435 of SEQ ID No: 10, SEQ ID No: 12, and amino acids 23-448 of SEQ ID No: 12.. In one embodiment, the sequence of said nucleic acid comprises a sequence selected from the group consisting of nucleotides 1-1251 of SEQ ID No: 5, nucleotides 88-1251 of SEQ ID No: 5, nucleotides 1-1248 of SEQ ID No: 7, nucleotides 88-1248 of SEQ ID No: 7, nucleotides 1-1305 of SEQ ID No: 9, nucleotides 67-1305 of SEQ ID No: 9, nucleotides 1-1344 of SEQ ID No: 11, and nucleotides 67-1344 of SEQ ID No: 11. In one embodiment, the disclosure provides for an expression vector comprising the nucleic acid of the disclosure operably linked to a regulatory sequence.

In one embodiment, the disclosure provides a method of increasing the level of a TNF antagonist in a mammal which comprises transforming cells of said mammal with the expression vector according to the disclosure to thereby express said TNF antagonist, wherein said vector drives expression of said TNFR.

In one embodiment the mammal is a human, such as a human is an individual having an inflammatory disease or condition.

In one embodiment said expression vector is a plasmid, or a virus.

In one embodiment cells are transformed in vivo.

In one embodiment cells are transformed ex vivo.

In one embodiment, said expression vector comprises a tissue specific promoter - said tissue specific promoter may, for example be derived from a hepatocyte or a macrophage.

In one embodiment the cells are selected from the group consisting of hepatocytes, hematopoietic cells, spleen cells, and muscle cells.

The disclosure provides for a cell transformed with the expression vector of the disclosure, such as a mammalian cell, an insect cell, or a microbial cell.

The disclosure provides for a process for producing a protein capable of binding tumor necrosis factor (TNF) which comprises culturing the cell of the disclosure under conditions suitable to express said protein, and recovering said protein.

The disclosure provides for a pharmaceutical composition comprising the nucleic acid or vector of the disclosure, in admixture with a pharmaceutically acceptable carrier.

The disclosure provides a method of treating an inflammatory disease or condition which comprises administering the expression vector of the disclosure to a subject for a time and in an amount sufficient to reduce TNF activity, such as TNF-α activity.

In one embodiment, disease or condition is selected from the group consisting of rheumatoid arthritis, juvenile rheumatoid arthritis, psoriasis, psoriatic arthritis, ankylosing spondylitis, inflammatory bowel disease (Crohn's disease or ulcerative colitis), hepatitis associated with hepatitis A virus, hepatitis associated with hepatitis B virus, hepatitis associated with hepatitis C virus, hepatitis associated with ischemia/reperfusion, sepsis, alcoholic liver disease, and non-alcoholic steatosis.

In one embodiment, the disclosure provides for a method of treating an inflammatory disease or condition which comprises administering one or more splice switching oligomers (SSOs) to a subject for a time and in an amount to reduce the activity of TNF, wherein said one or more SSOs are capable of altering the splicing of a pre-mRNA encoding a mammalian tumor necrosis factor receptor 2 (TNFR2) (or TNFR1) to increase production of a protein capable of binding tumor necrosis factor (TNF), wherein said protein has a sequence comprising the amino acids encoded by a cDNA derived from a gene for said receptor, wherein the cDNA comprises in 5' to 3' contiguous order, the codon encoding the first amino acid after the cleavage point of the signal sequence of said gene through exon 6 of said gene and exon 8 of said gene through exon 10 of said gene; or the codon encoding the first amino acid of the open reading frame of said gene through exon 6 of said gene and exon 8 of said gene through exon 10 of said gene.

In one embodiment, said disease or condition is selected from the group consisting of rheumatoid arthritis, juvenile rheumatoid arthritis, psoriasis, psoriatic arthritis, ankylosing spondylitis, inflammatory bowel disease (Crohn's disease or ulcerative colitis), hepatitis associated with hepatitis A virus, hepatitis associated with hepatitis B virus, hepatitis associated with hepatitis C virus, hepatitis associated with ischemia/reperfusion, sepsis, alcoholic liver disease, and non-alcoholic steatosis. In one embodiment, the administration is parenteral, topical, oral, rectal, or pulmonary.

In one embodiment, the disclosure provides a splice switching oligomer (SSO) comprising at least 8 nucleotides, said SSO capable of altering the splicing of a pre-mRNA encoding a mammalian tumor necrosis factor receptor 2 (TNFR2) (or TNFR1) to produce a protein capable of binding tumor necrosis factor (TNF), wherein said protein has a sequence comprising the amino acids encoded by a cDNA derived from a gene for said receptor, wherein the cDNA comprises in 5' to 3' contiguous order, the codon encoding the first amino acid after the cleavage point of the signal sequence of said gene through exon 6 of said gene and exon 8 of said gene through exon 10 of said gene; or the codon encoding the first amino acid of the open reading frame of said gene through exon 6 of said gene and exon 8 of said gene through exon 10 of said gene.

In one embodiment, the disclosure provides for a SSO which comprises at least 8 nucleotides which are complementary to a contiguous sequence from SEQ ID No: 13.

In one embodiment the sequence of said SSO comprises a sequence selected from the group consisting of SEQ ID Nos: 14, 30, 46, 70, 71, 72, and 73, and subsequences thereof at least 8 nucleotides.

In one embodiment the sequence of said SSO comprises a sequence selected from the group consisting of SEQ ID Nos: 14-61.

The disclosure provides for a method of increasing the production of a protein capable of binding tumor necrosis factor (TNF), in a cell, which comprises administering one or more splice switching oligomers (SSOs) to said cell, wherein said protein has a sequence comprising the amino acids encoded by a cDNA derived from a mammalian tumor necrosis factor receptor 2 (TNFR2) (or TNFR1) gene, wherein the cDNA comprises in 5' to 3' contiguous order, the codon encoding the first amino acid after the cleavage point of the signal sequence of said gene through exon 6 of said gene and exon 8 of said gene through exon 10 of said gene; or the codon encoding the first amino acid of the open reading frame of said gene through exon 6 of said gene and exon 8 of said gene through exon 10 of said gene, and wherein said one or more SSOs are capable of altering the splicing of a pre-mRNA encoding said receptor to increase production of said protein. In one embodiment, the method is performed in vivo.

The disclosure provides for a pharmaceutical composition comprising the SSO of the disclosure and a pharmaceutically acceptable carrier.

### EXAMPLES

The following Examples are identicial to those described in PCT/US2007/10557.

### Example 1

*Oligonucleotides.* Table 6 lists chimeric locked nucleic acid (LNA) SSOs with alternating 2'deoxy- and 2'O-4'-(methylene)-bicyclic-ribonucleoside phosphorothioates and having sequences as described as above. These were synthesized by Santaris Pharma, Denmark. For each SSO, the 5'-terminal nucleoside was a 2'O-4'-methylene-ribonucleoside and the 3'-terminal nucleoside was a 2'deoxy-ribonucleoside. Table 7 shows the sequences of chimeric LNA SSOs with alternating 2'-O-methyl-ribonucleoside-phosphorothioates (2'-OMe) and 2'O-4'-(methylene)-bicyclic-ribonucleoside phosphorothioates. These were synthesized by Santaris Pharma, Denmark. The LNA is shown in capital letters and the 2'-OME is shown in lower case letters.

*Cell culture and transfections.* L929 cells were maintained in minimal essential media supplemented with 10% fetal bovine serum and antibiotic (37°C, 5% CO₂). For transfection, L929 cells were seeded in 24-well plates at 10⁵ cells per well and transfected 24 hrs later. Oligonucleotides were complexed, at the indicated concentrations, with 2 µL of Lipofectamine™ 2000 transfection reagent (Invitrogen) as per the manufacturer's directions. The nucleotide/lipid complexes were then applied to the cells and incubated for 24 hrs. The media was then aspirated and cells harvested with TRI-Reagent™ (MRC, Cincinnati, OH).

*RT-PCR.* Total RNA was isolated with TRI-Reagent (MRC, Cincinnati, OH) and TNFR1 or TNFR2 mRNA was amplified by GeneAmp® RT-PCR using r*Tth* polymerase (Applied Biosystems) following supplier directions. Approximately 200 ng of RNA was used per reaction. Primers used in the examples described herein are included in Table 2. Cycles of PCR proceeded: 95°C, 60 sec; 56°C, 30 sec; 72°C, 60 sec for 22-30 cycles total.

In some instances a Cy5-labeled dCTP (GE Healthcare) was included in the PCR step for visualization (0.1 µL per 50 µL PCR reaction). The PCR products were separated on a 10% non-denaturing polyacrylamide gel, and Cy5-labeled bands were visualized with a Typhoon™ 9400 Scanner (GE Healthcare). Scans were quantified with ImageQuant™ (GE Healthcare) software. Alternatively, in the absence of the inclusion of Cy5-labeled dCTP, the PCR products were separated on a 1.5% agarose gel containing trace amounts of ethidium bromide for visualization.

*PCR.* PCR was performed with Platinum® *Taq* DNA Polymerase (Invitrogen) according to the manufacturer's directions. For each 50 µL reaction, approximately 30 pmol of both forward and reverse primers were used. Primers used in the examples described herein are included in Table 5. Thermocycling reaction proceeded, unless otherwise stated, as follows: 94°C, 3 minutes; then 30-40 cycles of 94°C, 30 sec; 55°C, 30 sec; and 72°C, 105 sec; followed by 72°C, 3 minutes. The PCR products were analyzed on 1.5% agarose gels and visualized with ethidium bromide.

**Table 5: RT-PCR and PCR Primers**

| **SEQ ID.** | **Name** | **Sequence 5' to 3'** |
|---|---|---|
| | | **Human TNFR2** |
| 190 | TR001 | ACT GGG CTT CAT CCC AGC ATC |
| 191 | TR002 | CAC CAT GGC GCC CGT CGC CGT CTG G |
| 192 | TR003 | CGA CTT CGC TCT TCC AGT TGA GAA GCC CTT GTG CCT GCA G |
| 193 | TR004 | TTA ACT GGG CTT CAT CCC AGC ATC |
| 194 | TR005 | CTG CAG GCA CAA GGG CTT CTC AAC TGG AAG AGC GAA GTC G |
| 195 | TR026 | TTA ACT GGG CTT CAT CCC AGC |
| 196 | TR027 | CGA TAG AAT TCA TGG CGC CCG TCG CCG TCT GG |
| 197 | TR028 | CCT AAC TCG AGT TAA CTG GGC TTC ATC CCA GC |
| 198 | TR029 | GAC TGA GCG GCC GCC ACC ATG GCG CCC GTC GCC GTC TGG |
| 199 | TR030 | CTA AGC GCG GCC GCT TAA CTG GGC TTC ATC CCA GCA TC |
| 200 | TR047 | CGT TCT CCA ACA CGA CTT CA |
| 201 | TR048 | CTT ATC GGC AGG CAA GTG AGG |
| 202 | TR049 | ACT GAA ACA TCA GAC GTG GTG TGC |
| 203 | TR050 | CCT TAT CGG CAG GCA AGT GAG |

| | | **Human TNFR1** |
|---|---|---|
| 204 | TR006 | CCT CAT CTG AGA AGA CTG GGC G |
| 205 | TR007 | GCC ACC ATG GGC CTC TCC ACC GTG C |
| 206 | TR008 | GGG CAC TGA GGA CTC AGT TTG TGG GAA ATC GAC ACC TG |
| 207 | TR009 | CAG GTG TCG ATT TCC CAC AAA CTG AGT CCT CAG TGC CC |
| 208 | TR010 | CAC CAT GGG CCT CTC CAC CGT GC |
| 209 | TR011 | TCT GAG AAG ACT GGG CG |
| 210 | TR031 | CGA TAG GAT CCA TGG GCC TCT CCA CCG TGC |
| 211 | TR032 | CCT AAC TCG AGT CAT CTG AGA AGA CTG GGC G |
| 212 | TR033 | GAC TGA GCG GCC GCC ACC ATG GGC CTC TCC ACC GTG C |
| 213 | TR034 | CTA AGC GCG GCC GCT CAT CTG AGA AGA CTG GGC G |

| | | **Mouse TNFR2** |
|---|---|---|
| 214 | TR012 | GGT CAG GCC ACT TTG ACT GC |
| 215 | TR013 | CAC CGC TGC CCC TAT GGC G |
| 216 | TR014 | CAC CGC TGC CAC TAT GGC G |
| 217 | TR015 | GGT CAG GCC ACT TTG ACT GCA ATC |
| 218 | TR016 | GCC ACC ATG GCG CCC GCC GCC CTC TGG |
| 219 | TR017 | GGC ATC TCT CTT CCA ATT GAG AAG CCC TCC TGC CTA CAA AG |
| 220 | TR018 | CTT TGT AGG CAG GAG GGC TTC TCA ATT GGA AGA GAG ATG CC |
| 221 | TR019 | GGC CAC TTT GAC TGC AAT CTG |
| 222 | TR035 | CAC CAT GGC GCC CGC CGC CCT CTG G |
| 223 | TR036 | TCA GGC CAC TTT GAC TGC AAT C |
| 224 | TR037 | CGA TAG AAT TCA TGG CGC CCG CCG CCC TCT GG |
| 225 | TR038 | CCT AAC TCG AGT CAG GCC ACT TTG ACT GCA ATC |
| 226 | TR039 | GAC TGA GCG GCC GCC ACC ATG GCG CCC GCC GCC CTC TGG |
| 227 | TR040 | CTA AGC GCG GCC GCT CAG GCC ACT TTG ACT GCA ATC |
| 228 | TR045 | GAG CCC CAA ATG GAA ATG TGC |
| 229 | TR046 | GCT CAA GGC CTA CTG CAT CC |

| | | **Mouse TNFR1** |
|---|---|---|
| 230 | TR020 | GGT TAT CGC GGG AGG CGG GTC G |
| 231 | TR021 | GCC ACC ATG GGT CTC CCC ACC GTG CC |
| 232 | TR022 | CAC AAA CCC CCA GGA CTC AGT TTG TAG GGA TCC CGT GCC T |
| 233 | TR023 | AGG CAC GGG ATC CCT ACA AAC TGA GTC CTG GGG GTT TGT G |
| 234 | TR024 | CAC CAT GGG TCT CCC CAC CGT GCC |
| 235 | TR025 | TCG CGG GAG GCG GGT CGT GG |
| 236 | TR041 | CGA TAG TCG ACA TGG GTC TCC CCA CCG TGC C |
| 237 | TR042 | CCT AAG AAT TCT TAT CGC GGG AGG CGG GTC G |
| 238 | TR043 | GAC TGA GCG GCC GCC ACC ATG GGT CTC CCC ACC GTG CC |
| 239 | TR044 | CTA AGC GCG GCC GCT TAT CGC GGG AGG CGG GTC G |

*Human hepatocyte cultures.* Human hepatocytes were obtained in suspension either from ADMET technologies, or from The UNC Cellular Metabolism and Transport Core at UNC-Chapel Hill. Cells were washed and suspended in RPMI 1640 supplemented with 10% FBS, 1 mg/mL human insulin, and 13 nM DexamethASONe. Hepatocytes were plated in 6-well plates at 0.5 × 10⁶ cells per plate in 3 mL media. After 1-1.5 hrs, non-adherent cells were removed, and the media was replaced with RPMI 1640 without FBS, supplemented with 1 mg/mL human insulin, and 130 nM DexamethASONe.

For delivery of SSOs to hepatocytes in 6-well plates, 10 mL of a 5 mM SSO stock was diluted into 100 mL of OPTI-MEM™, and 4 mL of Lipofectamine™ 2000 was diluted into 100 mL of OPTI-MEM™. The 200 mL complex solution was then applied to the cells in the 6-well plate containing 2800 mL of media, for a total of 3000 mL. The final SSO concentration was 17 nM. After 24 hrs, cells were harvested in TRI-Reagent™. Total RNA was isolated per the manufacturer's directions. Approximately 200 ng of total RNA was subjected to reverse transcription-PCR (RT-PCR).

*ELISA.* To determine the levels of soluble TNFR2 in cell culture media or sera, the Quantikine® Mouse sTNF RII ELISA kit from R&D Systems (Minneapolis, MN) or Quantikine® Human sTNF RII ELISA kit from R&D Systems (Minneapolis, MN) were used. The antibodies used for detection also detect the protease cleavage forms of the receptor. ELISA plates were read using a microplate reader set at 450 nm, with wavelength correction set at 570 nm.

For mouse *in vivo* studies, blood from the animals was clotted for 1 hour at 37°C and centrifuged for 10 min at 14,000 rpm (Jouan BRA4i centrifuge) at 4°C. Sera was collected and assayed according to the manufacturer's guide, using 50 mL of mouse sera diluted 1:10.

*L929 cytotoxicity assay.* L929 cells plated in 96-well plates at 10⁴ cells per well were treated with 0.1 ng/mL TNF-α and 1 mg/mL actinomycin D in the presence of 10% serum from mice treated with the indicated oligonucleotide in 100 mL total of complete MEM media (containing 10% regular FBS) and allowed to grow for ∼24 hrs at 37°C. Control lanes were plated in 10% serum from untreated mice. Cell viability was measured 24 hrs later by adding 20 mL CellTiter 96® AQᵤₑₒᵤₛ One Solution Reagent (Promega) and measuring absorbance at 490 nm with a microplate reader. Cell viability was normalized to untreated cells.

*Western blots.* Twenty mL of media or 20 mg of lysate were loaded in each well of a 4-12% NuPAGE® polyacrylamide gel (Invitrogen). The gel was run 40 min at 200V. The protein was transferred, for 1 hr at 30V, to an Invitrolon™ PVDF membrane (Invitrogen), which was then blocked with StartingBlock® Blocking Buffer (Pierce) for 1 hr at room temperature. The membrane was incubated for 3 hrs at room temperature with a rabbit polyclonal antibody that recognizes the C-terminus of human and mouse TNFR2 (Abcam), Following three washes in PBS-T buffer (1×PBS, 0.1% Tween-20), the membrane was incubated for one hour at room temperature with secondary goat anti-rabbit antibody (Abcam) and again washed three times with PBS-T buffer. The protein was then detected with ECL Plus™ (GE Healthcare), according to the manufacturer's recommendations and then photographed.

### Example 2 - SSO Splice Switching Activity with TNFR mRNA

Table 6 shows the splice switching activities of SSOs having sequences as described in U.S. Appl. No. 11/595,485 and targeted to mouse and human TNFRs. Of SSOs targeted to mouse TNFR2 exon 7, at least 8 generated some muTNFR2 Δ7 mRNA. In particular, SSO 3312, 3274 and 3305 induced at least 50% skipping of exon 7; SSO 3305 treatment resulted in almost complete skipping. Of SSOs transfected into primary human hepatocytes, and targeted to human TNFR2 exon 7, at least 7 SSOs generated some huTNFR2 Δ7 mRNA. In particular, SSOs 3378, 3379, 3384 and 3459 induced at least 75% skipping of exon 7 (FIG. **2**B), and significant induction of huTNFR2 Δ7 into the extracellular media (FIG. **2**A).

**Table 6: SSO Splice Switching Activity**

| **SEQ ID.** | **Name** | **Activity** |
|---|---|---|
| **Mouse TNFR2** | | |
| | 3272 | - |
| | 3304 | - |
| | 3305 | + |
| | 3306 | + |
| | 3307 | + |
| | 3308 | + |
| | 3309 | + |
| | 3310 | - |
| | 3311 | + |
| 62 | 3274 | + |
| | 3312 | + |
| | 3273 | - |

| **Mouse TNFR1** | | |
|---|---|---|
| | 3333 | + |

| **Human TNFR2** | | |
|---|---|---|
| 14 | 3378 | + |
| 30 | 3379 | + |
| | 3380 | - |
| 70 | 3381 | + |
| 71 | 3382 | + |
| | 3383 | - |
| 46 | 3384 | + |
| 72 | 3459 | + |
| | 3460 | - |
| 73 | 3461 | + |

| **Control** | | |
|---|---|---|
| | 3083 | - |

Table 7 contains the sequences of 10 nucleotide chimeric SSOs with alternating 2'-O-methyl-ribonucleoside-phosphorothioates (2'-OMe) and 2'O-4'-(methylene)-bicyclic-ribonucleoside phosphorothioates. These SSOs are targeted to exon 7 of mouse TNFR2.

**Table 7: LNA/2'-OMe-ribonucleosidephosphorothioate chimeric mouse targeted SSO**

| **SEQ ID.** | **Name** | **Sequence 5' to 3'*** |
|---|---|---|
| 178 | 3274 | AgAgCaGaAcCtTaCt |
| 179 | 3837 | gAaCcTuAcT |
| 180 | 3838 | aGaGcAgAaC |
| 181 | 3839 | gAgCaGaAcC |
| 182 | 3840 | aGcAgAaCcT |
| 183 | 3841 | gCaGaAcCuT |
| 184 | 3842 | cAgAaCcTuA |
| 185 | 3843 | aGaAcCuTaC |

| | | |
|---|---|---|
| *Capital letters are 2'O-4'-(methylene)-bicyclic-ribonucleosides; lowercase letters are 2'-OMe | | |

To analyze the *in vitro* splice-switching activity of the SSOs listed in Table 7, L929 cells were cultured and seeded as described in Example 1. For delivery of each of the SSOs in Table 7 to the L929 cells, SSOs were diluted into 50 mL of OPTI-MEM™, and then 50 mL Lipofectamine™ 2000 mix (1 part Lipofectamine™ 2000 to 25 parts OPTI-MEM™) was added and incubated for 20 minutes. Then 400 mL of serum free media was added to the SSOs and applied to the cells in the 24-well plates. The final SSO concentration was either 50 or 100 nM. After 24 hrs, cells were harvested in 800 mL TRI-Reagent™. Total RNA was isolated per the manufacturer's directions and analyzed by RT-PCR (FIG. 3) using the forward primer TR045 (SEQ ID No: 228) and the reverse primer TR046 (SEQ ID No: 229).

To analyze the *in vivo* splice-switching activity of the SSOs listed in Table 7, mice were injected with the SSOs listed in Table 4 intraperitoneal (i.p.) at 25 mg/kg/day for 5 days. Mice were bled before injection and again 1, 5 and 10 days after the last injection. The concentration of soluble TNFR2 Δ7 in the sera taken before the first injection and 10 days after the last injection were measured by ELISA (FIG. 4B). The mice were sacrificed on day 10 and total RNA from 5-10 mg of the liver was analyzed by RT-PCR (FIG. 4A) using the forward primer TR045 (SEQ ID No: 228) and the reverse primer TR046 (SEQ ID No: 229).

Of the 10 nucleotide SSOs subsequences of SSO 3274 tested *in vitro,* all of them generated at least some muTNFR2 Δ7 mRNA (FIG. 3). In particular, SSO 3839, 3840 and 3841 displayed greater splice-switching activity than the longer 16 nucleotide SSO 3274 from which they are derived. The three 10 nucleotide SSOs, 3839, 3840, 3841, that demonstrated the greatest activity *in vitro* also were able to generate significant amounts of muTNFR2 Δ7 mRNA (FIG. 4A) and soluble muTNFR2 Δ7 protein (FIG. 4B) in mice *in vivo.*

To assess the effect of SSO length on splice switching activity in human TNFR2, cells were treated with SSOs of different lengths. Primary human hepatocytes were transfected with the indicated SSOs selected from Table 4. These SSOs were synthesized by Santaris Pharma, Denmark with alternating 2'deoxy- and 2'O-4'-(methylene)-bicyclic-ribonucleoside phosphorothioates. For each SSO, the 5'-terminal nucleoside was a 2'O-4'-methylene-ribonucleoside and the 3'-terminal nucleoside was a 2'deoxy-ribonucleoside.These SSOs were either 10-, 12-, 14- or 16-mers. The concentration of soluble TNFR2 Δ7 was measured by ELISA (FIG. 5, top panel). Total RNA was analyzed by RT-PCR for splice switching activity (FIG. 5, bottom panel).

### Example 3 - Analysis of the Splice Junction of SSO-induced TNFR2 Splice Variants

To confirm that the SSO splice switching, both in mice and in human cells, leads to the expected TNFR2 Δ7 mRNA, SSO-induced TNFR2 Δ7 mRNA was analyzed by RT-PCR and was sequenced.

*Mice.* Mice were injected with SSO 3274 intraperitoneal (i.p.) at 25 mg/kg/day for 10 days. The mice were then sacrificed and total RNA from the liver was analyzed by RT-PCR using the forward primer TR045 (SEQ ID No: 228) and the reverse primer TR046 (SEQ ID No: 229). The products were analyzed on a 1.5% agarose gel (FIG. 6A) and the product for the TNFR2 Δ7 was isolated using standard molecular biology techniques. The isolated TNFR2 Δ7 product was amplified by PCR using the same primers and then sequenced (FIG. 6B). The sequence data contained the sequence CTCTCTTCCAATTGAGAAGCCCTCCTGC (nucleotides 777-804 of SEQ ID No: 127), which confirms that the SSO-induced TNFR2 Δ7 mRNA lacks exon 7 and that exon 6 is joined directly to exon 8.

*Human hepatocytes.* Primary human hepatocytes were transfected with SSO 3379 as described in Example 1. Total RNA was isolated 48 hrs after transfection. The RNA was converted to cDNA with the Superscript™ II Reverse Transcriptase (Invitrogen) using random hexamer primers according to the manufacturer's directions. PCR was performed on the cDNA using the forward primer TR049 (SEQ ID No: 202) and the reverse primer TR050 (SEQ ID No: 203). The products were analyzed on a 1.5% agarose gel (FIG. **7**A). The band corresponding to TNFR2 Δ7 was isolated using standard molecular biology techniques and then sequenced (FIG. **7**B). The sequence data contained the sequence CGCTCTTCCAGTTGAGAAGCCCTTGTGC (nucleotides 774-801 of SEQ ID No: 125), which confirms that the SSO-induced TNFR2 Δ7 mRNA lacks exon 7 and that exon 6 is joined directly to exon 8.

### Example 4 - SSO Dose-Dependent Production of TNFR2 Δ7 Protein in Primary Human Hepatocytes

The dose response of splice-switching activity of SSOs in primary human hepatocytes was tested. Human hepatocytes were obtained in suspension from ADMET technologies. Cells were washed three times and suspended in seeding media (RPMI 1640 supplemented with L-Glut, with 10% FBS, penicillin, streptomycin, and 12 nM DexamethASONe). Hepatocytes were evaluated for viability and plated in 24-well, collagen-coated plates at 1.0 × 10⁵ cells per well. Typically, cell viability was 85-93%. After approximately 24 hrs, the media was replaced with maintenance media (seeding media without FBS).

For delivery of each of the SSOs to the hepatocytes, SSOs were diluted into 50 mL of OPTI-MEM™, and then 50 mL Lipofectamine™ 2000 mix (1 part Lipofectamine™ 2000 to 25 parts OPTI-MEM™) was added and incubated for 20 minutes. The SSOs were then applied to the cells in the 24-well plates. The final SSO concentration ranged from 1 to 150 nM. After 48 hrs, cells were harvested in 800 mL TRI-Reagent™.

Total RNA from the cells was analyzed by RT-PCR using the forward primer TR047 (SEQ ID No: 200) and the reverse primer TR048 (SEQ ID No: 201) (FIG. 8A). The concentration of soluble TNFR2 Δ7 in the serum was measured by ELISA (FIG. 8B). Both huTNFR2 Δ7 mRNA (FIG. 8A) and secreted huTNFR2 Δ7 protein (FIG. 8B) displayed dose dependent increases.

### Example 5 - Secretion of TNFR2 Splice Variants from Murine Cells

The ability of SSOs to induce soluble TNFR2 protein production and secretion into the extracellular media was tested. L929 cells were treated with SSOs as described in Example 1, and extracellular media samples were collected ∼48 hrs after transfection. The concentration of soluble TNFR2 in the samples was measured by ELISA (FIG. 9). SSOs that best induced shifts in RNA splicing, also secreted the most protein into the extracellular media. In particular, SSOs 3305, 3312, and 3274 increased soluble TNFR2 at least 3.5-fold over background. Consequently, induction of the splice variant mRNA correlated with production and secretion of the soluble TNFR2.

### Example 6 - In Vivo Injection of SSOs Generated muTNFR2 Δ7 mRNA in Mice

SSO 3305 in saline was injected intraperitoneal (i.p.) daily for 4 days into mice at doses from 3 mg/kg to 25 mg/kg. The mice were sacrificed on day 5 and total RNA from the liver was analyzed by RT-PCR. The data show splice switching efficacy similar to that found in cell culture. At the maximum dose of 25 mg/kg, SSO 3305 treatment induced almost full conversion to Δ7 mRNA (FIG. 10, bottom panel).

A similar experiment with SSO 3274 induced about 20% conversion to Δ7 mRNA. To optimize SSO 3274 induction of Δ7 mRNA, both the dose regimen and the time from the last injection to the sacrifice of the animal were varied. SSO 3274 was injected (i.p.) into mice daily for 4 days. SSO treatment induced about 30% conversion to Δ7 mRNA in mice analyzed on day 15, whereas a 20% shift was observed in mice analyzed on day five (FIG. 10, top panel). Furthermore, mice given Δ7injections for 10 days, and sacrificed on day 11 showed a 50% induction of mRNA (FIG. 10, top). These *in vivo* data suggest that TNFR2 SSOs can produce muTNFR2 Δ7 mRNA for at least 10 days after administration.

### Example 7 - Circulatory TNFR2 Δ7

Mice were injected with SSO 3274, 3305, or the control 3083 intraperitoneal (i.p.) at 25 mg/kg/day for 10 days. Mice were bled before injection and again 1, 5 and 10 days after the last injection. The concentration of soluble TNFR2 Δ7 in the serum was measured. SSO treatment induced soluble TNFR2 Δ7 protein levels over background for at least 10 days (FIG. 11).

To test the effects at longer time points, the experiment was repeated, except that serum samples were collected until day 27 after the last injection. The results show only a slight decrease in soluble TNFR2 Δ7 levels 27 days after the last SSO injection (FIG. **12**).

### Example 8 - Anti-TNF-α Activity in Mice Serum

The anti-TNF-a activity of serum from SSO 3274 treated mice was tested in an L929 cytotoxicity assay. In this assay, serum is assessed for its ability to protect cultured L929 cells from the cytotoxic effects of a fixed concentration of TNF-α as described in Example 1. Serum from mice treated with SSO 3274 but not control SSOs (3083 or 3272) increased viability of the L929 cells exposed to 0.1 ng/mL TNF-α (FIG. **13**). Hence, the SSO 3274 serum contained TNF-α antagonist sufficient to bind and to inactivate TNF-α, and thereby protect the cells from the cytotoxic effects of TNF-α. This anti-TNF-a activity was present in the serum of animals 5 and 27 days after the last injection of SSO 3274.

### Example 9 - Comparison of SSO Generated TNFR2 Δ7 to other anti-TNF-a antagonists

L929 cells were seeded as described above. Samples were prepared containing 90 µL of serum-free MEM, 0.1 ng/ml TNF-α and 1 µg/ml of actinomycin D, with either (i) recombinant soluble protein (0.01-3 mg/mL)) from Sigma® having the 236 amino acid residue extracellular domain of mouse TNFR2, (ii) serum from SSO 3274 or SSO 3305 treated mice (1.25-10%, diluted in serum from untreated mice; the concentration of TNFR2 Δ7 was determined by ELISA) or (iii) Enbrel® (0.45-150 pg/ml) to a final volume of 100 µl with a final mouse serum concentration of 10%. The samples were incubated at room temperature for 30 minutes. Subsequently, the samples were applied to the plated cells and incubated for ∼24 hrs at 37°C in a 5% CO₂ humidified atmosphere. Cell viability was measured by adding 20 µL CellTiter 96® AQᵤₑₒᵤₛ One Solution Reagent (Promega) and measuring absorbance at 490 nm with a microplate reader. Cell viability was normalized to untreated cells and plotted as a function of TNF antagonist concentration (FIG. 14).

### Example 10 - Stability of TNFR2 Δ7 mRNA and protein

Mice were treated with either SSO 3274 or 3272 (control) (n=5) by i.p. injection at a dose of 25 mg/kg/day daily for five days. Mice were bled before injection and again 5, 15, 22, 27, and 35 days after the last injection. The concentration of soluble TNFR2 Δ7 in the serum was measured (FIG. **15**A). Splice shifting of TNFR2 in the liver was also determined at the time of sacrifice by RT-PCR of total RNA from the liver (FIG. **15**B). Combined with data from Example 7 , a time course of TNFR2 mRNA levels after SSO treatment was constructed, and compared with the time course of TNFR2 Δ7 protein in serum (FIG. 16). The data show that TNFR2 Δ7 mRNA *in vivo* decays at a rate approximately 4 times faster than that of TNFR2 Δ7 protein in serum. On day 35, TNFR2 Δ7 mRNA was only detectable in trace amounts, whereas TNFR2 Δ7 protein had only decreased by 20% from its peak concentration.

### Example 11 - Generation of Human TNFR2 Δ7 cDNA (for comparison only)

A plasmid containing the full length human TNFR2 cDNA was obtained commercially from OriGene (Cat. No: TC119459, NM_001066.2). The cDNA was obtained by performing PCR on the plasmid using reverse primer TR001 (SEQ ID No: 116) and forward primer TR002 (SEQ ID No: 117). The PCR product was isolated and was purified using standard molecular biology techniques, and contains the 1383 bp TNFR2 open reading frame without a stop codon.

Alternatively, full length human TNFR2 cDNA is obtained by performing RT-PCR on total RNA from human mononuclear cells using the TR001 reverse primer and the TR002 forward primer. The PCR product is isolated and is purified using standard molecular biology techniques.

To generate human TNFR2 Δ7 cDNA, two separate PCR reactions were performed on the full length human TNFR2 cDNA, thereby creating overlapping segments of the TNFR2 Δ7 cDNA. In one reaction, PCR was performed on full length TNFR2 cDNA using the forward primer TR003 (SEQ ID No: 190) and the reverse primer TR004 (SEQ ID No: 191). In the other reaction, PCR was performed on full length TNFR2 cDNA using the reverse primer TR005 (SEQ ID No: 192) and the TR002 forward primer. Finally, the 2 overlapping segments were combined, and PCR was performed using the TR002 forward primer and the TR004 reverse primer. The PCR product was isolated and was purified using standard molecular biology techniques, and was expected to contain the 1308 bp TNFR2 Δ7 open reading frame with a stop codon (SEQ ID No: 125).

Similarly, by using the TR001 reverse primer instead of the TR004 reverse primer in these PCR reactions the 1305 bp human TNFR2 Δ7 open reading frame without a stop codon was generated. This allows for the addition of in-frame C-terminal affinity purification tags, such as His-tag, when the final PCR product is inserted into an appropriate vector.

### Example 12 - Generation of Human TNFR1 Δ7 cDNA (for comparison only)

A plasmid containing the full length human TNFR2 cDNA is obtained commercially from OriGene (Cat. No: TC127913, NM_001065.2). The cDNA is obtained by performing PCR on the plasmid using the TR006 reverse primer (SEQ ID No: 204) and the TR007 forward primer (SEQ ID No: 205). The full length human TNFR1 cDNA PCR product is isolated and is purified using standard molecular biology techniques.

Alternatively, full length human TNFR1 cDNA is obtained by performing RT-PCR on total RNA from human mononuclear cells using the TR006 reverse primer and the TR007 forward primer. The full length human TNFR1 cDNA PCR product is isolated and is purified using standard molecular biology techniques.

To generate human TNFR1 Δ7 cDNA, two separate PCR reactions are performed on the full length human TNFR1 cDNA, thereby creating overlapping segments of the TNFR1 Δ7 cDNA. In one reaction, PCR is performed on full length TNFR1 cDNA using the TR008 forward primer (SEQ ID No: 206) and the TR006 reverse primer. In the other reaction, PCR is performed on full length TNFR1 cDNA using the TR009 reverse primer (SEQ ID No: 207) and the TR010 forward primer (SEQ ID No: 208). Finally, the 2 overlapping segments are combined, and PCR is performed using the TR010 forward primer and the TR006 reverse primer. The PCR product is isolated and is purified using standard molecular biology techniques, and contains the 1254 bp human TNFR1 Δ7 open reading frame with a stop codon (SEQ ID No: 121).

Alternatively, by using the TR011 reverse primer (SEQ ID No: 209) instead of the TR006 reverse primer in these PCR reactions the 1251 bp human TNFR1 Δ7 open reading frame without a stop codon is generated. This allows for the addition of in-frame C-terminal affinity purification tags, such as His-tag, when the final PCR product is inserted into an appropriate vector.

### Example 13 - Generation of Murine TNFR2 Δ7 cDNA (for comparison only)

To generate full length murine TNFR2 cDNA, PCR was performed on the commercially available FirstChoice™ PCR-Ready Mouse Liver cDNA (Ambion, Cat. No: AM3300) using the TR012 reverse primer (SEQ ID No: 214) and the TR013 forward primer (SEQ ID No: 215). The full length murine TNFR2 cDNA PCR product is isolated and is purified using standard molecular biology techniques. Then by performing PCR on the resulting product using the TR014 forward primer (SEQ ID No: 216) and the TR012 reverse primer the proper Kozak sequence was introduced.

Alternatively, full length murine TNFR2 cDNA is obtained by performing RT-PCR on total RNA from mouse mononuclear cells or mouse hepatocytes using the TR015 reverse primer (SEQ ID No: 217) and the TR016 forward primer (SEQ ID No: 218). The full length murine TNFR2 cDNA PCR product is isolated and is purified using standard molecular biology techniques.

To generate murine TNFR2 Δ7 cDNA, two separate PCR reactions were performed on the full length murine TNFR2 cDNA, thereby creating overlapping segments of the TNFR2 Δ7 cDNA. In one reaction, PCR was performed on full length TNFR2 cDNA using the TR017 forward primer (SEQ ID No: 219) and the TR015 reverse primer. In the other reaction, PCR was performed on full length TNFR2 cDNA using the TR018 reverse primer (SEQ ID No: 220) and the TR016 forward primer. Finally, the 2 overlapping segments were combined, and PCR was performed using the TR016 forward primer and the TR015 reverse primer. The PCR product was isolated and was purified using standard molecular biology techniques, and was expected to contain the 1348 bp murine TNFR2 Δ7 open reading frame with a stop codon (SEQ ID No: 127).

Alternatively, by using the TR019 reverse primer (SEQ ID No: 221) instead of the TR015 reverse primer in these PCR reactions the 1345 bp murine TNFR2 Δ7 open reading frame without a stop codon was generated. This allows for the addition of in-frame C-terminal affinity purification tags, such as His-tag, when the final PCR product is inserted into an appropriate vector.

### Example 14 - Generation of Murine TNFR1 Δ7 cDNA (for comparison only)

To generate full length murine TNFR1 cDNA, PCR is performed on the commercially available FirstChoice™ PCR-Ready Mouse Liver cDNA (Ambion, Cat. No: AM3300) using the TR020 reverse primer (SEQ ID No: 230) and the TR021 forward primer (SEQ ID No: 231). The full length murine TNFR1 cDNA PCR product is isolated and is purified using standard molecular biology techniques.

Alternatively, full length murine TNFR1 cDNA is obtained by performing RT-PCR on total RNA from mouse mononuclear cells using the TR020 reverse primer and the TR021 forward primer. The full length murine TNFR1 cDNA PCR product is isolated and is purified using standard molecular biology techniques.

To generate murine TNFR1 Δ7 cDNA, two separate PCR reactions are performed on the full length human TNFR1 cDNA, thereby creating overlapping segments of the TNFR1 Δ7 cDNA. In one reaction, PCR is performed on full length TNFR1 cDNA using the TR022 forward primer (SEQ ID No: 232) and the TR020 reverse primer. In the other reaction, PCR is performed on full length TNFR1 cDNA using the TR023 reverse primer (SEQ ID No: 233) and the TR024 forward primer (SEQ ID No: 234). Finally, the 2 overlapping segments are combined, and PCR is performed using TR024 forward primer and the TR020 reverse primer. The 1259 bp PCR product is isolated and is purified using standard molecular biology techniques, and contains the 1251 bp murine TNFR1 Δ7 open reading frame with a stop codon (SEQ ID No: 123).

Alternatively, by using the TR025 reverse primer (SEQ ID No: 235) instead of the TR020 reverse primer in these PCR reactions the 1248 bp murine TNFR1 Δ7 open reading frame without a stop codon is generated. This allows for the addition of in-frame C-terminal affinity purification tags, such as His-tag, when the final PCR product is inserted into an appropriate vector.

### Example 15 - Construction of Vectors for the Expression of Human TNFR2 Δ7 in Mammalian Cells (for comparison only)

For expression of the human TNFR2 Δ7 protein in mammalian cells, a human TNFR2 Δ7 cDNA PCR product from Example 12 was incorporated into an appropriate mammalian expression vector. The TNFR2 Δ7 cDNA PCR product from Example 12 , both with and without a stop codon, and the pcDNA™3.1D/V5-His TOPO® expression vector (Invitrogen) were blunt-end ligated and isolated according to the manufacturer's directions. Plasmids containing inserts encoding human TNFR2 Δ7 were transformed into OneShot® Top10 competent cells (Invitrogen), according to the supplier's directions. Fifty mL of the transformation mix were plated on LB media with 100 mg/mL of ampicillin and incubated overnight at 37°C. Single colonies were used to inoculate 5 mL cultures of LB media with 100 mg/mL ampicillin and incubated overnight at 37°C. The cultures were then used to inoculate 200 mL of LB media with 100 mg/mL of ampicillin and grown overnight at 37°C. The plasmids were isolated using GenElute™ Plasmid Maxiprep kit (Sigma) according to manufacturer's directions. Purification efficiency ranged from 0.5 to 1.5 mg of plasmid per preparation.

Three human TNFR2 Δ7 clones (1319-1, 1138-5 and 1230-1) were generated and sequenced. Clone 1319-1 contains the human TNFR2 Δ7 open reading frame without a stop codon followed directly by an in-frame His-tag from the plasmid; while clones 1138-5 and 1230-1 contain the TNFR2 Δ7 open reading frame followed immediately by a stop codon. The sequence of the His-tag from the plasmid is given in SEQ ID No: 242. The sequences of the TNFR2 Δ7 open reading frames of clones 1230-1 and 1319-1 were identical to SEQ ID No: 125 with and without the stop codon, respectively. However relative to SEQ ID No: 125, the sequence (SEQ ID No: 231) of the TNFR2 Δ7 open reading frames of clone 1138-5 differed by a single nucleotide at position 1055 in exon 10, with an A in the former and a G in the later. This single nucleotide change causes the amino acid 352 to change from a glutamine to an arginine.

### Example 16 - Expression of Human TNFR2 Δ7 in E. coli (for comparison only)

For expression of the human TNFR2 Δ7 protein in bacteria, a human TNFR2 Δ7 cDNA from Example 12 is incorporated into an appropriate expression vector, such as a pET Directional TOPO® expression vector (Invitrogen). PCR is performed on the PCR fragment from Example 12 using forward (TR002) (SEQ ID No: 191) and reverse (TR026) (SEQ ID No: 195) primers to incorporate a homologous recombination site for the vector. The resulting PCR fragment is incubated with the pET101/D-TOPO® vector (Invitrogen) according to the manufacturer's directions, to create the human TNFR2 Δ7 bacterial expression vector. The resulting vector is transformed into the *E. coli* strain BL21(DE3). The human TNFR2 Δ7 is then expressed from the bacterial cells according to the manufacturer's instructions.

### Example 17 - Expression of Human TNFR2 Δ7 in insect cells (for comparison only)

For expression of the human TNFR2 Δ7 protein in insect cells, a human TNFR2 Δ7 cDNA from Example 12 is incorporated into a baculoviral vector. PCR is performed on a human TNFR2 Δ7 cDNA from Example 12 using forward (TR027) (SEQ ID No: 196) and reverse (TR028) (SEQ ID No: 197) primers. The resulting PCR product is digested with the restriction enzymes EcoRI and XhoI. The digested PCR product is ligated with a EcoRI and XhoI digested pENTR™ Vector (Invitrogen), such as any one of the pENTR™1A, pENTR™2B, pENTR™3C, pENTR™4, or pENTR™ 11 Vectors, to yield an entry vector. The product is then isolated, amplified, and purified using standard molecular biology techniques.

A baculoviral vector containing the human TNFR2 Δ7 cDNA is generated by homologous recombination of the entry vector with BaculoDirect™ Linear DNA (Invitrogen) using LR Clonase™ (Invitrogen) according to the manufacturer's directions. The reaction mixture is then used to infect Sf9 cells to generate recombinant baculovirus. After harvesting the recombinant baculovirus, expression of human TNFR2 Δ7 is confirmed. Amplification of the recombinant baculovirus yields a high-titer viral stock. The high-titer viral stock is used to infect Sf9 cells, thereby expressing human TNFR2 Δ7 protein.

### Example 18 - Generation of Adeno-Associated viral vectors for the expression of Human TNFR2 Δ7 (for comparison only)

For *in vitro* or *in vivo* delivery to mammalian cells of the human TNFR2 Δ7 gene for expression in those mammalian cells, a recombinant adeno-associated virus (rAAV) vector is generated using a three plasmid transfection system as described in Grieger, J., et al., 2006, Nature Protocols 1:1412. PCR is performed on a purified human TNFR2 D7 PCR product of Example 12 using forward (TR029) (SEQ ID No: 198) and reverse (TR030) (SEQ ID No: 199) primers to introduce unique flanking NotI restriction sites. The resulting PCR product is digested with the NotI restriction enzyme, and isolated by standard molecular biology techniques. The NotI-digested fragment is then ligated to NotI-digested pTR-UF2 (University of North Carolina (UNC) Vector Core Facility), to create a plasmid that contains the human TNFR2 D7 open reading frame, operably linked to the CMVie promoter, flanked by inverted terminal repeats. The resulting plasmid is then transfected with the plasmids pXX680 and pHelper (UNC Vector Core Facility) into HEK-293 cells, as described in Grieger, J., et al., to produce rAAV particles containing the human TNFR2 Δ7 gene where expression is driven by the strong constitutive CMVie promoter. The virus particles are harvested and purified, as described in Grieger, J., et al., to provide an rAAV stock suitable for transducing mammalian cells.

### Example 19 - Expression of Human TNFR1 Δ7 in E. coli (for comparison only)

For expression of the human TNFR1 Δ7 protein in bacteria, the cDNA is incorporated into an appropriate expression vector, such as a pET Directional TOPO® expression vector (Invitrogen). PCR is performed on the cDNA using forward (TR010) (SEQ ID No: 208) and reverse (TR006) (SEQ ID No: 204) primers to incorporate a homologous recombination site for the vector. The resulting PCR fragment is incubated with the pET101/D-TOPO® vector (Invitrogen) according to the manufacturer's directions, to create the human TNFR1 Δ7 bacterial expression vector. The resulting vector is transformed into the *E. coli* strain BL21(DE3). The human TNFR1 Δ7 is then expressed from the bacterial cells according to the manufacturer's instructions.

### Example 20 - Expression of Human TNFR1 Δ7 in mammalian cells (for comparison only)

For expression of the human TNFR1 Δ7 protein in mammalian cells, a human TNFR1 Δ7 cDNA PCR product is incorporated into an appropriate mammalian expression vector. human TNFR1 Δ7 cDNA PCR product and the pcDNA™3.1D/V5-His TOPO® expression vector (Invitrogen) are blunt-end ligated according to the manufacturer's directions. The product is then isolated, amplified, and purified using standard molecular biology techniques to yield the mammalian expression vector. The vector is then transfected into a mammalian cell, where expression of the human TNFR1 Δ7 protein is driven by the strong constitutive CMVie promoter.

### Example 21 - Expression of Human TNFR1 Δ7 in insect cells (for comparison only)

For expression of the human TNFR1 Δ7 protein in insect cells, the cDNA from Example 12 is incorporated into a baculoviral vector. PCR is performed on the cDNA from Example 12 using forward (TR031) (SEQ ID No: 210) and reverse (TR032) (SEQ ID No: 211) primers. The resulting PCR product is digested with the restriction enzymes EcoRI and XhoI. The digested PCR product is ligated with a EcoRI and XhoI digested pENTR™ Vector (Invitrogen), such as any one of the pENTR™1A, pENTR™2B, pENTR™3C, pENTR™4, or pENTR™11 Vectors, to yield an entry vector. The product is then isolated, amplified, and purified using standard molecular biology techniques.

A baculoviral vector containing the human TNFR1 Δ7 cDNA is generated by homologous recombination of the entry vector with BaculoDirect™ Linear DNA (Invitrogen) using LR Clonase™ (Invitrogen) according to the manufacturer's directions. The reaction mixture is then used to infect Sf9 cells to generate recombinant baculovirus. After harvesting the recombinant baculovirus, expression of human TNFR1 Δ7 is confirmed. Amplification of the recombinant baculovirus yields a high-titer viral stock. The high-titer viral stock is used to infect Sf9 cells, thereby expressing human TNFR1 Δ7 protein.

### Example 22 - Generation of Adeno-Associated viral vectors for the expression of Human TNFR1 Δ7 (for comparison only)

For *in vitro* or *in vivo* delivery to mammalian cells of the human TNFR1 Δ7 gene for expression in those mammalian cells, a recombinant adeno-associated virus (rAAV) vector is generated using a three plasmid transfection system as described in Grieger, J., et al., 2006, Nature Protocols 1:1412. PCR is performed on the purified human TNFR1 D7 PCR product using forward (TR033) (SEQ ID No: 212) and reverse (TR034) (SEQ ID No: 213) primers to introduce unique flanking NotI restriction sites. The resulting PCR product is digested with the NotI restriction enzyme, and isolated by standard molecular biology techniques. The NotI-digested fragment is then ligated to NotI-digested pTR-UF2 (University of North Carolina (UNC) Vector Core Facility), to create a plasmid that contains the human TNFR1 D7 open reading frame, operably linked to the CMVie promoter, flanked by inverted terminal repeats. The resulting plasmid is then transfected with the plasmids pXX680 and pHelper (UNC Vector Core Facility) into HEK-293 cells, as described in Grieger, J., et al., to produce rAAV particles containing the human TNFR1 Δ7 gene where expression is driven by the strong constitutive CMVie promoter. The virus particles are harvested and purified, as described in Grieger, J., et al., to provide an rAAV stock suitable for transducing mammalian cells.

### Example 23 - Construction of Vectors for the Expression of Mouse TNFR2 Δ7 in mammalian cells (for comparison only)

For expression of the murine TNFR2 Δ7 protein in mammalian cells, a murine TNFR2 Δ7 cDNA PCR product from Example 14 was incorporated into an appropriate mammalian expression vector. The TNFR2 Δ7 cDNA PCR product from Example 14 both with and without a stop codon, and the pcDNA™3.1D/V5-His TOPO® expression vector (Invitrogen) was blunt-end ligated and isolated according to the manufacturer's directions. Plasmids containing inserts encoding murine Δ7 TNFR2 were transformed into OneShot® Top10 competent cells (Invitrogen), according to the supplier's directions. Fifty mL of the transformation mix were plated on LB media with 100 mg/mL of ampicillin and incubated overnight at 37°C. Single colonies were used to inoculate 5 mL cultures of LB media with 100 mg/mL ampicillin and incubated overnight at 37°C. The cultures were then used to inoculate 200 mL of LB media with 100 mg/mL of ampicillin and grown overnight at 37°C. The plasmids were isolated using GenElute™ Plasmid Maxiprep kit (Sigma) according to manufacturer's directions. Purification efficiency ranged from 0.5 to 1.5 mg of plasmid per preparation.

Two murine TNFR2 Δ7 clones (1144-4 and 1145-3) were generated and sequenced. Clone 1144-4 contains the murine TNFR2 Δ7 open reading frame without a stop codon followed directly by an in-frame His-tag from the plasmid; while clone 1145-3 contains the TNFR2 Δ7 open reading frame followed immediately by a stop codon. The sequence of the His-tag from the plasmid is given in SEQ ID No: 242. Relative to SEQ ID No: 127, the sequence (SEQ ID No: 240) of the TNFR2 Δ7 open reading frames of the two clones, 1144-4 and 1145-3, differed by a single nucleotide at eleven positions. As a result of these single nucleotide changes there are four amino acid differences relative to SEQ ID No: 128.

### Example 24 - Expression of Murine TNFR2 Δ7 in E. coli (for comparison only)

For expression of the mouse TNFR2 Δ7 protein in bacteria, a murine TNFR2 Δ7 cDNA from Example 14 is incorporated into an appropriate expression vector, such as a pET Directional TOPO® expression vector (Invitrogen). PCR is performed on the PCR fragment from Example 14 using forward (TR035) (SEQ ID No: 222) and reverse (TR036) (SEQ ID No: 223) primers to incorporate a homologous recombination site for the vector. The resulting PCR fragment is incubated with the pET101/D-TOPO® vector (Invitrogen) according to the manufacturer's directions, to create the murine TNFR2 Δ7 bacterial expression vector. The resulting vector is transformed into the *E. coli* strain BL21(DE3). The murine TNFR2 Δ7 is then expressed from the bacterial cells according to the manufacturer's instructions.

### Example 25 - Expression of Mouse TNFR2 Δ7 in insect cells (for comparison only)

For expression of the murine TNFR2 Δ7 protein in insect cells, the cDNA from Example 14 is incorporated into a baculoviral vector. PCR is performed on the cDNA from Example 14 using forward (TR037) (SEQ ID No: 224) and reverse (TR038) (SEQ ID No: 225) primers. The resulting PCR product is digested with the restriction enzymes EcoRI and XhoI. The digested PCR product is ligated with a EcoRI and XhoI digested pENTR™ Vector (Invitrogen), such as any one of the pENTR™1A, pENTR™2B, pENTR™3C, pENTR™4, or pENTR™11 Vectors, to yield an entry vector. The product is then isolated, amplified, and purified using standard molecular biology techniques.

A baculoviral vector containing the murine TNFR2 Δ7 cDNA is generated by homologous recombination of the entry vector with BaculoDirect™ Linear DNA (Invitrogen) using LR Clonase™ (Invitrogen) according to the manufacturer's directions. The reaction mixture is then used to infect Sf9 cells to generate recombinant baculovirus. After harvesting the recombinant baculovirus, expression of murine TNFR2 Δ7 is confirmed. Amplification of the recombinant baculovirus yields a high-titer viral stock. The high-titer viral stock is used to infect Sf9 cells, thereby expressing murine TNFR2 Δ7 protein.

### Example 26 - Generation of Adeno-Associated viral vectors for the expression of Murine TNFR2 Δ7 (for comparison only)

For *in vitro* or *in vivo* delivery to mammalian cells of the murine TNFR2 Δ7 gene for expression in those mammalian cells, a recombinant adeno-associated virus (rAAV) vector is generated using a three plasmid transfection system as described in Grieger, J., et al., 2006, Nature Protocols 1:1412. PCR is performed on the purified murine TNFR2 D7 PCR product of Example 14 using forward (TR039)(SEQ ID No: 226) and reverse (TR040)(SEQ ID No: 227) primers to introduce unique flanking NotI restriction sites. The resulting PCR product is digested with the NotI restriction enzyme, and isolated by standard molecular biology techniques. The NotI-digested fragment is then ligated to NotI-digested pTR-UF2 (University of North Carolina (UNC) Vector Core Facility), to create a plasmid that contains the murine TNFR2 D7 open reading frame, operably linked to the CMVie promoter, flanked by inverted terminal repeats. The resulting plasmid is then transfected with the plasmids pXX680 and pHelper (UNC Vector Core Facility) into HEK-293 cells, as described in Grieger, J., et al., to produce rAAV particles containing the murine TNFR2 Δ7 gene where expression is driven by the strong constitutive CMVie promoter. The virus particles are harvested and purified, as described in Grieger, J., et al., to provide a rAAV stock suitable for transducing mammalian cells.

### Example 27 - Expression of Murine TNFR1 Δ7 in E. coli (for comparison only)

For expression of the mouse TNFR1 Δ7 protein in bacteria, the cDNA from Example 15 is incorporated into an appropriate expression vector, such as a pET Directional TOPO® expression vector (Invitrogen). PCR is performed on the cDNA from Example 15 using forward (TR024)(SEQ ID No: 234) and reverse (TR020)(SEQ ID No: 235) primers to incorporate a homologous recombination site for the vector. The resulting PCR fragment is incubated with the pET101/D-TOPO® vector (Invitrogen) according to the manufacturer's directions, to create the murine TNFR1 Δ7 bacterial expression vector. The resulting vector is transformed into the *E. coli* strain BL21(DE3). The murine TNFR1 Δ7 is then expressed from the bacterial cells according to the manufacturer's instructions.

### Example 28 - Expression of Mouse TNFR1 Δ7 in mammalian cells (for comparison only)

For expression of the murine TNFR1 Δ7 protein in mammalian cells, a murine TNFR1 Δ7 cDNA PCR product from Example 15 is incorporated into an appropriate mammalian expression vector. The murine TNFR1 Δ7 cDNA PCR product from Example 15 and the pcDNA™3.1D/V5-His TOPO® expression vector (Invitrogen) are blunt-end ligated according to the manufacturer's directions. The product is then isolated, amplified, and purified using standard molecular biology techniques to yield the mammalian expression vector. The vector is then transfected into a mammalian cell, where expression of the murine TNFR1 Δ7 protein is driven by the strong constitutive CMVie promoter.

### Example 29 - Expression of Mouse TNFR1 Δ7 in insect cells (for comparison only)

For expression of the murine TNFR1 Δ7 protein in insect cells, the cDNA from Example 15 is incorporated into a baculoviral vector. PCR is performed on the cDNA from Example 15 using forward (TR041)(SEQ ID No: 236) and reverse (TR042) (SEQ ID No: 237) primers. The resulting PCR product is digested with the restriction enzymes EcoRI and XhoI. The digested PCR product is ligated with a EcoRI and XhoI digested pENTR™ Vector (Invitrogen), such as any one of the pENTR™1A, pENTR™2B, pENTR™3C, pENTR™4, or pENTR™11 Vectors, to yield an entry vector. The product is then isolated, amplified, and purified using standard molecular biology techniques.

A baculoviral vector containing the murine TNFR1 Δ7 cDNA is generated by homologous recombination of the entry vector with BaculoDirect™ Linear DNA (Invitrogen) using LR Clonase™ (Invitrogen) according to the manufacturer's directions. The reaction mixture is then used to infect Sf9 cells to generate recombinant baculovirus. After harvesting the recombinant baculovirus, expression of murine TNFR1 Δ7 is confirmed. Amplification of the recombinant baculovirus yields a high-titer viral stock. The high-titer viral stock is used to infect Sf9 cells, thereby expressing murine TNFR1 Δ7 protein.

### Example 30 - Generation of Adeno-Associated viral vectors for the expression of Murine TNFR1 Δ7 (for comparison only)

For *in vitro* or *in vivo* delivery to mammalian cells of the murine TNFR1 Δ7 gene for expression in those mammalian cells, a recombinant adeno-associated virus (rAAV) vector is generated using a three plasmid transfection system as described in Grieger, J., et al., 2006, Nature Protocols 1:1412. PCR is performed on the purified murine TNFR1 D7 PCR product of Example 14 , using forward (TR043)(SEQ ID No: 238) and reverse (TR044)(SEQ ID No: 239) primers to introduce unique flanking NotI restriction sites. The resulting PCR product is digested with the NotI restriction enzyme, and isolated by standard molecular biology techniques. The NotI-digested fragment is then ligated to NotI-digested pTR-UF2 (University of North Carolina (UNC) Vector Core Facility), to create a plasmid that contains the murine TNFR1 D7 open reading frame, operably linked to the CMVie promoter, flanked by inverted terminal repeats. The resulting plasmid is then transfected with the plasmids pXX680 and pHelper (UNC Vector Core Facility) into HEK-293 cells, as described in Grieger, J., et al., to produce rAAV particles containing the murine TNFR1 Δ7 gene where expression is driven by the strong constitutive CMVie promoter. The virus particles are harvested and purified, as described in Grieger, J., et al., to provide an rAAV stock suitable for transducing mammalian cells.

### Example 31 - Generation of Lentiviral vectors for the expression of TNFR Δ7 (for comparison only)

For *in vitro* or *in vivo* delivery to mammalian cells of a TNFR Δ7 gene for expression in those mammalian cells, a replication-incompetent lentivirus vector is generated. A PCR product from Examples 27, 30, 35 and 38 and the pLenti6/V5-D-TOP0® vector (Invitrogen) are blunt-end ligated according to the manufacturer's directions. The resulting plasmid is transformed into *E. coli,* amplified, and purified using standard molecular biology techniques. This plasmid is transfected into 293FT cells (Invitrogen) according to the manufacturer's directions to produce lentivirus particles containing the TNFR Δ7 gene where expression is driven by the strong constitutive CMVie promoter. The virus particles are harvested and purified, as described in Tiscornia, G., et al., 2006, Nature Protocols 1:241, to provide a lentiviral stock suitable for transducing mammalian cells.

### Example 32 - Expression of TNFR2 Δ7 in Mammalian Cells (for comparison only)

The plasmids generated in Examples 26 and 34 were used to express active protein in mammalian HeLa cells, and the resulting proteins were tested for anti-TNF-α activity. HeLa cells were seeded in at 1.0 × 10⁵ cells per well in 24-well plates in SMEM media containing L-glutamine, gentamicin, kanamycin, 5% FBS and 5% HS. Cells were grown overnight at 37°C in a 5% CO₂ humidified atmosphere. Approximately 250 ng of plasmid DNA was added to 50 mL of OPTI-MEM™, and then 50 mL Lipofectamine™ 2000 mix (1 part Lipofectamine™ 2000 to 25 parts OPTI-MEM™) was added and incubated for 20 minutes. Then 400 mL of serum free media was added and then applied to the cells in the 24-well plates. After incubation for ∼48 hrs at 37°C in a 5% CO₂ humidified atmosphere, the media was collected and the cells were harvested in 800 mL TRI-Reagent™. Total RNA was isolated from the cells per the manufacturer's directions and analyzed by RT-PCR using the forward primer TR047 (SEQ ID No: 200) and the reverse primer TR048 (SEQ ID No: 201) for human TNFR2 Δ7, or the forward primer TR045 (SEQ ID No: 228) and the reverse primer TR046 (SEQ ID No: 229) for mouse TNFR2 Δ7. The concentration of soluble TNFR2 in the media was measured by ELISA.

The anti-TNF-α activity of the above media was tested in an L929 cytotoxicity assay. L929 cells were plated in 96-well plates at 2 × 10⁴ cells per well in MEM media containing 10% regular FBS, penicillin and streptomycin and grown overnight at 37°C in a 5% CO₂ humidified atmosphere. The media samples were diluted 1, 2, 4, 8 and 16 fold with media from non-transfected HeLa cells. Ninety µL of each of these samples was added to 10 µL of serum-free media, containing 1.0 ng/ml TNF-α and 1 µg/ml of actinomycin D. The media from the cells were removed and replaced with these 100 µL samples. The cells were then grown overnight at 37°C in a 5% CO₂ humidified atmosphere. Twenty mL CellTiter 96® AQᵤₑₒᵤₛ One Solution Reagent (Promega) was then added to each well. Cell viability was measured 4 hrs later by measuring absorbance at 490 nm with a microplate reader. Cell viability was normalized to untreated cells nd plotted as a function of TNF antagonist concentration (FIG. **17**).

The data from this example and from Example 9 were analyzed using the GraphPad Prism® software to determine the EC₅₀ value for each antagonist. For each antagonist from these examples a sigmoidal dose-response curve was fit by non-linear regression with the maximum and minimum responses held fixed to 100% and 0%, respectively. The EC₅₀ values shown in Table 8 correspond to a 95% confidence level, and each curve had an r² value ranging from 0.7 to 0.9.

**Table 8: Activity of TNF-α antagonists**

| **TNF-α Antagonist** | **EC₅₀ (ng/mL)** |
|---|---|
| Etanercept | 1.1 ± 0.5 |
| Recombinant soluble TNFR2 (rsTNFR2) | 698 ± 180 |
| SSO 3305 treated mice serum (mouse TNFR2 Δ7) | 0.6 ± 0.2 |
| SSO 3274 treated mice serum (mouse TNFR2 Δ7) | 0.8 ± 0.3 |
| Extracellular media from 1144-4 transfected HeLa cells (mouse TNFR2 Δ7) | 2.4 ± 1.4 |
| Extracellular media from 1145-3 transfected HeLa cells (mouse TNFR2 Δ7) | 2.4 ± 0.8 |
| Extracellular media from 1230-1 transfected HeLa cells (human TNFR2 Δ7) | 1.4 ± 1.1 |
| Extracellular media from 1319-1 transfected HeLa cells (human TNFR2 Δ7) | 1.7 ± 1.0 |
| Extracellular media from 1138-5 transfected HeLa cells (human TNFR2 Δ7) | 1.8 ± 1.1 |

### Example 33 - Expression and Purification of TNFR2 Δ7 in Mammalian Cells (for comparison only)

The plasmids generated in Example 15 and Example were used to express and purify TNFR2 Δ7 from mammalian HeLa cells. HeLa cells were plated in 6-well plates at 5 × 10⁵ cells per well, and grown overnight at 37°C, 5% CO₂, in humidified atmosphere. Each well was then transfected with 1.5 mg of plasmid DNA using either 1144-4 (mouse TNFR2 Δ7 with His-tag), 1145-1 (mouse TNFR2 Δ7, no His-tag), 1230-1 (human TNFR2 Δ7, no His-tag) or 1319-1 (human TNFR2 Δ7 with His-tag) plasmids. Media was collected ∼48 hrs after transfection and concentrated approximately 40-fold using Amicon MWCO 30,000 filters. The cells were lysed in 120 mL of RIPA lysis buffer (Invitrogen) with protease inhibitors (Sigma-aldrich) for 5 minutes on ice. Protein concentration was determined by the Bradford assay. Proteins were isolated from aliquots of the cell lysates and the extracellular media and analyzed by western blot for TNFR2 as described in Example 1 (FIG. **18**).

Human and mouse TNFR2 D7 with a His-tag (clones 1319-1 and 1144-4, respectively) were purified from the above media by affinity chromatography. HisPur™ cobalt spin columns (Pierce) were used to purify mouse and human TNFR2 Δ7 containing a His-tag from the above media. Approximately 32 mL of media were applied to a 1 mL HisPur™ column equilibrated with 50 mM sodium phosphate, 300 mM sodium chloride, 10 mM imidazole buffer (pH 7.4) as recommended by the manufacturer. The column was then washed with two column volumes of the same buffer and protein was eluted with 1 mL of 50 mM sodium phosphate, 300 mM sodium chloride, 150 mM imidazole buffer (pH 7.4). Five mL of each eluate were analyzed by Western blot as described above (FIG. 19). TNFR2 Δ7 appears in the eluate and the multiple bands represent variably glycosylated forms of TNFR2 D7. As negative controls, the TNFR2 D7 proteins expressed from plasmids 1230-1 or 1145-1 which do not contain a His-tag where subjected to the above purification procedure. These proteins do not bind the affinity column and do not appear in the eluate (FIG. 19).

## Claims

1. An oligomer of 12 - 16 nucleobases in length, comprising of a contiguous nucleobase sequence which consists of SEQ ID No 246: Aₛ^{o}cₛAₛ^{o}aₛTₛ^{o}cₛAₛ^{o}gₛTₛ^{o}cₛ^{m}Cₛ^{o}t or an oligomer 16 nucleobases in length, comprising of a contiguous nucleobase sequence which consists of SEQ ID No 244: ^{m}Cₛ^{o}cₛAₛ^{o}cₛAₛ^{o}aₛTₛ^{o}cₛAₛ^{o}gₛTₛ^{o}cₛ^{m}Cₛ^{o}tₛAₛ^{o}g
wherein capital letters represent LNA; superscript "o" represents oxy LNA; subscript "s" represents phosphorothiate linkage; lower case letters represent DNA; ^{m}C represents 5-methylcytosine;
and wherein the LNA is beta-D-oxy LNA.

2. A conjugate comprising the oligomer according to claim 1 and at least one non-nucleotide moiety covalently attached to said oligomer.

3. A pharmaceutical composition comprising the oligomer according to claim 1, or the conjugate according to claim 2, and a pharmaceutically acceptable carrier.

4. An oligomer according to claim 1 or a conjugate according to claim 2, for the treatment of an inflammatory disease or condition.

5. An *in vitro* method of increasing the expression of a soluble form of TNFRSF1B TNFalpha receptor in a mammalian cell which expresses said TNFalpha receptor, said method comprising administering the oligomer according to claim 1 or the conjugate according to claim 2 or the pharmaceutical composition according to claim 3.

6. Use of an oligomer according to claim 1 or a conjugate according to claim 2, in the manufacture of a medicament for the treatment of an inflammatory disease or condition.

## Patentansprüche

1. Oligomer mit einer Länge von 12 - 16 Nukleobasen, umfassend eine zusammenhängende Nukleobasensequenz, die aus SEQ ID Nr. 246:
Aₛ^{o}cₛAₛ^{o}aₛTₛ^{o}cₛAₛ^{o}gₛTₛ^{o}Cₛ^{m}Cₛ^{o}t besteht, oder Oligomer mit einer Länge von 16 Nukleobasen, umfassend eine zusammenhängende Nukleobasensequenz, die aus SEQ ID Nr. 244: ^{m}Cₛ^{o}cₛAₛ^{o}cₛAₛ^{o}aₛTₛ^{o}cₛAₛ^{o}gₛTₛ^{o}cₛ^{m}Cₛ^{o}tₛAₛ^{o}g besteht,
wobei Großbuchstaben LNA bedeuten; ein hochgestelltes "o" Oxy-LNA bedeutet; ein tiefgestelltes "s" eine Phosphorothioatverknüpfung bedeutet; Kleinbuchstaben DNA bedeuten; ^{m}C 5-Methylcytosin bedeutet;
und wobei es sich bei der LNA um Beta-D-oxy-LNA handelt.

2. Konjugat, umfassend das Oligomer nach Anspruch 1 und wenigstens eine an das Oligomer kovalent gebundene Nicht-Nukleotid-Gruppierung.

3. Pharmazeutische Zusammensetzung, umfassend das Oligomer nach Anspruch 1 oder das Konjugat nach Anspruch 2 und einen pharmazeutisch unbedenklichen Träger.

4. Oligomer nach Anspruch 1 oder Konjugat nach Anspruch 2, zur Behandlung einer Entzündungskrankheit bzw. eines Entzündungsleidens.

5. In-vitro-Verfahren zur Erhöhung der Expression einer löslichen Form von TNFRSF1B-TNFalpha-Rezeptor in einer Säugerzelle, die den TNFalpha-Rezeptor exprimiert, wobei das Verfahren Verabreichen des Oligomers nach Anspruch 1 oder des Konjugats nach Anspruch 2 oder der pharmazeutischen Zusammensetzung nach Anspruch 3 umfasst.

6. Verwendung eines Oligomers nach Anspruch 1 oder eines Konjugats nach Anspruch 2 bei der Herstellung eines Arzneimittels zur Behandlung einer Entzündungskrankheit bzw. eines Entzündungsleidens.

## Revendications

1. Oligomère d'une longueur de 12 à 16 nucléobases, comprenant une séquence de nucléobases contiguës qui est constituée par la SEQ ID n° : 246 :
Aₛ^{o}cₛAₛ^{o}aₛTₛ^{o}cₛAₛ^{o}gₛTₛ^{o}cₛ^{m}Cₛ^{o}t, ou oligomère d'une longueur de 16 nucléobases, comprenant une séquence de nucléobases contiguës qui est constituée par la SEQ ID n° : 244 : ^{m}_{Cs}^{o}cₛAₛ^{o}cₛAₛ^{o}aₛTₛ^{o}cₛAₛ^{o}gₛTₛ^{o}cₛ^{m}Cₛ^{o}tₛAₛ^{o}g,
dans lequel les lettres majuscules représentent un LNA ; l'exposant "^{o}" représente un oxy LNA ; l'indice "ₛ" représente une liaison de phosphorothioate ; les lettres en minuscules représentent un ADN ; "^{m}C" représente une 5-méthyl-cytosine ;
et dans lequel le LNA est un bêta-D-oxy LNA.

2. Conjugué comprenant l'oligomère, selon la revendication 1, et au moins une fraction non-nucléotidique étant fixée par covalence au dit oligomère.

3. Composition pharmaceutique comprenant l'oligomère, selon la revendication 1, ou le conjugué, selon la revendication 2, ainsi qu'un véhicule acceptable d'un point de vue pharmaceutique.

4. Oligomère, selon la revendication 1, ou conjugué, selon la revendication 2, destiné au traitement d'une maladie ou condition inflammatoire.

5. Procédé *in vitro* d'augmentation de l'expression d'une forme soluble de récepteur TNFRSF1B au TNF-alpha dans une cellule de mammifère qui exprime ledit récepteur au TNF-alpha, ledit procédé comprenant une administration de l'oligomère, selon la revendication 1, ou du conjugué, selon la revendication 2, ou de la composition pharmaceutique selon la revendication 3.

6. Utilisation d'un oligomère, selon la revendication 1, ou d'un conjugué, selon la revendication 2, dans la fabrication d'un médicament destiné au traitement d'une maladie ou condition inflammatoire.
